# EUROPEAN PATENT APPLICATION

(11) **EP 4 656 203 A1**
(43) Date of publication of application: **03.12.2025**
(21) Application number: 25179565.4
(22) Date of filing: 28.05.2025
(51) Int. Cl.: A61K 39/00, A61K 39/12, A61P 31/18, C07K 16/10, C07K 16/28, C07K 16/44

(54) **COMPOSITIONS AND METHODS FOR OPTIMIZED IGG PRODUCTION**

(30) Priority: 02.06.2024 US 202463655049 P
(71) Applicant: Abbratech Inc., Branford, CT 06405 (US)
(72) Inventor: WEINER, Michael P., Branford 06405 (US)
(74) Representative: Troesch Scheidegger Werner AG

(57) **Abstract**

The present disclosure provides a method of producing an antigen-binding protein (e.g., antigen-binding region, immunoglobulin, or antigen-binding fragment thereof), including cloning from a single B-cell at least one immunoglobulin heavy chain or an antigen-binding fragment thereof and at least one immunoglobulin light chain or an antigen-binding fragment thereof; producing at least one antigen-binding protein expressing host cell that expresses one or more of a single antigen-binding protein comprising one of the at least one immunoglobulin heavy chain or antigen-binding fragment thereof and one of the at least one immunoglobulin light chain or antigen-binding fragment thereof; screening/examining each of the at least one antigen-binding protein expressing host cell for the specificity, avidity, and/or affinity of the antigen-binding protein for a protein or peptide epitope of interest to find or select at least one antigen-binding protein; and expressing the at least one antigen-binding protein of interest in a eukaryotic cell.

## Description

### FIELD OF THE INVENTION

The present disclosure relates to methods of producing at least one (e.g., a plurality of) antigen-binding protein from a single B-lymphocyte.

### BACKGROUND

B lymphocytes (or B cells) are white blood cells that produce antibodies that may be secreted or inserted into the plasma membered, serving as part of the B-cell receptors. It was recently discovered that two or more V_{H}DJ_{H} or V_{L}J_{L} recombination patterns of immunoglobulin heavy chains or immunoglobulin light chains are present in single B lymphocyte, each immunoglobulin class showing unique V_{H}DJ_{H} recombination pattern in the single B lymphocyte that is expressing multiple immunoglobulin classes. Thus, when preparing an antigen-binding protein (e.g., an antibody or monoclonal antibody) from a single B-cell it is possible that the antigen-binding region of the protein is not specific to the epitope of interest (e.g., the antigen-binding region with the desired specificity, avidity, and/or affinity). In other words, typical B-cell derived monoclonal antibody discovery techniques might result in heterogeneous antibody populations.

Therefore, there exists in the art an ongoing need for a method of producing antigen-binding proteins (e.g., an antigen-binding region, antibody, immunoglobulin (Ig), immunoglobulin G (IgG), or antigen-binding fragment or portion thereof, such as scFv or Fab) with the desired epitope specificity, avidity, and/or affinity desired from a single B-cell.

### SUMMARY OF THE INVENTION

The description provides a method of producing a homogeneous antigen-binding protein (e.g., an antibody, immunoglobulin (Ig), immunoglobulin G (IgG), or antigen-binding fragment or portion thereof, such as scFv or Fab), the method comprising, consisting essentially of, or consisting of: (i) cloning from a single B-cell or B-lymphocyte (I) at least one (e.g., 1, 2, 3, 4, 5, 6, 7, 8, 9, 10, or more) immunoglobulin (Ig) heavy chain or an antigen-binding fragment thereof (e.g., a plurality of immunoglobulin (Ig) heavy chain or an antigen-binding fragment thereof) and (II) at least one (e.g., 1, 2, 3, 4, 5, 6, 7, 8, 9, 10, or more) immunoglobulin (Ig) light chain or an antigen-binding fragment thereof (e.g., a plurality of immunoglobulin (Ig) light chain or an antigen-binding fragment thereof); (ii) producing at least one (e.g., 1, 2, 3, 4, 5, 6, 7, 8, 9, 10, or more) antigen-binding protein containing/producing/expressing host cell (e.g., a plurality of antigen-binding protein producing/expressing host cells) that expresses one or more of (e.g., 1, 2, 3, 4, 5, 6, 7, 8, 9, 10, or more) a single antigen-binding protein (e.g., antibody, immunoglobulin (Ig), or antigen-binding fragment or portion thereof, such as scFv or Fab) comprising, consisting essentially of, or consisting of, one of the at least one immunoglobulin (Ig) heavy chain or an antigen-binding fragment thereof and one of the at least one immunoglobulin (Ig) light chain or an antigen-binding fragment thereof; (iii) screening or examining each of the at least one antigen-binding protein containing/producing/expressing host cell for the specificity, avidity, and/or affinity of the antigen-binding protein (e.g., antibody, immunoglobulin, or an antigen-binding fragment or portion thereof) for a protein or peptide epitope of interest to find or select at least one antigen-binding protein of interest (e.g., selecting a host cell based on the specificity, avidity, and/or affinity for the protein or peptide epitope of interest); and (iv) expressing the at least one antigen-binding protein of interest in a eukaryotic cell (e.g., mammalian cell, a Chinese hamster ovary cell, or a HEK293 cell), and (v) optionally isolating or purifying the at least one antigen-binding protein.

In any aspect or embodiment described herein, the step of cloning from a single B-cell or B-lymphocyte (I) at least one (e.g., a plurality of) immunoglobulin (Ig) heavy chain and (II) at least one (e.g., a plurality of) immunoglobulin (Ig) light chain comprises, consisting essentially of, or consisting of: (a) cloning the at least one (e.g., 1, 2, 3, 4, 5, 6, 7, 8, 9, 10, or more) immunoglobulin (Ig) heavy chain or an antigen-binding fragment or portion thereof into a first expression vector to produce at least one (e.g., 1, 2, 3, 4, 5, 6, 7, 8, 9, 10, or more) heavy chain expression vector or construct (e.g., plasmid) (e.g., a plurality of heavy chain expression vectors or construct or plasmid); (b) cloning the at least one (e.g., 1, 2, 3, 4, 5, 6, 7, 8, 9, 10, or more) immunoglobulin (Ig) light chain or an antigen-binding fragment or portion thereof into a second expression vector to produce at least one (e.g., 1, 2, 3, 4, 5, 6, 7, 8, 9, 10, or more) light chain expression vector or construct (e.g., plasmid) (e.g., a plurality of light chain expression vectors or construct or plasmid); or (c) a combination thereof.

In any aspect or embodiment described herein, the method further comprises, consists essentially of, or consists of: (i) isolating and/or extracting (e.g., isolating and/or extracting the deoxyribonucleic acid (DNA) of) the at least one heavy chain expression vector; (ii) isolating and/or extracting (e.g., isolating and/or extracting the deoxyribonucleic acid (DNA) of) the at least one light chain expression vector; or (iii) a combination thereof.

In any aspect or embodiment described herein, producing at least one (e.g., 1, 2, 3, 4, 5, 6, 7, 8, 9, 10, or more) antigen-binding protein containing/producing/expressing host cell that expresses one or more of a single antigen-binding protein comprising, consisting essentially of, or consisting of, pairing each of the plurality (e.g., 1, 2, 3, 4, 5, 6, 7, 8, 9, 10, or more) of immunoglobulin (Ig) heavy chain or an antigen-binding fragment thereof with each of the plurality (e.g., 1, 2, 3, 4, 5, 6, 7, 8, 9, 10, or more) of immunoglobulin (Ig) light chain or an antigen-binding fragment thereof to produce a plurality of antigen-binding protein containing/producing/expressing host cells that each expresses a single (homogeneous) antigen-binding protein immunoglobulin.

In any aspect or embodiment described herein, producing at least one antigen-binding protein containing/producing/expressing host cell (e.g., a plurality of antigen-binding protein producing/expressing host cells) comprises, consists of, or consists essentially of: (i) transforming each of the at least one (e.g., each of the plurality of, 1, 2, 3, 4, 5, 6, 7, 8, 9, 10, or more) heavy chain expression vector into an individual host cell (e.g., a yeast/fungi cell) to produce at least one (e.g., 1, 2, 3, 4, 5, 6, 7, 8, 9, 10, or more) heavy chain containing/producing/expressing host cell (e.g., a plurality of heavy chain containing/producing/expressing host cells); (ii) transforming each of the at least one (e.g., each of the plurality of) light chain expression vector into an individual host cell (e.g., a yeast/fungi cell) to produce at least one (e.g., 1, 2, 3, 4, 5, 6, 7, 8, 9, 10, or more) light chain containing/producing/expressing host cell (e.g., a plurality of light chain containing/producing/expressing host cells); and (iii) mating one of the at least one heavy chain containing/producing/expressing host cell and one of the at least one light chain containing/producing/expressing host cell (e.g. mating each of the at least one heavy chain containing/producing/expressing host cell with each of the at least one light chain containing/producing/expressing host cell to produce a plurality antigen-binding protein containing/producing/expressing host cells that expresses one or more of a single antigen-binding protein), and optionally integrating the heavy chain expression vector and the light chain expression vector in the at least one antigen-binding protein containing/producing/expressing host cell (e.g. the plurality of antigen-binding protein containing/producing/expressing host cells).

In any aspect or embodiment described herein, (i) expressing the at least one antigen-binding protein further comprises amplifying the expression vector (e.g., if the heavy chain and light chain expression vectors have been integrated) or expression vectors (e.g., the heavy chain expression vector and the light chain expression vector) in a bacterial cell(s) (e.g., *Escherichia coli* and/or transforming a bacterial cell(s) and growing the transformed bacterial cell(s)) with the expression vector or expression vectors); (ii) the method further comprises isolating and/or extracting the expression vector (e.g., if the heavy and light chain expression vectors have been integrated) or expression vectors (e.g., the heavy chain expression vector and the light chain expression vector) for each of the at least one antigen-binding protein from the transformed bacterial cell(s); (iii) expressing the at least one antigen-binding protein further comprises transfecting the eukaryotic cell with the expression vector (e.g., if the heavy chain and light chain expression vectors have been integrated) or expression vectors (e.g., the heavy chain expression vector and the light chain expression vector); or (iv) a combination thereof.

In any aspect or embodiment described herein, the method further comprises, consists essentially of, or consists of, prior to cloning from a single B-cell or B-lymphocyte, selecting and/or isolating a B-cell or B-lymphocyte expressing CD19 (e.g., the B-cell or B-lymphocyte expresses at least one of CD19 and CD38 and/or the B-cell or B-lymphocyte does not express CD10) and that binds (e.g., binds with sufficient specificity, avidity, and/or affinity) the protein or peptide epitope of interest.

In any aspect or embodiment described herein, selecting and/or isolating a B-cell or B-lymphocyte comprises, consists essentially of, or consists of, selecting and/or isolating a naive B cell (e.g., a CD19⁺CD38^{+/-}CD10⁻CD27⁻ B-cell), a memory B-cell (e.g., CD19⁺CD38^{+/-}CD10⁻CD27⁺ B-cell), or a plasma cell (e.g., a CD19⁺CD38⁺CD10⁻ B-cell) that binds (e.g., binds with sufficient specificity, avidity, and/or affinity) the protein or peptide epitope of interest.

In any aspect or embodiment described herein, the first expression vector and the second expression vector have the same sequence.

In any aspect or embodiment described herein, the first expression vector and the second expression vector have different sequences.

In any aspect or embodiment described herein, screening or examining each of the at least one antigen-binding protein containing/producing/expressing host cell for the specificity, avidity, and/or affinity of the antigen-binding protein comprises, consists essentially of, or consists of: providing a set of fusion proteins that comprises two or more (e.g., 2, 3, 4, 5, 6, 7, 8, 9, 10, 11, 12, 13, 14, 15, 16, 17, 18, 19, or 20) fusion proteins, wherein each fusion protein includes a maltose-binding protein, or amylose-binding derivative thereof, fuses directly to or via a linker to a target sequence of about 8 amino acids to 18 amino acids (e.g., about 10 to about 18 amino acids, about 10 to about 16 amino acids, or 8, 9, 10, 11, 12, 13, 14, 15, 16, 17, or 18 amino acids) from the protein or peptide epitope of interest; and each target sequence of the set of fusion proteins has a different proteinogenic amino acid at a site of interest in the target sequence, including (i) one that has the native amino acid of the target sequence or (ii) one or more has a native amino acid when the site of interest is a variant site; and examining or detecting which of the fusion proteins that the antigen-binding protein (e.g., antibody or an antigen-binding fragment or portion thereof) binds.

In any aspect or embodiment described herein, detecting is performed via an enzyme-linked immunosorbent assay, and each fusion protein is a different antigen of the enzyme-linked immunosorbent assay (e.g., each fusion protein is located in a different well).

In any aspect or embodiment described herein, the maltose-binding protein derivative has been engineered/modified to provide tighter binding (e.g., enhance the binding) to amylose resin; the linker is a protein linker; the linker is a protein linker, wherein the protein linker includes or consists of about 1 to about 10 amino acids (e.g., 1, 2, 3, 4, 5, 6, 7, 8, 9, or 10 amino acids, each amino acid is independently selected from glycine and serine (e.g., a GS, GSG, GSGS (SEQ ID NO:16), GSGSG (SEQ ID NO: 17), or GSGSGS (SEQ ID NO:18) linker), or a combination thereof); the target sequence is linked to the C-terminus of the maltose-binding protein; or a combination thereof.

In any aspect or embodiment described herein, the set of fusion proteins includes a fusion protein comprising a target sequence for two or more variant of the protein or peptide epitope of interest (e.g., each variant of the protein or peptide epitope of interest).

In any aspect or embodiment described herein, (i) the antigen-binding protein binds to a specific variant of the protein or peptide epitope of interest (e.g., a variant sited antigen binding region, antibody, or antigen binding fragment or portion thereof), or (ii) the antigen-binding protein binds to a specific set of (e.g., 1, 2, 3, 4, 5, 6, or more) variants (e.g., all variants) of the protein or peptide epitope of interest (e.g., a pan variant antigen-binding protein, antigen binding region, antibody, or antigen binding fragment or portion thereof).

In any aspect or embodiment described herein, the antigen-binding protein binds to only a target sequence with the native amino acid at the site of interest (e.g., a sited antigen-binding protein, antigen binding region, antibody, or antigen binding fragment or portion thereof).

In any aspect or embodiment described herein, the method further comprises, consists essentially of, or consists of: (i) expressing each fusion protein via an expression vector or construct (e.g., plasmid) that expresses the fusion protein (e.g., expressing in bacteria (such as *Escherichia coli),* fungi, or eukaryotic cell (such as mammalian cell)); (ii) purifying each fusion protein from a cell (e.g., bacteria (such as *Escherichia coli),* fungi, or eukaryotic cell (such as mammalian cell)) in which it was expressed; or (iii) a combination thereof.

In any aspect or embodiment described herein, purifying comprises, consists essentially of, or consists of, for one or more of the fusion proteins (e.g., each fusion protein): (i) adding a cell lysate containing the fusion protein to a gravity flow column comprising amylose resin (e.g., the cell lysate that flows through the gravity flow column is collected and added to the gravity glow column one or more (e.g., 1, 2, 3, or 4) times); (ii) optionally washing the gravity flow column after the cell lysate is added; (iii) eluting and/or collecting the fusion protein; or (iv) a combination thereof.

In any aspect or embodiment described herein, the method further comprises, consists essentially of, or consists of, determining or quantifying the lower limit of detection of the antigen-binding protein (e.g., antibody or an antigen-binding fragment or portion thereof) by examining the protein or peptide epitope of interest at a plurality of concentrations/amount (e.g., the lower limit of detection of the antigen-binding protein is the lowest concentration/amount of the protein or peptide epitope of interest that is detected and a lower concentration/amount is not detected).

In any aspect or embodiment described herein, the method further comprises, consists essentially of, or consists of, generating the B-cell or B-lymphocyte.

In any aspect or embodiment described herein, generating the B-cell or B-lymphocyte comprises, consisting essentially of, or consisting of: (a) immunizing an animal at least once with a modified peptide having an amino acid sequence of about 8 to about 20 amino acids identical to an amino acid sequence of the protein or peptide epitope of interest that includes the amino acids of the target sequence, except wherein one internal amino acid (e.g., the site of interest or adjacent to the site of interest) has been substituted with a non-native amino acid (nnAA); and (b) boosting the animal at least once with a first unmodified peptide comprising a core amino acid sequence identical to the modified peptide of step (a), except wherein the nnAA has been substituted with the native amino acid (nAA), a first N-terminal amino acid sequence that is not native to the protein or peptide epitope of interest, and a first C-terminal amino acid sequence that is not native to the protein or peptide epitope of interest.

In any aspect or embodiment described herein, the method further comprises, consists essentially of, or consists of: (c) cloning B-cells obtained from the animal; and (d) identifying a clone of step (c) that: (i) binds to the first unmodified peptide and a second unmodified peptide comprising a core amino acid sequence that is identical to the first unmodified peptide, a second N-terminal amino acid sequence that is not native to the protein or peptide epitope of interest, and a second C-terminal amino acid sequence that is not native to the protein or peptide epitope of interest; and (ii) does not bind to the modified peptide of step (a).

In any aspect or embodiment described herein, the method further comprises, consists essentially of, or consists of, identifying a clone that binds to the protein or peptide epitope of interest.

In any aspect or embodiment described herein, the method further comprises, consists essentially of, or consists of: (c) cloning B-cells obtained from the animal; and (d) identifying a clone of step (c) that bind to the protein or peptide epitope of interest in its native conformation.

In any aspect or embodiment described herein, the animal is a human, a rabbit, a mouse, a rat, a goat, a cow, a pig, a camelid, or a chicken.

In any aspect or embodiment described herein, the animal is a non-human animal that has a human or humanized immune system.

In any aspect or embodiment described herein, the modified peptide, the first unmodified peptide, the second unmodified peptide, or a combination thereof, is administered with an adjuvant (e.g., the adjuvant is Complete Freund's Adjuvant (CFA), Incomplete Freund's Adjuvant (IFA), aluminum, monophosphoryl lipid A (MPL) and aluminum salt (AS04), oil-in-water emulsion, oil-in-water emulsion of squalene (MF59), AS03 (Vitamin E, Surfactant polysorbate 80, and squalene), MPL and QS-21 in a liposome formulation (AS01), or cytosine phosphoguanine (CpG)).

In any aspect or embodiment described herein, the modified peptide is conjugated to one or more carriers, the first unmodified peptide is conjugated to one or more carriers, or a combination thereof.

In any aspect or embodiment described herein, generating the B-cell or B-lymphocyte comprises, consisting essentially of, or consisting of: (a) providing a modified peptide having an amino acid sequence of about 8 to about 20 amino acids identical to an amino acid sequence of the protein or peptide epitope of interest and includes the amino acids of the target sequence, except wherein the wild-type amino acid at the site of interest (e.g., a variant site) has been substituted with a non-native amino acid (nnAA); (b) screening the modified peptides against a library; (c) isolating one or more binding agents that bind to the modified peptide; (d) generating a library of clonotypes of the one or more binding agents isolated in step (c); (e) screening the library of clonotypes against: (i) the modified peptide of step (a); and (ii) a first unmodified peptide comprising a core amino acid sequence identical to the modified peptide of step (a), except wherein the non-native amino acid (nnAA) has been substituted with the wild-type amino acid (nAA) and comprising a first N-terminal amino acid sequence that is not native to the protein or peptide epitope of interest and a first C-terminal amino acid sequence that is not native to the protein or peptide epitope of interest; and (f) isolating a binding agent that bind to both the first unmodified peptide and the modified peptide, wherein the binding agent is the antigen-binding protein (e.g., antibody or an antigen-binding fragment or portion thereof) or derived therefrom.

In any aspect or embodiment described herein, generating the B-cell or B-lymphocyte comprises, consisting essentially of, or consisting of: (a) providing a modified peptide having an amino acid sequence of about 8 to about 20 amino acids identical to an amino acid sequence of the protein or peptide epitope of interest and includes the amino acids of the target sequence, except wherein the wild-type amino acid at the site of interest (e.g., a variant site) has been substituted with a non-native amino acid (nnAA); (b) screening the modified peptides against a library; (c) isolating one or more binding agents that bind to the modified peptide; (d) generating a library of clonotypes of the one or more binding agents isolated in step (c); (e) screening the library of clonotypes against: (i) the modified peptide of step (a); and (ii) a first unmodified peptide comprising a core amino acid sequence identical to the modified peptide of step (a), except wherein the non-native amino acid (nnAA) has been substituted with the wild-type amino acid (nAA) and comprising a first N-terminal amino acid sequence that is not native to the protein or peptide epitope of interest and a first C-terminal amino acid sequence that is not native to the protein or peptide epitope of interest; (f) isolating a binding agent that bind to both the first unmodified peptide and the modified peptide; (g) generating a library of clonotypes of the binding agent isolated in step (f); (h) screening the library of clonotypes of step (g) against: (i) the modified peptide; (ii) the first unmodified peptide; and (iii) a second unmodified peptide comprising a core amino acid sequence that is identical to the first unmodified peptide, a second N -terminal amino acid sequence that is not native to the protein or peptide epitope of interest, and a second C-terminal amino acid sequence that is not native to the protein or peptide of epitope interest; and (f) isolating a binding agent that binds to the first unmodified peptide and the second unmodified peptide, wherein the binding agent does not bind the modified peptide, and the binding agent is the antigen-binding protein (e.g., antibody or an antigen-binding fragment or portion thereof) or derived therefrom.

In any aspect or embodiment described herein, generating the B-cell or B-lymphocyte comprises, consisting essentially of, or consisting of: (a) providing a modified peptide having an amino acid sequence of about 8 to about 20 amino acids identical to an amino acid sequence of the protein or peptide epitope of interest and includes the amino acids of the target sequence, except wherein the wild-type amino acid at the site of interest (e.g., a variant site) has been substituted with a non-native amino acid (nnAA); (b) screening the modified peptides against a library; (c) isolating one or more binding agents that bind to the modified peptide; (d) generating a library of clonotypes of the one or more binding agents isolated in step (c); (e) screening the library of clonotypes against: (i) the modified peptide of step (a); and (ii) a first unmodified peptide comprising a core amino acid sequence identical to the modified peptide of step (a), except wherein the non-native amino acid (nnAA) has been substituted with the wild-type amino acid (nAA) and comprising a first N-terminal amino acid sequence that is not native to the protein or peptide epitope of interest and a first C-terminal amino acid sequence that is not native to the protein or peptide epitope of interest; (f) isolating a binding agent that bind to both the first unmodified peptide and the modified peptide; (g) generating a library of clonotypes of the binding agent isolated in step (f); (h) screening the library of clonotypes of step (g) against: (i) the modified peptide; (ii) the first unmodified peptide; and (iii) a second unmodified peptide comprising a core amino acid sequence that is identical to the first unmodified peptide, except wherein the wild-type amino acid (nAA) has been substituted with an amino acid corresponding to a variant and comprising a second N -terminal amino acid sequence that is not native to the protein or peptide epitope of interest and a second C-terminal amino acid sequence that is not native to the protein or peptide epitope of interest; and (i) isolating a binding agent that binds to the second unmodified peptide and does not bind the first modified peptide, wherein the binding agent is the antigen-binding protein (e.g., antibody or an antigen-binding fragment or portion thereof) or derived therefrom.

In any aspect or embodiment described herein, the non-native amino acid (nnAA) is offset from or relative to the site of interest (e.g., offset by +/-4, +/-3, +/-2, or +/-1 relative to the site of interest (position 0); offset by +/- 1, +/-2, or +/-3 relative to the site of interest when it is a post-translational modification site (position 0); or offset by +/- 2 position relative to the site of interest when it is a splice site/junction (position 0)).

In any aspect or embodiment described herein, the non-native amino acid (nnAA) is a non-synonymous amino acid.

In any aspect or embodiment described herein, the non-native amino acid (nnAA) is phosphorylated, acetylated, isocyanated, sulfated, or nitrated.

In any aspect or embodiment described herein, (i) the non-native amino acid (nnAA) acid is O-phosphoserine (SEP), phosphotyrosine, or phosphothreonine; (ii) the N-terminal amino acid sequence is SerGlySer, GlySerGly, GlyGlyGly, or SerSerSer; (iii) the C-terminal amino acid sequence is SerGlySer, GlySerGly, GlyGlyGly, or SerSerSer; or (iv) a combination thereof.

The preceding general areas of utility are given by way of example only and are not intended to be limiting on the scope of the present disclosure and appended claims. Additional objects and advantages associated with the compositions, methods, and processes of the present disclosure will be appreciated by one of ordinary skill in the art in light of the instant claims, description, and examples. For example, the various aspects and embodiments of the present disclosure may be utilized in numerous combinations, all of which are expressly contemplated by the present description. These additional advantages objects and embodiments are expressly included within the scope of the present disclosure. The publications and other materials used herein to illuminate the background of the present disclosure, and in particular cases, to provide additional details respecting the practice, are incorporated by reference.

### BRIEF DESCRIPTION OF THE DRAWINGS

The accompanying drawings, which are incorporated into and form a part of the specification, illustrate several embodiments of the present disclosure and, together with the description, serve to explain the principles of the present disclosure. The drawings are only for the purpose of illustrating an embodiment of the present disclosure and are not to be construed as limiting the present disclosure. Further objects, features and advantages of the disclosure will become apparent from the following detailed description taken in conjunction with the accompanying figures showing illustrative embodiments of the disclosure.

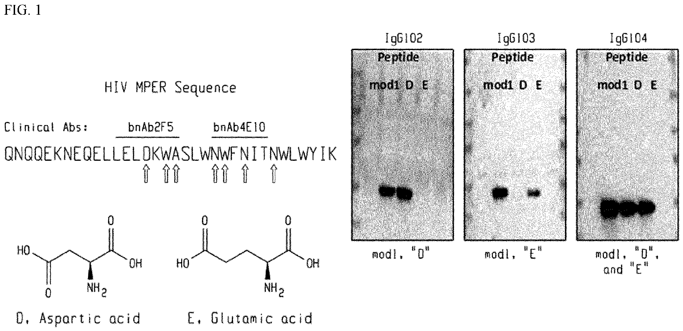

**Figure 1** is an illustration of how the method for examining specificity and/or affinity of an antigen-binding protein of the present disclosure (also referred to herein as "SUNDAE") is used to develop antibodies that have reduced off-target effects/binding. The method can be used to understand the promiscuity of antibodies and choose candidates that have reduced off-target binding. Antibodies for membrane-proximal external region (MPER) of human immunodeficiency virus-1 (HIV-1) glycoprotein 41 (gp41) (a portion of GP41 (SEQ ID NO:19), including the sequence of MPER, is shown) were used for illustrative purposes.

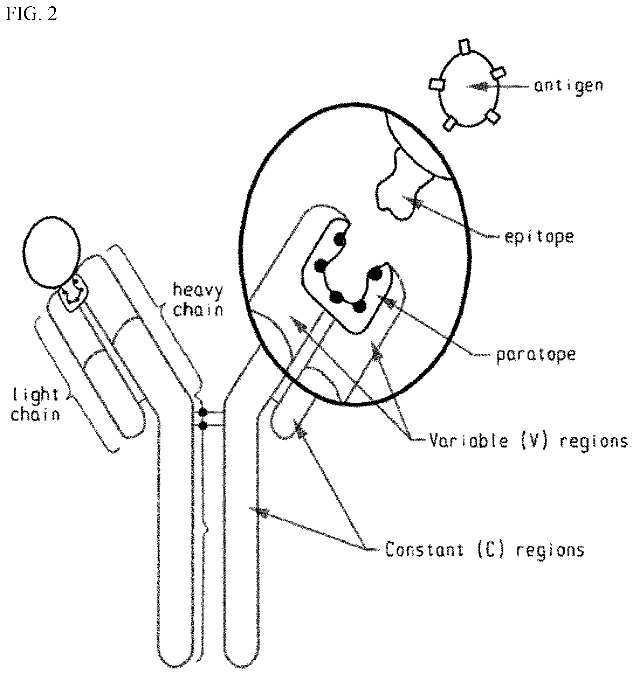

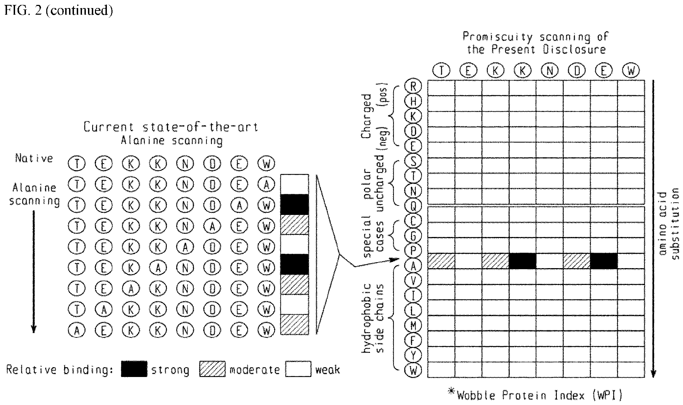

**Figure 2** is an illustration of how the method for examining specificity and/or affinity of an antigen-binding protein of the present disclosure (also referred to herein as "SUNDAE") technology can develop variant specific antibodies to the entire human proteome.

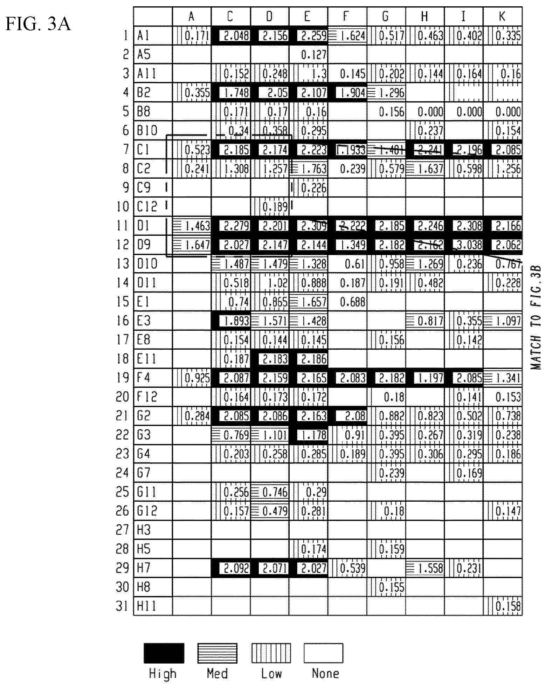

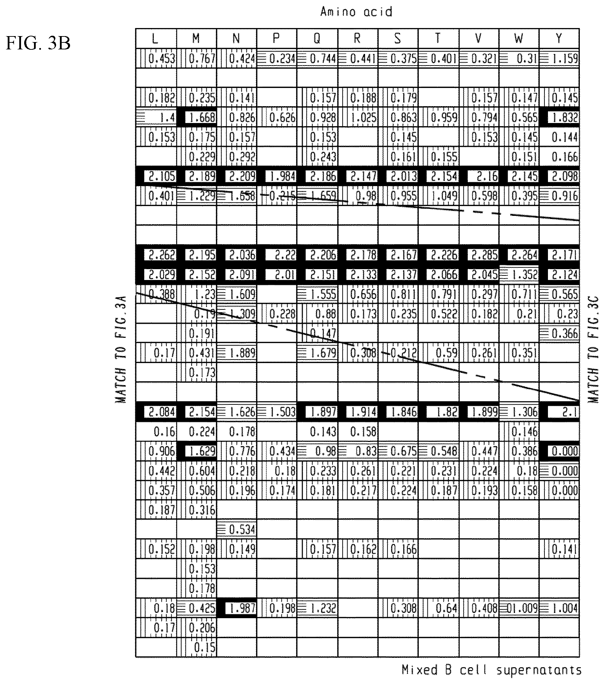

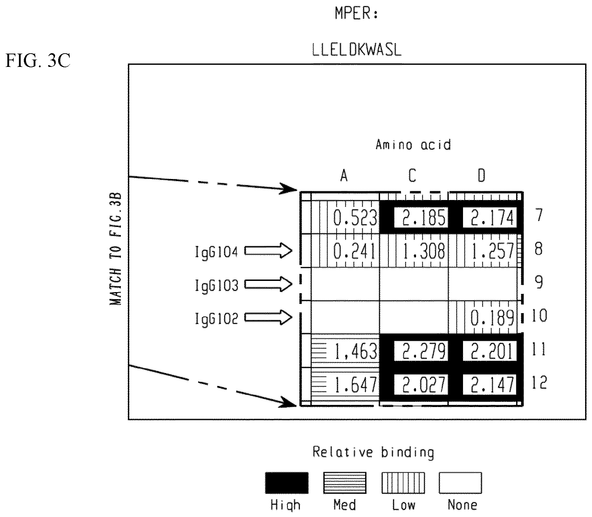

**Figures 3A****,** **3B****, and** **3C** are an illustration of some antibodies ranked using the method for examining specificity and/or affinity of an antigen-binding protein of the present disclosure (also referred to herein as "SUNDAE"). The method permits the engineering of (i) affinity without reducing the specificity of the antibody and (ii) antibodies with reduced or eliminated off-target effects.

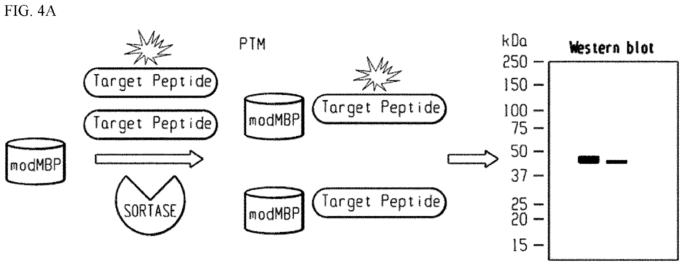

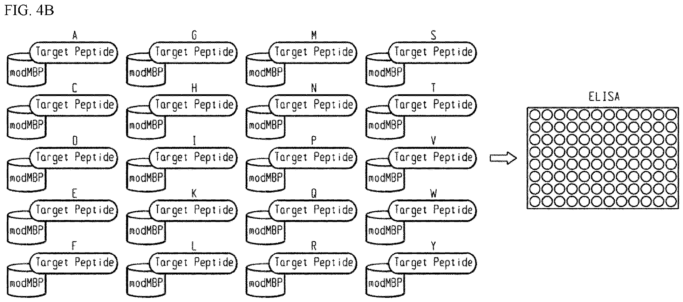

**Figures 4A** **and** **4B****. (4A)** Workflow for Western blot analysis with maltose-binding proteins conjugated to a target peptide sequence. The maltose-binding proteins can be conjugated to a target peptide sequence via a conjugation reaction between a target peptide (with or without a post translational modification) and a maltose-binding protein (such as a modified maltose-binding protein with tighter binding to amylose resin) using the sortase enzyme. These proteins can then be tested to determine the specificity of antibodies directed to post-translational modifications. (4B) Workflow for examining specificity and/or affinity of an antigen binding region with maltose-binding proteins fused with a target sequence. The method contains a set of, e.g., 20 maltose-binding proteins that contain genetically encoded target sequences, wherein one residue (site of interest, such as a variant site) has been replaced by all 20 proteinogenic or naturally occurring amino acids. These fusion proteins can be used in, e.g., enzyme-linked immunosorbent assay (ELISA) to determine the binding affinity of antibodies when this single residue is replaced.

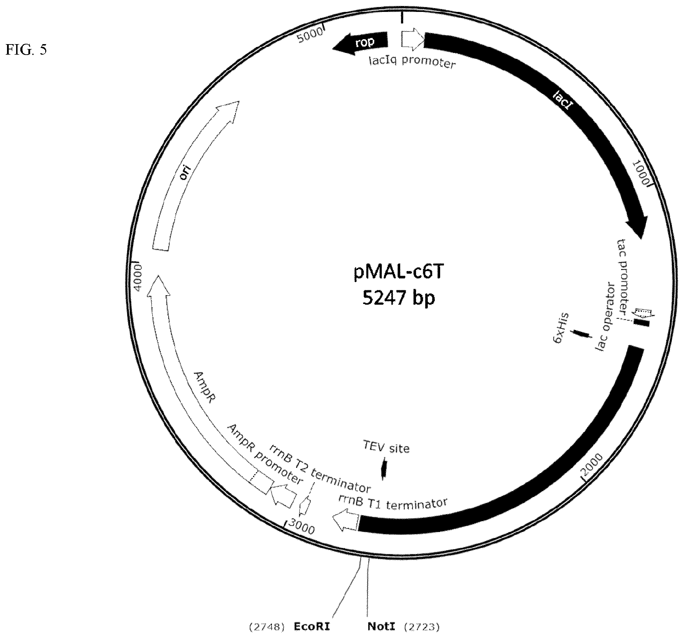

**Figure 5****.** Plasmid map of pMAL-c6T. This vector (New England BioLabs^{®}; Ipswich, Massachusetts) was modified to construct both the sortase acceptor plasmid for the maltose-binding proteins conjugated to a target peptide and the fusion proteins containing a maltose-binding protein fused with a target peptide.

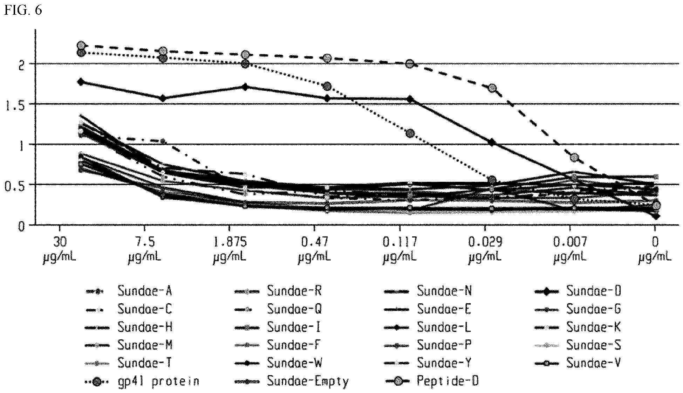

**Figure 6****.** The set of fusion proteins containing a maltose-binding protein fused to a target sequence shed light on which residues are important for antibody binding. 2F5 is a broadly neutralizing human monoclonal antibody known to bind the sequence 657-EQELLEL**D**KWASLW-670 (SEQ ID NO:1) found in the membrane-proximal external region (MPER) of human immunodeficiency virus-1 (HIV-1) glycoprotein 41 (gp41). The fusion proteins were constructed with an insert of this HIV sequence in which the underlined residue (EQELLEL**X**KWASLW; SEQ ID NO:2) was replaced with each of the naturally occurring amino acids (Sundae-A for alanine, Sundae-C for cysteine, etc.). These fusion proteins were then tested in an enzyme-linked immunosorbent assay (ELISA) for binding to 2F5. ELISA plates were coated with 1 µg mL⁻¹ of the fusion proteins as the antigen, and primary antibody 2F5 (Polymun Scientific; Klosterneuburg, Austria) was tested using 4-fold dilutions in 2% bovine serum albumin (BSA). The 2F5 antibody binds strongly to a full length gp41 HIV protein (Abcam, Cambridge, United Kingdom), a native HIV sequence peptide, as well as the Sundae-D protein containing the sequence EQELLEL**D**KWASLW (SEQ ID NO:1). Binding is reduced when tested against the fusion/Sundae proteins containing each of the other 19 amino acids, as well Sundae-Empty (maltose-binding protein with no HIV sequence present) as a negative control. These data suggest that the D at position 664 is critical for 2F5 antibody binding.

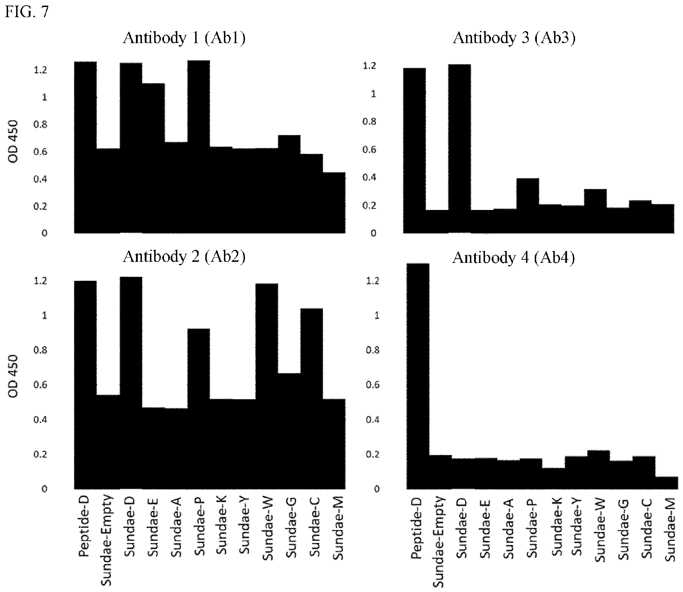

**Figure 7****.** Antibody reactivity varies depending on which residue is present in fusion/Sundae proteins. Different rabbit Immunoglobulin G (IgG) clones (measured at 5 µg mL⁻¹) show different reactivity when a single residue in the antibody's epitope (an aspartic acid in the native sequence) is replaced with other amino acids (Sundae-A, Sundae-E, etc.). Antibody 3 (Ab3) is able to bind the original amino acid, aspartic acid as well as a peptide version containing aspartic acid (D). However, Antibody 1 (Ab1) is able to bind both those antigens as well as fusion/Sundae proteins with glutamic acid and proline substitutions (Sundae-E and Sundae-P). Antibody 2 (Ab2) recognizes tryptophan and cysteine (Sundae-W and Sundae-C) as well at that same position in the epitope sequence, but not glutamic acid. Antibody 4 (Ab4) is only able to recognize the peptide version of the antigen sequence and not any of the Sundae proteins. None of these antibodies were able to bind alanine at this position. Unlike alanine scanning, the method described herein using maltose-binding fusion proteins can fully interrogate the contribution of a single residue to antibody binding.

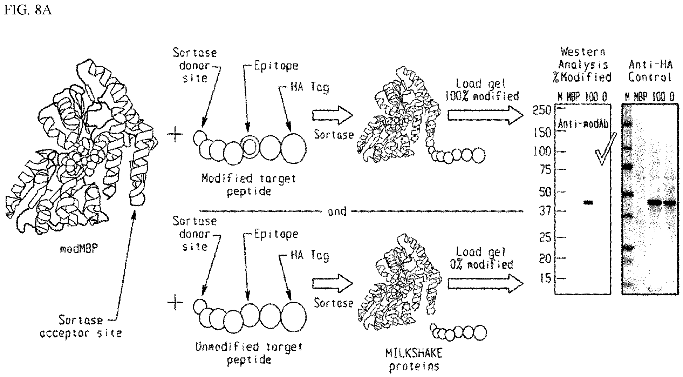

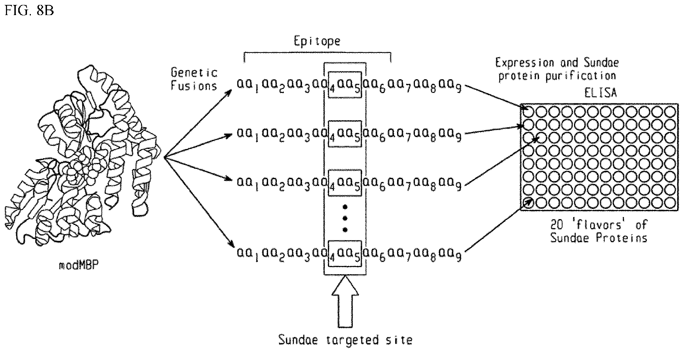

**Figures 8A** **and** **8B****.** Exemplary workflows for antibody analysis with maltose-binding protein conjugated to a target peptide and fusion proteins containing a maltose-binding protein fused with a target peptide described herein. **(8A)** Maltose-binding protein (e.g., a maltose-binding protein engineered to more tightly bind amylose resin) was purified from *E. coli* cultures and conjugated via sortase enzyme to peptides containing an N-terminal sortase conjugation site (GGGSGSS; SEQ ID NO:3), a target-specific sequence that is either modified (e.g., phosphorylated) or non-modified at a single amino acid target site, and a C-terminal hemagglutinin (HA) tag (YPYDVPDYA; SEQ ID NO:4). Western blot analysis reveals binding specificity of the antibody under testing. The expected size of conjugated protein is 42 kDa. The anti- hemagglutinin control blot was incubated with anti-hemagglutinin HRP antibody (Abcam, Cambridge, United Kingdom) at 1:5,000 dilution. Exposure 51 seconds. Each lane contains 500 ng of conjugated protein and 15 µg HEK293 cell lysate. Lanes: M, Precision Plus Protein Standard (BioRad; Hercules, California); 1, unconjugated maltose-binding protein negative control; 2, phospho conjugated maltose-binding protein Target A; 3, non-phospho conjugated maltose-binding protein Target A. **(8B)** Fusion proteins contain genetically encoded epitope target sequences fused to the C-terminus of a maltose-binding protein (e.g., a maltose-binding protein engineered to more tightly bind amylose resin) in a set of twenty different constructs. In each construct, one residue has been replaced by each of the 20 naturally occurring amino acids. These proteins can be used in single-well or titration enzyme-linked immunosorbent assay (ELISA) to determine binding affinity of antibodies when this single residue is replaced.

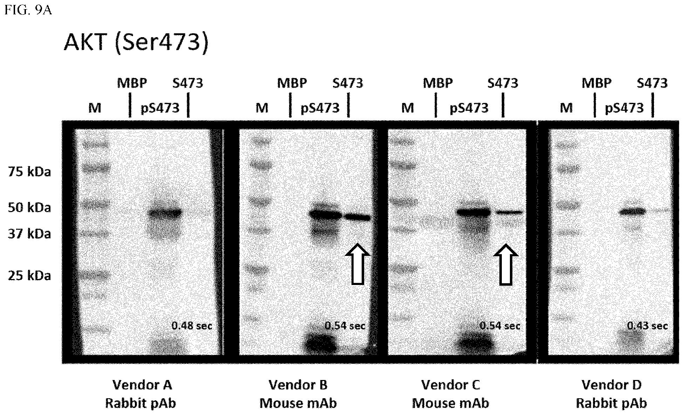

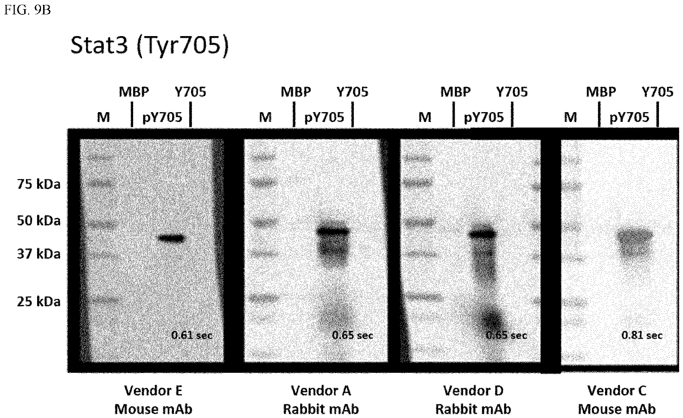

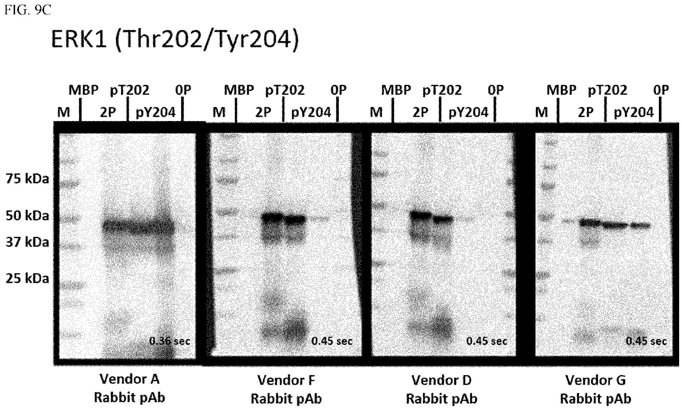

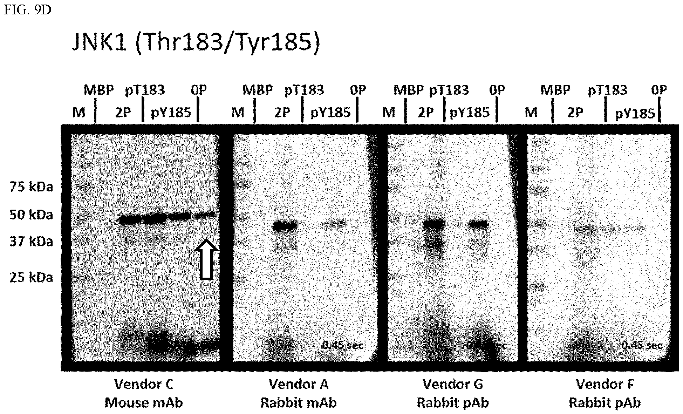

**Figures 9A****,** **9B****,** **9C****, and** **9D****.** Published phosphor-specific antibodies tested in a low stringency assay using maltose-binding proteins conjugated to a target peptide. Blots were incubated with primary antibodies from seven vendors diluted to vendor recommended concentrations for four different desired target sequences **(9A)** phosphor-AKT (Ser473), **(9B)** phosphor-Stat3 (Tyr705), **(9C)** phosphor-ERK1 (Thr202/Tyr204), and **(9D)** phosphor-JNK1 (Thr183/Tyr185). Each Immunoglobulin G (IgG) antibody binds the phosphorylated residues listed for each target, which are present in the phosphor conjugated maltose-binding protein in Lane **2 (9A** and **9B)** or the dual phosphorylated peptide conjugated maltose-binding protein in Lane 2P or the single-phosphorylated peptide conjugated maltose-binding protein (in **9C** and **9D)** In some cases, antibodies also bind non-phosphorylated peptide conjugated maltose-binding protein (see arrows in **9A** and Lane 0P in **9D).** Each lane in **(9A)** and **(9B)** was loaded with 3 µg conjugated maltose-binding protein only, and each lane in **(9C)** and **(9D)** was loaded with 10 µg conjugated maltose-binding protein only. Exposure times are listed on each blot. Lane M: Precision Plus Protein Standard (BioRad; Hercules, California). Lane MBP: unconjugated maltose-binding protein, negative control.

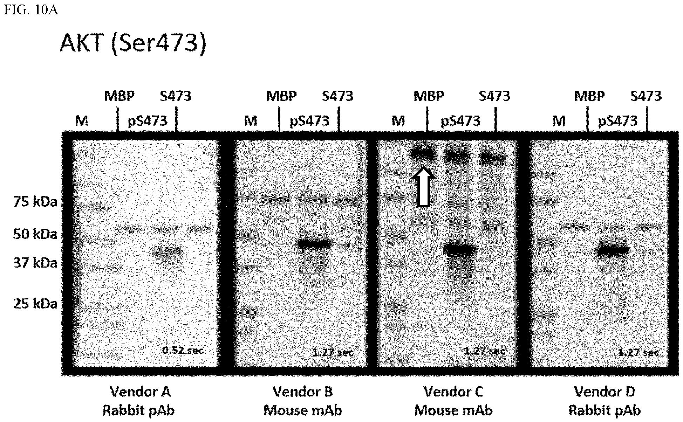

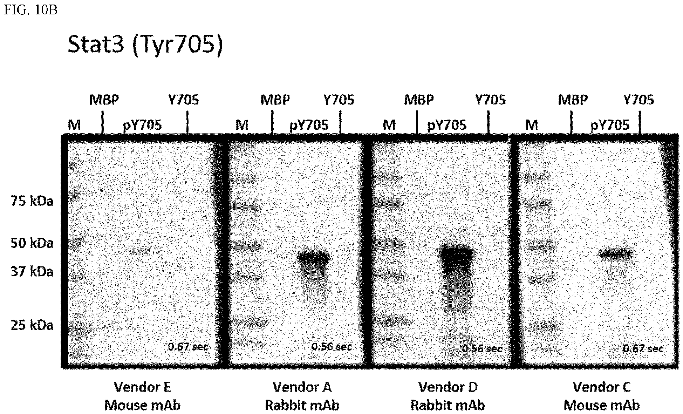

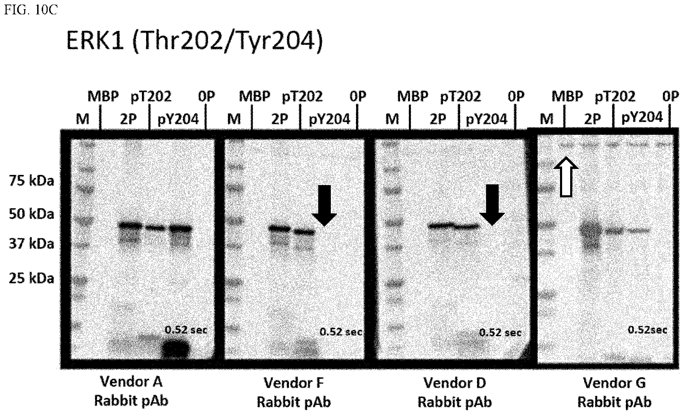

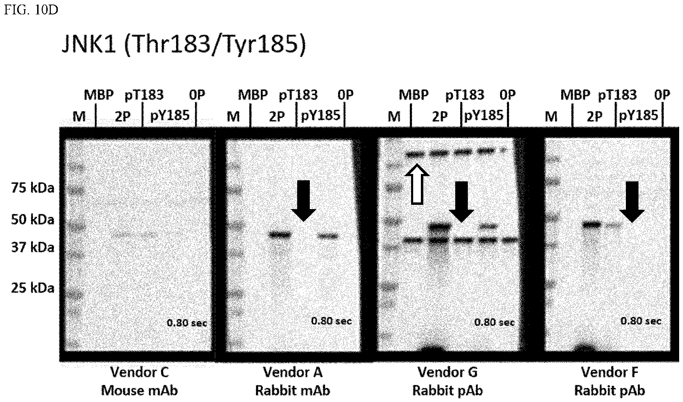

**Figures 10A****,** **10B****,** **10C****, and** **10D****.** Published phospho-specific antibodies show varying degrees of specificity in high stringency assay using maltose-binding proteins conjugated to a target peptide. Blots were incubated with primary antibodies from seven vendors at vendor recommended concentrations for four different desired target sequences **(10A)** phospho-AKT (Ser473), **(10B)** phospho-Stat3 (Tyr705), **(10C)** phosphor-ERK1 (Thr202/Tyr204), and **(10D)** phosphor-JNK1 (Thr183/Tyr185). Each IgG Ab binds the phospho conjugated modMBP in Lane 2 **(10A** and **10B)** or the dual phosphorylated peptide conjugated to a maltose-binding protein in Lane 2P or the single-phosphorylated peptide conjugated to maltose-binding protein (in **10C** and **10D).** In some cases, the antibodies also bind non-specifically to other cellular proteins that do not match the expected size of the target protein (see white arrows in **10A, 10D)** In the dual post-translational modification blots, some antibodies failed to recognize both phospho sites individually (see black arrows **10C and 10D).** Each lane contains 500 ng maltose-binding protein and 15 µg of HEK293 cell lysate. Exposure times are listed on each blot. Lane M: Precision Plus Protein Standard (BioRad; Hercules, California). Lane MBP: unconjugated maltose-binding protein, negative control.

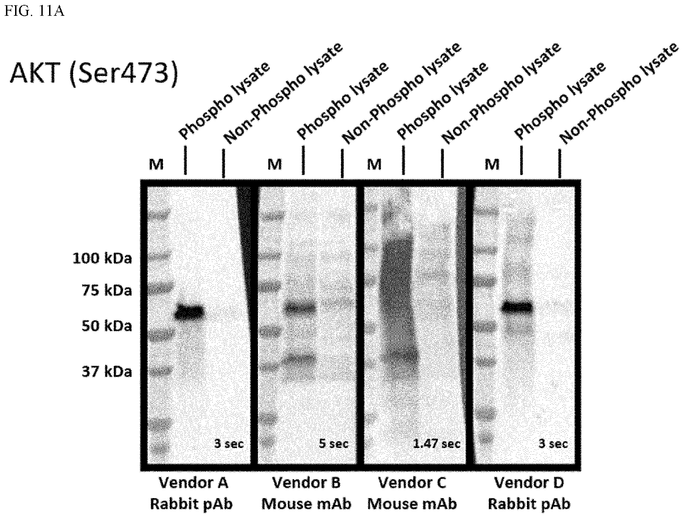

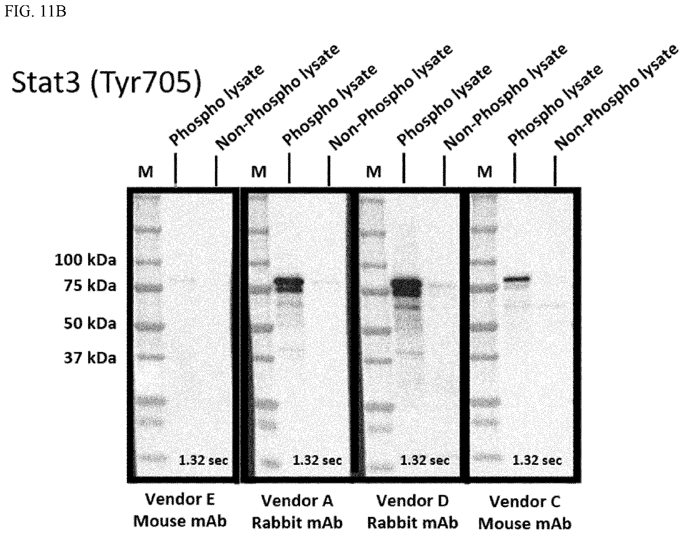

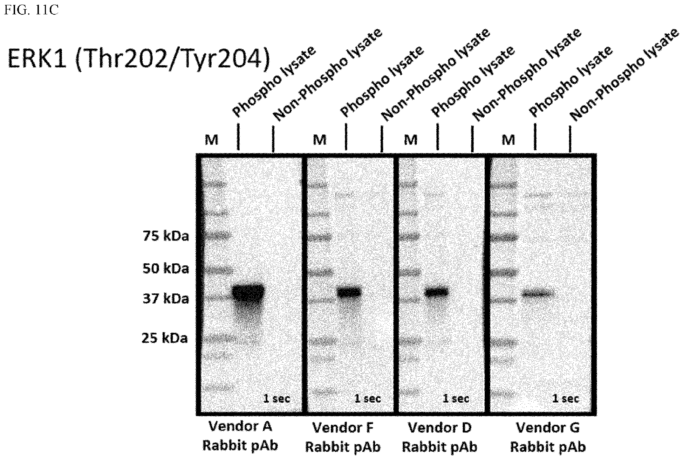

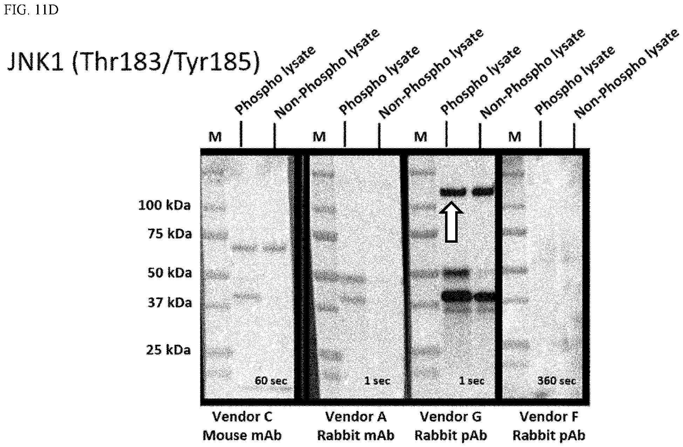

**Figures 11A****,** **11B****,** **11C****, and** **11D****.** Phospho-specific antibodies tested with commercially available treated cell lysates. Lanes were loaded with cell lysates specifically treated for each post-translational modification site under testing (Cell Signaling Technology, Danvers, Massachusetts). Lane M: Precision Plus Protein Standard (BioRad; Hercules, California). Lane Phospho lysate: cell lysate treated to produce phosphorylated site specific to the primary antibody tested. Lane Non-phospho lysate: cell lysate treated to produce non-phosphorylated site specific to the primary antibody tested. **(11A)** Phospho lysate (20 µl): Jurkat cells, serum starved overnight followed by treatment with Calyculin A. Non-phospho lysate (20 µl): Jurkat cells, serum starved overnight followed by treatment with 50 µM LY294002. The expected size of AKT is 60 kDa. **(11B)** Phospho lysate (10 µl): serum-starved HeLa cells treated with 100 ng/ml interferon-alpha for 5 minutes. Non-phospho lysate (10 µl): serum-starved HeLa cell lysate. The expected size of Stat3 is 86 kDa. **(11C)** Phospho lysate (15 µl): Jurkat cells treated with 200 nM TPA for 20 minutes. Non-phospho lysate (15 µl): Jurkat cells treated with 10 µM U0126 for 1 hour. The expected size of ERK1 is 42 kDa. **(11D)** Phospho lysate (15 µl): 293 cells, treated with 50 mJ UV light followed by a 30-minute recovery. Non-phospho lysate (15 µl): 293 cells. The expected size of JNK1 is 46 and 54 kDa.

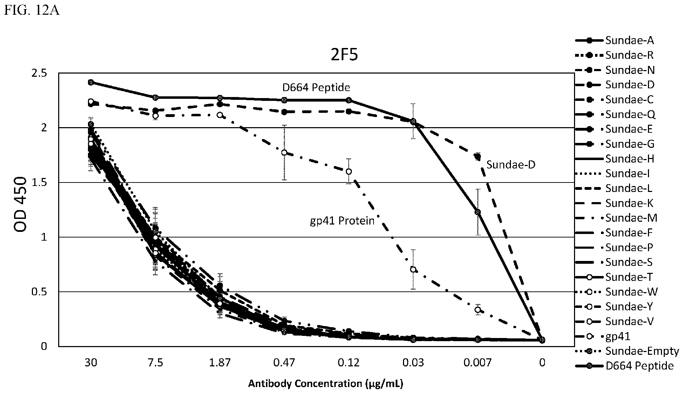

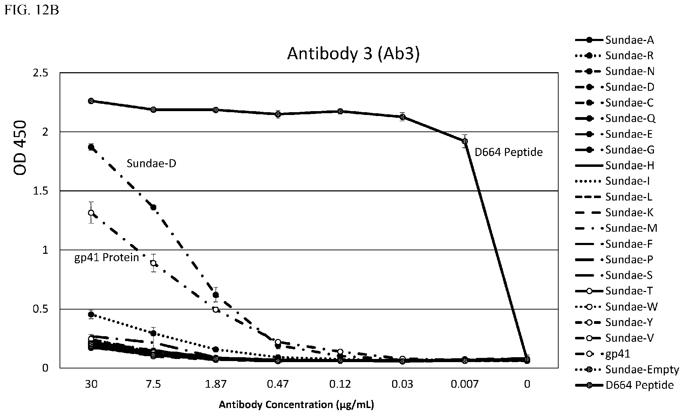

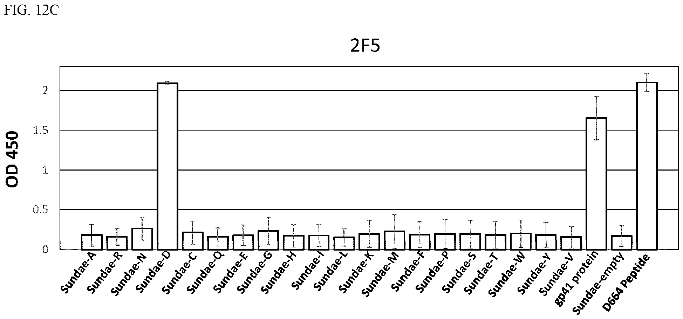

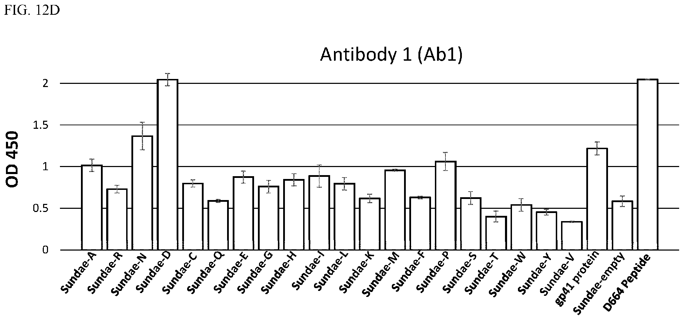

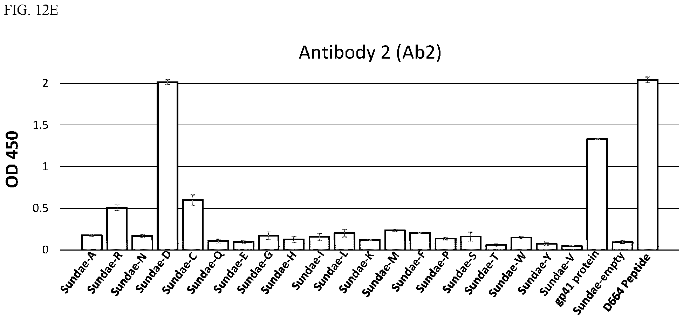

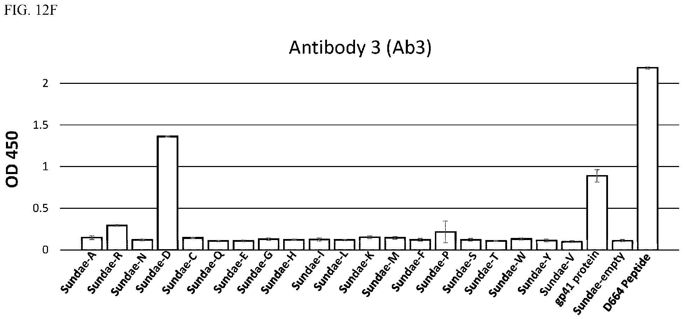

**Figures 12A****,** **12B****,** **12C****,** **12D****,** **12E****, and** **12F****. Enzyme-linked immunosorbent assay** (ELISA) **analysis of anti-membrane-proximal external region (MPER) antibodies using a panel of fusion proteins containing maltose-binding protein fused to a target peptide varying a single site with the 20 naturally occurring amino acids. (12A and 12B)** Titration enzyme-linked immunosorbent assay (ELISA) analysis (*n* = 3) of two Immunoglobulin Gs (IgGs): broadly neutralizing antibody (bnAb) 2F5 and Antibody 3 (Ab3). The data suggest that the aspartic acid (D) at position 664 is critical for 2F5 binding. **(12C-12F)** Single point enzyme-linked immunosorbent assay (ELISA) analysis (*n* = 3) of four IgGs including 2F5 (measured at 7.5 µg mL⁻¹). Antibody reactivity varies depending on which residue is present at position 664 in the peptides of the variant fusion proteins. The three different rabbit IgG clones show different reactivity when a single residue in the antibody's epitope (an aspartic acid in the native sequence) is replaced with other amino acids (Sundae-A, Sundae-E, etc.).

### DETAILED DESCRIPTION

The present disclosure is based in part upon the discovery that many B-cells have two or more V_{H}DJ_{H} or V_{L}J_{L} recombination patterns of immunoglobulin heavy chain or immunoglobulin light chain present, wherein each immunoglobulin class showed unique V_{H}DJ_{H} recombination pattern in the single B-cell. Furthermore, the discovery that the immune system's ability to generate site specific antibodies (ssAbs) can be exploited to make "site directed antibodies" (sited Abs). The method which has been termed "Epivolve", and referred to herein as site-specific immunization method, allows one to precisely target the immune system against any pre-determined site(s) on a protein target. (*See,* United States Patent Application Serial Number 17/954,037, published as U.S. Patent Application Publication No. 2023/0212271 A1, and United States Provisional Patent Application Serial Number 63/513064, each of which is incorporated by reference herein in their entirety), the contents of which are hereby incorporated by reference in its entirety). Specifically, once site-specific antibodies (ssAbs) are generated they can be matured to recognize the naturally occurring amino acid. Surprisingly and unexpectedly, this is not restricted to the recognition of the synonymous amino acid. Alternatively, the Epivolve method can be used to derive antibodies with promiscuity-that is, antibodies that can specifically bind two or more different amino acids at a defined variant site within the antibody binding site. The method of screening and/or examining antibody specificity/promiscuity disclosed herein, which has been termed "Sundae" is also described in United States Provisional Patent Application Serial Number 63/513064, which is incorporated by reference herein in their entirety.

The method described herein can be used for example, to develop antibodies from B-cells that produce two or more V_{H}DJ_{H} or V_{L}J_{L} recombination patterns of immunoglobulin heavy chain or immunoglobulin light chain. The ability to examine the two or more V_{H}DJ_{H} or V_{L}J_{L} recombination patterns of immunoglobulin heavy chain or immunoglobulin light chain in a single B-cell permits one to identify, and optionally produce, the antigen-binding protein that provides the effect of interest.

The description provides a method of producing an antigen-binding protein (e.g., an antigen-binding region, antibody, immunoglobulin (Ig), immunoglobulin G (IgG), or antigen-binding fragment or portion thereof, such as scFv or Fab), the method comprising, consisting essentially of, or consisting of: (i) cloning from a single B-cell or B-lymphocyte (I) at least one (e.g., 1, 2, 3, 4, 5, 6, 7, 8, 9, 10, or more) immunoglobulin (Ig) heavy chain or an antigen-binding fragment thereof (e.g., a plurality of immunoglobulin (Ig) heavy chain or an antigen-binding fragment thereof) and (II) at least one (e.g., 1, 2, 3, 4, 5, 6, 7, 8, 9, 10, or more) immunoglobulin (Ig) light chain or an antigen-binding fragment thereof (e.g., a plurality of immunoglobulin (Ig) light chain or an antigen-binding fragment thereof); (ii) producing at least one (e.g., 1, 2, 3, 4, 5, 6, 7, 8, 9, 10, or more) antigen-binding protein containing/producing/expressing host cell (e.g., a plurality of antigen-binding protein producing/expressing host cells) that expresses one or more of (e.g., 1, 2, 3, 4, 5, 6, 7, 8, 9, 10, or more) a single antigen-binding protein (e.g., antigen-binding region, antibody, immunoglobulin (Ig), or antigen-binding fragment or portion thereof, such as scFv or Fab) comprising, consisting essentially of, or consisting of, one of the at least one immunoglobulin (Ig) heavy chain or an antigen-binding fragment thereof and one of the at least one immunoglobulin (Ig) light chain or an antigen-binding fragment thereof; (iii) screening or examining each of the at least one antigen-binding protein containing/producing/expressing host cell for the specificity, avidity, and/or affinity of the antigen-binding protein (e.g., antigen-binding region, antibody, immunoglobulin, or an antigen-binding fragment or portion thereof) for a protein or peptide epitope of interest to find or select at least one antigen-binding protein of interest (e.g., selecting a host cell based on the specificity, avidity, and/or affinity for the protein or peptide epitope of interest); and (iv) expressing the at least one antigen-binding protein of interest in a eukaryotic cell (e.g., mammalian cell, a Chinese hamster ovary cell, or a HEK293 cell); and (v) optionally isolating or purifying the at least one antigen-binding protein.

In any aspect or embodiment described herein, the step of cloning from a single B-cell or B-lymphocyte (I) at least one (e.g., a plurality of) immunoglobulin (Ig) heavy chain and (II) at least one (e.g., a plurality of) immunoglobulin (Ig) light chain comprises, consisting essentially of, or consisting of: (a) cloning the at least one (e.g., 1, 2, 3, 4, 5, 6, 7, 8, 9, 10, or more) immunoglobulin (Ig) heavy chain or an antigen-binding fragment or portion thereof into a first expression vector to produce at least one (e.g., 1, 2, 3, 4, 5, 6, 7, 8, 9, 10, or more) heavy chain expression vector or construct (e.g., plasmid) (e.g., a plurality of heavy chain expression vectors or construct or plasmid); (b) cloning the at least one (e.g., 1, 2, 3, 4, 5, 6, 7, 8, 9, 10, or more) immunoglobulin (Ig) light chain or an antigen-binding fragment or portion thereof into a second expression vector to produce at least one (e.g., 1, 2, 3, 4, 5, 6, 7, 8, 9, 10, or more) light chain expression vector or construct (e.g., plasmid) (e.g., a plurality of light chain expression vectors or construct or plasmid); or (c) a combination thereof.

In any aspect or embodiment described herein, the method further comprises, consists essentially of, or consists of: (i) isolating and/or extracting (e.g., isolating and/or extracting the deoxyribonucleic acid (DNA) of) the at least one heavy chain expression vector; (ii) isolating and/or extracting (e.g., isolating and/or extracting the deoxyribonucleic acid (DNA) of) the at least one light chain expression vector; or (iii) a combination thereof.

In any aspect or embodiment described herein, producing at least one (e.g., 1, 2, 3, 4, 5, 6, 7, 8, 9, 10, or more) antigen-binding protein containing/producing/expressing host cell that expresses one or more of a single antigen-binding protein comprising, consisting essentially of, or consisting of, pairing each of the plurality (e.g., 1, 2, 3, 4, 5, 6, 7, 8, 9, 10, or more) of immunoglobulin (Ig) heavy chain or an antigen-binding fragment thereof with each of the plurality (e.g., 1, 2, 3, 4, 5, 6, 7, 8, 9, 10, or more) of immunoglobulin (Ig) light chain or an antigen-binding fragment thereof to produce a plurality of antigen-binding protein containing/producing/expressing host cells that expresses a single antigen-binding protein.

In any aspect or embodiment described herein, producing at least one antigen-binding protein containing/producing/expressing host cell (e.g., a plurality of antigen-binding protein producing/expressing host cells) comprises, consists of, or consists essentially of: (i) transforming each of the at least one (e.g., each of the plurality of, 1, 2, 3, 4, 5, 6, 7, 8, 9, 10, or more) heavy chain expression vector into an individual host cell (e.g., a yeast/fungi cell) to produce at least one (e.g., 1, 2, 3, 4, 5, 6, 7, 8, 9, 10, or more) heavy chain containing/producing/expressing host cell (e.g., a plurality of heavy chain containing/producing/expressing host cells); (ii) transforming each of the at least one (e.g., each of the plurality of) light chain expression vector into an individual host cell (e.g., a yeast/fungi cell) to produce at least one (e.g., 1, 2, 3, 4, 5, 6, 7, 8, 9, 10, or more) light chain containing/producing/expressing host cell (e.g., a plurality of light chain containing/producing/expressing host cells); and (iii) mating one of the at least one heavy chain containing/producing/expressing host cell and one of the at least one light chain containing/producing/expressing host cell (e.g. mating each of the at least one heavy chain containing/producing/expressing host cell with each of the at least one light chain containing/producing/expressing host cell to produce a plurality antigen-binding protein containing/producing/expressing host cells that expresses one or more of a single antigen-binding protein), and optionally integrating the heavy chain expression vector and the light chain expression vector in the at least one antigen-binding protein containing/producing/expressing host cell (e.g. the plurality of antigen-binding protein containing/producing/expressing host cells).

In any aspect or embodiment described herein, (i) expressing the at least one antigen-binding protein of interest further comprises amplifying the expression vector (e.g., if the heavy chain and light chain expression vectors have been integrated) or expression vectors (e.g., the heavy chain expression vector and the light chain expression vector) in a bacterial cell(s) (e.g., *Escherichia coli* and/or transforming a bacterial cell(s) and growing the transformed bacterial cell(s)) with the expression vector or expression vectors); (ii) the method further comprises, consists essentially of, or consists of, isolating and/or extracting the expression vector (e.g., if the heavy and light chain expression vectors have been integrated) or expression vectors (e.g., the heavy chain expression vector and the light chain expression vector) for each of the at least one antigen-binding protein of interest from the transformed bacterial cell(s); (iii) expressing the at least one antigen-binding protein of interest further comprises transfecting the eukaryotic cell with the expression vector (e.g., if the heavy chain and light chain expression vectors have been integrated) or expression vectors (e.g., the heavy chain expression vector and the light chain expression vector); or (iv) a combination thereof.

In any aspect or embodiment described herein, the method further comprises, consists essentially of, or consists of, prior to cloning from a single B-cell or B-lymphocyte, selecting and/or isolating a B-cell or B-lymphocyte expressing CD19 (e.g., the B-cell or B-lymphocyte expresses at least one of CD19 and CD38 and/or the B-cell or B-lymphocyte does not express CD10) and that binds (e.g., binds with sufficient specificity, avidity, and/or affinity) the protein or peptide epitope of interest. For example, in any aspect or embodiment described herein, the step of selecting and/or isolating a B-cell or B-lymphocyte expressing CD19 (e.g., the B-cell or B-lymphocyte expresses at least one of CD19 and CD38 and/or the B-cell or B-lymphocyte does not express CD10) and that binds (e.g., binds with sufficient specificity, avidity, and/or affinity) the protein or peptide epitope of interest includes conducting a biomarker screen to identify a B-cell or B-lymphocyte subtype (e.g., a B-cell or B-lymphocyte expressing CD19 (such as, the B-cell or B-lymphocyte expresses at least one of CD19 and CD38 and/or the B-cell or B-lymphocyte does not express CD10), a naive B cell (such as, a CD19⁺CD38^{+/-}CD10⁻CD27⁻ B-cell), a memory B-cell (such as, CD19⁺CD38^{+/-}CD10⁻CD27⁺ B-cell), a plasma cell (such as, a CD19⁺CD38⁺CD10⁻ B-cell), or a combination thereof). In any aspect or embodiment described herein, the B-cell is a subpopulation of B-cell.

In any aspect or embodiment described herein, selecting and/or isolating a B-cell or B-lymphocyte comprises, consists essentially of, or consists of, selecting and/or isolating a naive B cell (e.g., a CD19⁺CD38^{+/-}CD10⁻CD27⁻ B-cell), a memory B-cell (e.g., CD19⁺CD38^{+/-}CD10⁻CD27⁺ B-cell), or a plasma cell (e.g., a CD19⁺CD38⁺CD10⁻ B-cell) that binds (e.g., binds with sufficient specificity, avidity, and/or affinity) the protein or peptide epitope of interest.

In any aspect or embodiment described herein, the first expression vector and the second expression vector have the same sequence.

In any aspect or embodiment described herein, the first expression vector and the second expression vector have different sequences.

Antibodies developed using the method of the disclosure have application in a wide variety of fields, for example, in the development of vaccines, diagnostics, biosimilars, chimeric antigen receptor T-cell (CAR-T) therapeutics, bispecific antibodies, and multispecific antibodies.

### Site-Specific Immunization Method

The site-specific immunization method (Epivolve) is based upon a unique antigen design. The antigens are designed such that they can be used *in vivo* as an immunogen to first induce a sited specific antibody immune response.

The site-specific immunization method (Epivolve) starts with the replacement of a native amino acid in an initial peptide or protein immunogen with a specifically modified non-native or non-naturally occurring amino acid (nnAA). The modified peptide or protein is used *in vivo* as an immunogen or *in vitro* as the substrate-bound target for phage display biopanning.

*In vivo,* the site-specific immunization method (Epivolve) can be used to exploit somatic hyper-mutagenesis to produce an antibody that specifically recognize the natural occurring variant sites.

*In vitro,* clones of antibodies biased against the nnAA site and its adjacent context sequence are isolated. In phage display, first the nnAA peptide is biopanned against a naive variant antibody "discovery" library to identify nnAA-peptide specific antibodies or antigen binding fragment thereof, such as scFvs. These antibodies or antigen binding fragment thereof, such as scFv, parentals are then epivolved using a method called "discovery maturation" or "DisMat". DisMat uses AXM mutagenesis under our initial discovery conditions against the native variant peptide or native variant protein. If needed, these DisMatted antibodies or antigen binding fragment thereof can be affinity matured (AffMatted) for higher affinity to the native peptide or protein.

### Immunogen Design to Generate Site-Specificity

In any aspect or embodiment described herein, the method further comprises, consists essentially of, or consists of, generating the B-cell or B-lymphocyte. Sited antibodies (sited antibodies), such as variant sited antibodies (vsited antibodies), can be produced against any site, such as a variant site, in any protein of interest. For example, in any aspect or embodiment described herein, the protein of interest can be proteins from two different species (such as human, monkey, mouse, rabbit, dog, or guinea pig). In any aspect or embodiment described herein, the target site can be for example, an amino acid site that differs between two proteins, such as viral proteins. The site-specific immunization method (Epivolve) works by employing a nonsynonymous non-native amino acid (nnAA) residue in the middle of a peptide antigen as a means of making a "targeted epitope".

The site-specification immunization method (Epivolve) employs the use of three peptides: a first peptide (the "nnAA peptide"); a second peptide( NATive 2); and a third peptide NATive 3).

### nnAA Peptide Design

The nnAA peptide utilizes a non-naturally occurring amino acid (nnAA) to generate a site-specific immune response. In any aspect or embodiment described herein, the non-native amino acid (nnAA) is phosphorylated, acetylated, isocyanated, sulfated, or nitrated. In any aspect or embodiment described herein, the non-naturally occurring amino acid include or is, for example, phosphoserine (SEP), phosphothreonine, or phosphotyrosine.

The non-naturally occurring serves as a "pseudohapten" and from a nnAA-specific antibody can be produced with the foreknowledge that the location of the antibody binding site overlaps or is adjacent to the nnAA and directly affected by the nnAA.

In any aspect or embodiment described herein, (i) the non-native amino acid (nnAA) acid is O-phosphoserine (SEP), phosphotyrosine, or phosphothreonine; (ii) the N- terminal amino acid sequence is SerGlySer, GlySerGly, GlyGlyGly, or SerSerSer; (iii) the C-terminal amino acid sequence is SerGlySer, GlySerGly, GlyGlyGly, or SerSerSer; or (iv) a combination thereof.

The position of the non-native or non-naturally occurring amino acid (nnAA) is dependent upon the specific application of the sited antibody. For example, in any aspect or embodiment described herein, for splice-site specific antibodies the non-naturally occurring amino acid (nnAA) is offset by +/- 2 position relative to the splice site (position 0), such that the antibodies can react with amino acids on both sides of the neojunction. Similarly, in any aspect or embodiment described herein, for post-translationally modified site-specific antibodies, the non-naturally occurring amino acid (nnAA) is offset by +/- 1, +/-2, or +/-3 relative to the post-translational modification site (position 0), such as glycosylation site.

Preferably, in any aspect or embodiment described herein, the non-naturally occurring amino acid (nnAA) is phosphoserine (SEP). Phosphoserine is inexpensive to incorporate into synthetic peptides, particularly systems to incorporate SEP into proteins expressed in *E. coli.* However, in any aspect or embodiment described herein, other non-naturally occurring amino acids (nnAA) may be used, such as other charged modifications including, but not limited to acetylate, isocyanate, sulfate, or nitrate.

In any aspect or embodiment described herein, the nnAA peptide is approximately 10 to 20 amino acid in length. For example, the nnAA peptide is 10,11, 12, 13, 14, 15, 16, 17, 18, 19, 20, or more amino acids in length.

The amino acid sequence of the nnAA peptide is determined by identifying the amino acid or posttranslational modification location (i.e. the "target site") in the protein of interest to which it is desired that the sited antibody is to be directed toward. The target site is referred to herein as position 0. Once the target site is identified, the nnAA peptide is produced by replacing the amino acid residue at the target site or at a position offset with respect to the target site with a non-naturally occurring amino acid (nnAA). In any aspect or embodiment described herein, the non-native amino acid (nnAA) is offset from or relative to the site of interest. In any aspect or embodiment described herein, the offset can be -4, -3, -2, -1, +1, +2, +3, +4 relative to the target site "0" (e.g., offset by +/- 1, +/-2, or +/-3 relative to the site of interest when it is a post-translational modification site (position 0); or offset by +/- 2 position relative to the site of interest when it is a splice site/junction (position 0))). The nnAA is flanked on both sides with context amino acids (i.e. the "context sequence") that are identical to the amino acid sequence on both sides of the target site or offset position. Depending upon the length of the nnAA peptide, the amino acid terminus and the carboxy terminus context sequence are both, and independently, about 5, 6, 7, 8, 9, or 10 amino acids in length.

In any aspect or embodiment described herein, to target a variant, two nnAA peptides (nnAA1 and nnAA2) are produced, each targeting a different amino acids/variant.

In any aspect or embodiment described herein, one nnAA peptide (nnAA1) and one naturally occurring amino acid (nAA) peptide (nAA2) is joined by a linker to form a nnAA1-nAA2 peptide.

In any aspect or embodiment described herein, one nnAA (nnAA2)peptide and one nAA peptide (nAA1) are joined by a linker to form a nnAA2-nAA1 peptide.

### NATive 2 and NATive 3 Peptide Design

NATive 2 is identical to the nnAA peptide except that the modified amino acid is replaced by the naturally occurring amino acid (nAA) (i.e., a nAA peptide) and has 2, 3, 4, 5, or more additional amino acid residues at both the amino terminus and the carboxyl terminus of NATive 2. These additional amino acids do not correspond to the corresponding amino acid of the protein of interest.

NATive 3 is identical to NATive 2 with the exception that the additional amino acid residues at both the amino terminus and carboxyl terminus of NATive 3 are different from that in NATive 2.

NATive-NATive 2 peptide is formed by joining nAA1 and nAA2 with a linker and the amino terminus and carboxyl terminus has 2, 3, 4, 5, or more additional amino acid residues that do not correspond to the corresponding amino acid of the protein of interest. In any aspect or embodiment described herein, the Native-Native 2 peptide linker is different from the nnAA peptides.

NATive-NATive 3 peptide is formed by joining nAA1 and nAA2 with a different linker from NATive-NATive 2 and the nnAA peptides. The amino and carboxyl terminus of NATive-NATive 3 peptide has 2, 3, 4, 5, or more additional amino acid residues that do not correspond to the corresponding amino acid of the protein of interest and are different from that of NATive-NATive 2.

In any aspect or embodiment described herein, the amino acid sequences at the amino and carboxyl terminus of NATive peptides can be for example SerGlySer, GlySerGly, GlyGlyGly, or SerSerSer.

In any aspect or embodiment described herein, peptides containing the non-naturally occurring amino acid (nnAA) are produced in an organism (e.g., bacteria, yeast/fungi, or mammalian system) capable of incorporating the non-naturally occurring amino acid. Organisms capable of incorporating the non-naturally occurring amino acids are known in the art. For example, an *E*. *coli* stain engineered to incorporate a phosphoserine at an amber stop codon or an *E.coli* strain that incorporates a modified tyrosine that can be changed to a phosphotyrosine *in vitro,* or a bacterium that is an O-phosphoserine-incorporating suppressing bacterium may be utilized. This immunogen could be a peptide fragment, a peptide genetically fused to a protein (e.g., a highly expressed protein), or the full-length protein itself.

### Methods of Producing and Identifying a Sited Antibody or a Variant Sited (vsited) Antibodies

Sited antibodies or variant sited (vsited) antibodies can be produced *in vivo* by immunizing an animal with the nnAA peptides, or *in vitro* by using the nnAA peptides to screen an antibody display library.

In any aspect or embodiment described herein, an animal is immunized by administering the nnAA peptide, NATive 2 peptide, and the protein of interest in its native three-dimensional form (i.e. folded form) (referred to herein as native protein or NP).

In any aspect or embodiment described herein, nnAA peptide, NATive 2 peptide, the NP or a combination thereof, is administered with or conjugated to one or more (e.g., 1, 2, 3, 4, 5, or more) carriers (e.g., a carrier protein or peptide, such as keyhole limpet hemocyanin (KLH), ovalbumin, or both).

In any aspect or embodiment described herein, immunizing an animal comprises either: (i) administering the nnAA peptide at least once (e.g., one, two, three, or more times), (ii) administering the NATive2 at least once (e.g., one , two, three, or more times), separately or simultaneously, and (iii) optionally, administering the NP at least once (e.g., one , two, three, or more times) after the nnAA peptide and the NATive2 peptide are administered.

In any aspect or embodiment described herein, each immunization/administration (e.g., the nnAA peptide, the NATive 2 peptide, and/or the NP, when administered) is administered at least two weeks apart (e.g., each is administered two weeks to 6 months apart from the prior administration and the subsequent administration).

In any aspect or embodiment described herein, the nnAA peptide, the NATive 2 peptide, the NP, or a combination thereof, is administered with an adjuvant (e.g., Complete Freund's Adjuvant (CFA), Incomplete Freund's Adjuvant (IFA), aluminum, monophosphoryl lipid A (MPL) and aluminum salt (AS04), oil-in-water emulsion, oil-in-water emulsion of squalene (MF59), AS03 (Vitamin E , Surfactant polysorbate 80, and squalene), MPL and QS-21 in a liposome formulation (AS01), cytosine phosphoguanine (CpG), or a combination thereof).

In any aspect or embodiment described herein, the animal is, for example, a primate (e.g., a human or a non-human primate), a rabbit, a chicken, a rat, a goat, cow, pig, or a mouse. In any aspect or embodiment described herein, the animal is a non-human animal that has a human or humanized immune system.

In any aspect or embodiment described herein, the method further comprises after immunizing (e.g., 5, 6, 7, 8, 9, 10, 11, 12, 13, 14, 15, 16, 17, 18, 19, 20, or more days post final immunization), obtaining/isolating B-cells from the animal. In any aspect or embodiment described herein, the B-cells are obtained/isolated from peripheral blood mononuclear cells (PBMCs), splenocytes. bone marrow, or any lymphoid tissue known to contain B-cells. In any aspect or embodiment described herein, the B-cells are cloned (e.g., by any method known in the art).

In any aspect or embodiment described herein, B-cell clones producing sited or vsited antibodies of interest are identified by selecting monoclonal antibodies (mAbs) that do not bind to an appropriate set of negative controls (e.g., a scrambled peptide sequence and/or any carrier protein(s) used in the immunizations) and specifically binds to the nnAA peptide, the NATive 2 peptide, and, NATive 3 peptide, as the monoclonal antibodies are presumed to be binding to the common amino acid central core. Additionally, because they bind both (i) the nnAA and (ii) the unmodified peptide sequences of the NATive 2 peptide and the NATive 3 peptide the monoclonal antibodies are deemed "nnAA-status independent"-i.e., their binding is independent of the nnAA. In any aspect or embodiment described herein, mAbs that bind to both the NATive 2 peptide and the NATive 3 peptide, but not to nnAA peptide are deemed to be binding to the central core but are "native-sequence specific".

In any aspect or embodiment described herein, vsited antibodies are obtained from a phage display library by sequentially screening the library for binding to the nnAA peptide, the NATive 2 peptide, and the NATive 3 peptide, and generating a library of clonotypes that bind all three, as these antibodies are presumed to be binding to the common amino acid central core.

In any aspect or embodiment described herein, preparing the one or more monoclonal antibodies includes: (i) sequencing the heavy and light chain of the sited or vsited antibody identified by the methods herein, (ii) cloning the sited or vsited antibody heavy chain and light chain in an expression plasmid; (iii) expressing the sited or vsited antibody expression plasmid, (iv) purifying the expressed sited or vsited antibody, or (v) a combination thereof.

In any aspect or embodiment described herein, generating the B-cell or B-lymphocyte comprises, consisting essentially of, or consisting of: (a) immunizing an animal at least once with a modified peptide having an amino acid sequence of about 8 to about 20 amino acids identical to an amino acid sequence of the protein or peptide epitope of interest that includes the amino acids of the target sequence, except wherein one internal amino acid (e.g., the site of interest or adjacent to the site of interest) has been substituted with a non-native amino acid (nnAA); and (b) boosting the animal at least once with a first unmodified peptide comprising a core amino acid sequence identical to the modified peptide of step (a), except wherein the nnAA has been substituted with the native amino acid (nAA), a first N-terminal amino acid sequence that is not native to the protein or peptide epitope of interest, and a first C-terminal amino acid sequence that is not native to the protein or peptide epitope of interest.

In any aspect or embodiment described herein, the method further comprises, consists essentially of, or consists of: (c) cloning B-cells obtained from the animal; and (d) identifying a clone of step (c) that: (i) binds to the first unmodified peptide and a second unmodified peptide comprising a core amino acid sequence that is identical to the first unmodified peptide, a second N-terminal amino acid sequence that is not native to the protein or peptide of interest, and a second C-terminal amino acid sequence that is not native to the protein or peptide epitope of interest; and (ii) does not bind to the modified peptide of step (a).

In any aspect or embodiment described herein, the method further comprises, consists essentially of, or consists of, identifying a clone that binds to the protein or peptide epitope of interest.

In any aspect or embodiment described herein, the method further comprises, consists essentially of, or consists of: (c) cloning B-cells obtained from the animal; and (d) identifying a clone of step (c) that bind to the protein or peptide epitope of interest in its native conformation.

In any aspect or embodiment described herein, the animal is a human, a rabbit, a mouse, a rat, a goat, a cow, a pig, a camelid, or a chicken.

In any aspect or embodiment described herein, the animal is a non-human animal that has a human or humanized immune system.

In any aspect or embodiment described herein, the modified peptide, the first unmodified peptide, the second unmodified peptide, or a combination thereof, is administered with an adjuvant (e.g., the adjuvant is Complete Freund's Adjuvant (CFA), Incomplete Freund's Adjuvant (IFA), aluminum, monophosphoryl lipid A (MPL) and aluminum salt (AS04), oil-in-water emulsion, oil-in-water emulsion of squalene (MF59), AS03 (Vitamin E, Surfactant polysorbate 80, and squalene), MPL and QS-21 in a liposome formulation (AS01), or cytosine phosphoguanine (CpG)).

In any aspect or embodiment described herein, the modified peptide is conjugated to one or more carriers, the first unmodified peptide is conjugated to one or more carriers, or a combination thereof.

In any aspect or embodiment described herein, generating the B-cell or B-lymphocyte comprises, consisting essentially of, or consisting of: (a) providing a modified peptide having an amino acid sequence of about 8 to about 20 amino acids identical to an amino acid sequence of the protein or peptide epitope of interest and includes the amino acids of the target sequence, except wherein the wild-type amino acid at the site of interest (e.g., a variant site) has been substituted with a non-native amino acid (nnAA); (b) screening the modified peptides against a library; (c) isolating one or more binding agents that bind to the modified peptide; (d) generating a library of clonotypes of the one or more binding agents isolated in step (c); (e) screening the library of clonotypes against: (i) the modified peptide of step (a); and (ii) a first unmodified peptide comprising a core amino acid sequence identical to the modified peptide of step (a), except wherein the non-native amino acid (nnAA) has been substituted with the wild-type amino acid (nAA) and comprising a first N-terminal amino acid sequence that is not native to the protein or peptide epitope of interest and a first C-terminal amino acid sequence that is not native to the protein or peptide epitope of interest; and (f) isolating a binding agent that bind to both the first unmodified peptide and the modified peptide, wherein the binding agent is the antigen-binding protein (e.g., antibody or an antigen-binding fragment or portion thereof) or derived therefrom.

In any aspect or embodiment described herein, generating the B-cell or B-lymphocyte comprises, consisting essentially of, or consisting of: (a) providing a modified peptide having an amino acid sequence of about 8 to about 20 amino acids identical to an amino acid sequence of the protein or peptide epitope of interest and includes the amino acids of the target sequence, except wherein the wild-type amino acid at the site of interest (e.g., a variant site) has been substituted with a non-native amino acid (nnAA); (b) screening the modified peptides against a library; (c) isolating one or more binding agents that bind to the modified peptide; (d) generating a library of clonotypes of the one or more binding agents isolated in step (c); (e) screening the library of clonotypes against: (i) the modified peptide of step (a); and (ii) a first unmodified peptide comprising a core amino acid sequence identical to the modified peptide of step (a), except wherein the non-native amino acid (nnAA) has been substituted with the wild-type amino acid (nAA) and comprising a first N-terminal amino acid sequence that is not native to the protein or peptide epitope of interest and a first C-terminal amino acid sequence that is not native to the protein or peptide epitope of interest; (f) isolating a binding agent that bind to both the first unmodified peptide and the modified peptide; (g) generating a library of clonotypes of the binding agent isolated in step (f); (h) screening the library of clonotypes of step (g) against: (i) the modified peptide; (ii) the first unmodified peptide; and (iii) a second unmodified peptide comprising a core amino acid sequence that is identical to the first unmodified peptide, a second N -terminal amino acid sequence that is not native to the protein or peptide epitope of interest, and a second C-terminal amino acid sequence that is not native to the protein or peptide epitope of interest; and (f) isolating a binding agent that binds to the first unmodified peptide and the second unmodified peptide, wherein the binding agent does not bind the modified peptide, and the binding agent is the antigen-binding protein (e.g., antibody or an antigen-binding fragment or portion thereof) or derived therefrom.

In any aspect or embodiment described herein, generating the B-cell or B-lymphocyte comprises, consisting essentially of, or consisting of: (a) providing a modified peptide having an amino acid sequence of about 8 to about 20 amino acids identical to an amino acid sequence of the protein or peptide epitope of interest and includes the amino acids of the target sequence, except wherein the wild-type amino acid at the site of interest (e.g., a variant site) has been substituted with a non-native amino acid (nnAA); (b) screening the modified peptides against a library; (c) isolating one or more binding agents that bind to the modified peptide; (d) generating a library of clonotypes of the one or more binding agents isolated in step (c); (e) screening the library of clonotypes against: (i) the modified peptide of step (a); and (ii) a first unmodified peptide comprising a core amino acid sequence identical to the modified peptide of step (a), except wherein the non-native amino acid (nnAA) has been substituted with the wild-type amino acid (nAA) and comprising a first N-terminal amino acid sequence that is not native to the protein or peptide epitope of interest and a first C-terminal amino acid sequence that is not native to the protein or peptide epitope of interest; (f) isolating a binding agent that bind to both the first unmodified peptide and the modified peptide; (g) generating a library of clonotypes of the binding agent isolated in step (f); (h) screening the library of clonotypes of step (g) against: (i) the modified peptide; (ii) the first unmodified peptide; and (iii) a second unmodified peptide comprising a core amino acid sequence that is identical to the first unmodified peptide, except wherein the wild-type amino acid (nAA) has been substituted with an amino acid corresponding to a variant and comprising a second N -terminal amino acid sequence that is not native to the protein or peptide epitope of interest and a second C-terminal amino acid sequence that is not native to the protein or peptide epitope of interest; and (i) isolating a binding agent that binds to the second unmodified peptide and does not bind the first modified peptide, wherein the binding agent is the antigen-binding protein (e.g., antibody or an antigen-binding fragment or portion thereof) or derived therefrom.

In any aspect or embodiment described herein, detecting the binding capacity of an antibody produced by the methods described herein (e.g., specific binding) is determined by any appropriate method. For example, in any aspect or embodiment described herein, enzyme-linked immunosorbent assay (ELISA) (such as, titration ELISA), flowcytometry, surface plasmon resonance (SPR, such as Biacore^{™}), bio-layer interferometry (BLI, such as Octet^{®} RED), or a combination thereof, is in detecting the binding capacity of an antibody. In any aspect or embodiment described herein, the binding affinity (K_{d}) of the antibody or antigen binding fragment thereof to the target protein is less than about 100 µM, about 10 µM, about 1 µM, about 100 nM, about 10 nM, about 1 nM, about 100 pM, about 10 pM, or about 1 pM. In any aspect or embodiment described herein, the binding affinity (K_{d}) of the antibody or antigen binding fragment thereof to the target protein is about 1 pM to about 50 nM (e.g., about 1 nM, about 2 nM, about 3 nM, about 4 nM, about 5 nM, about 6 nM, about 7 nM, about 8 nM, about 9 nM, about 10 nM, about 15 nM, about 20 nM, about 25 nM, about 30 nM, about 35 nM, about 40 nM, about 45 nM, about 50 nM, about 1 pM, about 2 pM, about 3 pM, about 4 pM, about 5 pM, about 6 pM, about 7 pM, about 8 pM, about 9 pM, about 10 pM, about 15 pM, about 20 pM, about 25 pM, about 30 pM, about 35 pM, about 40 pM, about 45 pM, about 50 pM, any values in between, or a range from any combination of the values). In any aspect or embodiment described herein, the binding affinity (K_{d}) of the antibody or antigen binding fragment thereof to the target protein is about 1 pM to about 15 nM (e.g., about 1 nM, about 2 nM, about 3 nM, about 4 nM, about 5 nM, about 6 nM, about 7 nM, about 8 nM, about 9 nM, about 10 nM, about 11 nM, about 12 nM, about 13 nM, about 14 nM, about 15 nM, about 1 pM, about 2 pM, about 3 pM, about 4 pM, about 5 pM, about 6 pM, about 7 pM, about 8 pM, about 9 pM, about 10 pM, about 11 pM, about 12 pM, about 13 pM, about 14 pM, about 15 pM, any values in between, or a range from any combination of the values).

### Method for Examining Specificity and/or Affinity of an Antigen-binding Region

In any aspect or embodiment described herein, screening or examining each of the at least one antigen-binding protein (e.g., antigen-binding region) containing/producing/expressing host cell for the specificity, avidity, and/or affinity of the antigen-binding protein (e.g., antigen-binding region) comprises, consists essentially of, or consists of: providing a set of fusion proteins that comprises two or more (e.g., 2, 3, 4, 5, 6, 7, 8, 9, 10, 11, 12, 13, 14, 15, 16, 17, 18, 19, or 20) fusion proteins, wherein each fusion protein includes a maltose-binding protein, or amylose-binding derivative thereof, fuses directly to or via a linker to a target sequence of about 8 amino acids to 18 amino acids (e.g., about 10 to about 18 amino acids, about 10 to about 16 amino acids, or 8, 9, 10, 11, 12, 13, 14, 15, 16, 17, or 18 amino acids) from the protein or peptide epitope epitope of interest; and each target sequence of the set of fusion proteins has a different proteinogenic amino acid at a site of interest in the target sequence, including (i) one that has the native amino acid of the target sequence or (ii) one or more has a native amino acid when the site of interest is a variant site; and examining or detecting which of the fusion proteins that the antigen-binding protein (e.g., antibody or an antigen-binding fragment or portion thereof) binds.

In any aspect or embodiment described herein, detecting is performed via an enzyme-linked immunosorbent assay, and each fusion protein is a different antigen of the enzyme-linked immunosorbent assay (e.g., each fusion protein is located in a different well).

In any aspect or embodiment described herein, the maltose-binding protein derivative has been engineered/modified to provide tighter binding (e.g., enhance the binding) to amylose resin; the linker is a protein linker; the linker is a protein linker, wherein the protein linker includes or consists of about 1 to about 10 amino acids (e.g., 1, 2, 3, 4, 5, 6, 7, 8, 9, or 10 amino acids, each amino acid is independently selected from glycine and serine (e.g., a GS, GSG, GSGS (SEQ ID NO: 16), GSGSG (SEQ ID NO: 17), or GSGSGS (SEQ ID NO: 18) linker), or a combination thereof); the target sequence is linked to the C-terminus of the maltose-binding protein; or a combination thereof.

In any aspect or embodiment described herein, the set of fusion proteins includes a fusion protein comprising a target sequence for two or more variant of the protein or peptide epitope of interest (e.g., each variant of the protein or peptide epitope of interest).

In any aspect or embodiment described herein, (i) the antigen-binding protein binds to a specific variant of the protein or peptide epitope of interest (e.g., a variant sited antigen-binding protein, antigen binding region, antibody, or antigen binding fragment or portion thereof), or (ii) the antigen-binding protein binds to a specific set of (e.g., 1, 2, 3, 4, 5, 6, or more) variants (e.g., all variants) of the protein or peptide epitope of interest (e.g., a pan variant antigen-binding protein, antigen binding region, antibody, or antigen binding fragment or portion thereof).

In any aspect or embodiment described herein, the antigen-binding protein binds to only a target sequence with the native amino acid at the site of interest (e.g., a sited antigen-binding protein, antigen binding region, antibody, or antigen binding fragment or portion thereof).

In any aspect or embodiment described herein, the method further comprises, consists essentially of, or consists of: (i) expressing each fusion protein via an expression vector or construct (e.g., plasmid) that expresses the fusion protein (e.g., expressing in bacteria (such as *Escherichia coli),* fungi, or eukaryotic cell (such as mammalian cell)); (ii) purifying each fusion protein from a cell (e.g., bacteria (such as *Escherichia coli),* fungi, or eukaryotic cell (such as mammalian cell)) in which it was expressed; or (iii) a combination thereof.

In any aspect or embodiment described herein, purifying comprises, consists essentially of, or consists of, for one or more of the fusion proteins (e.g., each fusion protein): (i) adding a cell lysate containing the fusion protein to a gravity flow column comprising amylose resin (e.g., the cell lysate that flows through the gravity flow column is collected and added to the gravity glow column one or more (e.g., 1, 2, 3, or 4) times); (ii) optionally washing the gravity flow column after the cell lysate is added; (iii) eluting and/or collecting the fusion protein; or (iv) a combination thereof.

In any aspect or embodiment described herein, the method further comprises, consists essentially of, or consists of, determining or quantifying the lower limit of detection of the antigen-binding protein (e.g., antibody or an antigen-binding fragment or portion thereof) by examining the protein or peptide epitope of interest at a plurality of concentrations/amount (e.g., the lower limit of detection of the antigen-binding protein is the lowest concentration/amount of the protein or peptide epitope of interest that is detected and a lower concentration/amount is not detected).

### Methods of Using Antigen-binding Proteins

It would be readily apparent to one skilled in the art that the antigen-binding protein(s) produced by the methods described herein are useful in a variety of diagnostic and therapeutic applications. For example, the antibodies produced by the methods described herein are useful in the development of vaccines, diagnostics, biosimilars, chimeric antigen receptor T-cell (CAR-T) therapies, therapeutics, bispecific antibodies, and multispecific antibodies.

A further aspect of the present disclosure provides a method of treating, preventing, and/or ameliorating at least one symptom of a disease or disorder (e.g., pneumonia, a viral infection, a bacterial infection, a coronavirus infection (such as, severe acute respiratory syndrome coronavirus 2 (SARS-CoV-2) or Coronavirus Disease 2019 (COVID-19), a Human-Immunodeficiency Virus (HIV) infection, an ebolavirus infection (e.g., Ebola virus (EBOV) infection, Sudan virus (SUDV) infection, Bundibugyo virus (BDBV) infection, Reston virus (RESTV) infection, Tai Forest virus (TAFV) infection), etc.) in a subject in need thereof. In any aspect or embodiment described herein, the method comprises: providing a subject in need thereof; and administering an effective amount of the pharmaceutical composition or formulation described herein, wherein the antigen-binding protein (e.g., antibody or an antigen-binding fragment or portion thereof) effectuates the prevention, treatment, and/or amelioration of at least one symptom of the disease or disorder.

An additional aspect of the present disclosure provides, pharmaceutical compositions or formulations comprising the antigen-binding proteins of the present disclosure. In any aspect or embodiment described herein, the pharmaceutical compositions or formulations described herein further comprises an effective amount of an excipient (e.g., an effective amount of a pharmaceutically acceptable excipient) or carrier (e.g., an effective amount of a pharmaceutically acceptable carrier). As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the antigen-binding proteins of the present disclosure, use thereof in the compositions is contemplated. Supplementary active compounds can also be incorporated into the compositions.

The description provides methods for preparing pharmaceutical compositions or formulations. Such methods comprise formulating an effective amount of a pharmaceutically acceptable carrier or excipient with one or more antigen-binding proteins (e.g., antibody or an antigen-binding fragment or portion thereof) of the present disclosure. Such compositions or formulations can further include additional active agents as described above. Thus, the present disclosure further describes methods for preparing a pharmaceutical composition or formulation.

A pharmaceutical composition or formulation of the present disclosure is formulated to be compatible with its intended route of administration. Examples of routes of administration include parenteral (e.g., intravenous, intradermal, subcutaneous, intramuscular, intraperitoneal, intranodal, and intrasplenic) administration. Solutions or suspensions used for parenteral, intradermal, or subcutaneous application can include the following components: a sterile diluent (such as water for injection, saline solution, fixed oils, polyethylene glycols, glycerine, propylene glycol, or other synthetic solvents); antibacterial agents (such as benzyl alcohol); antioxidants (such as ascorbic acid or sodium bisulfate); chelating agents (such as ethylenediamine-tetraacetic acid); buffers (such as acetates, citrates or phosphates and agents for the adjustment of tonicity such as sodium chloride or dextrubinrubi). pH can be adjusted with acids or bases, such as hydrochloric acid or sodium hydroxide. The parenteral preparation can be enclosed in ampules, disposable syringes or multiple dose vials made of glass or plastic.

Pharmaceutical compositions or formulations suitable for injectable use, in any aspect or embodiment described herein, include sterile aqueous solutions (where water soluble) or dispersions and sterile powders for the extemporaneous preparation of sterile injectable solutions or dispersions. In any aspect or embodiment described herein, for intravenous administration, suitable carriers include physiological saline, bacteriostatic water, Cremophor EL (BASF; Parsippany, N.J.) or phosphate buffered saline (PBS). In all cases, the composition or formulation must be sterile and should be fluid to the extent that easy syringability exists. It must be stable under the conditions of manufacture and storage and must be preserved against the contaminating action of microorganisms such as bacteria and fungi. In any aspect or embodiment described herein, the carrier can be a solvent or dispersion medium containing, for example, water, ethanol, polyol (for example, glycerol, propylene glycol, and liquid polyethylene glycol, and the like), and suitable mixtures thereof. In any aspect or embodiment described herein, the proper fluidity can be maintained, for example, by the use of a coating (such as lecithin), by the maintenance of the required particle size in the case of dispersion, by the use of surfactants, or a combination thereof. In any aspect or embodiment described herein, prevention of the action of microorganisms is achieved by various antibacterial and antifungal agents (for example, chlorobutanol, phenol, ascorbic acid, and the like). In any aspect or embodiment described herein, it will be preferable to include isotonic agents (for example, sugars, polyalcohols (such as mannitol or sorbitol), or sodium chloride) in the composition or formulation. In any aspect or embodiment described herein, prolonged absorption of the injectable compositions or formulations can be brought about by including in the composition or formulation an agent which delays absorption (for example, aluminum monostearate, gelatin, or a combination thereof).

Sterile injectable solutions can be prepared by incorporating the active compound (e.g., antigen-binding proteins, such as an antibody or antigen-binding fragment or portion thereof) in the required amount in an appropriate solvent with one or a combination of ingredients enumerated above, as required, followed by filtered sterilization. Generally, dispersions are prepared by incorporating the active compound into a sterile vehicle which contains a basic dispersion medium, and then incorporating the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

It is especially advantageous to formulate parenteral compositions or formulations in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the present disclosure are dictated by and directly dependent on the unique characteristics of the antigen-binding protein (e.g., antibody or antigen-binding fragment or portion thereof), and the particular therapeutic effect to be achieved, and the limitations inherent in the art of compounding, such an antigen-binding protein (e.g., antibody or antigen-binding fragment or potion thereof), for the treatment of subjects.

In any aspect or embodiment described herein, the method can further include the step of administering (e.g., to a human) a dosage from about 100 ng to about 200 mg of a therapeutic or pharmaceutical composition or formulation as described herein. In any aspect or embodiment described herein, the pharmaceutical composition or formulation as described herein may contain mannitol as carrier, and the composition or formulation is administered from 10 µg to 200 mg, preferably 20 to 100 mg, in a single administration (e.g., to a human).

In any aspect or embodiment described herein, preferred pharmaceutically acceptable carriers can comprise, for example, xanthan gum, locust bean gum, galactose, other saccharides, oligosaccharides and/or polysaccharides, starch, starch fragments, dextrins, British gum, or mixtures thereof. Advantageously, in any aspect or embodiment described herein, the pharmaceutically acceptable carrier is of natural origin. In any aspect or embodiment described herein, the pharmaceutically acceptable carrier is, or further comprise, an inert saccharide diluent selected from a monosaccharide or disaccharide (e.g., mannitol).

In any aspect or embodiment described herein, the composition further comprises at least one stabilizer (e.g., one or more of salt(s), saccharide(s), and/or amino acid(s)). In any aspect or embodiment described herein, the composition further comprises at least one surfactant. In any aspect or embodiment described herein, the composition further comprises at least one buffering agent.

The term "surfactant" as used herein refers to a pharmaceutically acceptable, surface-active agent. For example, in any aspect or embodiment described herein, a non-ionic surfactant is used. Examples of pharmaceutically acceptable surfactants include, but are not limited to, polyoxyethylen-sorbitan fatty acid esters (Tween^{®}), polyoxyethylene alkyl ethers (Brij), alkylphenylpolyoxyethylene ethers (Triton X), polyoxyethylenepolyoxypropylene copolymers (Poloxamer, Pluronic), sodium dodecyl sulphate (SDS), or mixtures thereof. In any aspect or embodiment described herein, preferred polyoxyethylene-sorbitan fatty acid esters are polysorbate 20 (polyoxyethylene sorbitan monolaureate, sold under the trademark Tween 20^{™}) or polysorbate 80 (polyoxyethylene sorbitan monooleate, sold under the trademark Tween 80^{™}). In any aspect or embodiment described herein, preferred polyethylene-polypropylene copolymers are those sold under the names Pluronic^{®} F68 or Poloxamer188^{™}. In any aspect or embodiment described herein, preferred polyoxyethylene alkyl ethers are those sold under the trademark Brij^{™}. In any aspect or embodiment described herein, preferred alkylphenylpolyoxyethylene ethers are sold under the tradename Triton X, most preferred is p-tert-octylphenoxy polyethoxyethanol (sold under the tradename Triton X-100^{™}). In any aspect or embodiment described herein, preferred surfactants for use in the present disclosure are polyoxyethylen-sorbitan fatty acid esters, preferably polysorbate 20 or polysorbate 80, most preferably polysorbate 20. In any aspect or embodiment described herein, another preferred surfactant is Poloxamer 188^{™}.

The term "buffering agent" as used herein refers to a pharmaceutically acceptable excipient, which stabilizes the pH of a pharmaceutical preparation. Suitable buffers are well known in the art and can be found in the literature. In any aspect or embodiment described herein, pharmaceutically acceptable buffers comprise, but are not limited to histidine-buffers, citrate-buffers, succinate-buffers, acetate-buffers, arginine-buffers, phosphate-buffers, or mixtures thereof. Buffering agents are thus histidine salts, citrate salts, succinate salts, acetate salts, malate salts, phosphate salts and lactate salts. Buffering agents of particular interest comprise L-histidine or mixtures of L-histidine and L-histidine hydrochloride or L-histidine acetate with pH adjustment with an acid or a base known in the art. In any aspect or embodiment described herein, the above-mentioned buffers are used in an amount of about 5 mM to about 100 mM, particularly of about 10 mM to about 30 mM, and more particularly of about 20 mM. Independently from the buffer used, in any aspect or embodiment describe herein, the pH can be adjusted to a value in the range from about 4.5 to about 7.0, and particularly to a value in the range from about 5.0 to about 6.0, and most particularly to pH 6.0.+-.0.03 with an acid or a base known in the art, e.g. hydrochloric acid, acetic acid, phosphoric acid, sulfuric acid and citric acid, sodium hydroxide and potassium hydroxide.

The term "stabilizer" as used herein refers to a pharmaceutical acceptable excipient, which protects the active pharmaceutical ingredient and/or the formulation from chemical and/or physical degradation during manufacturing, storage and application. In any aspect or embodiment described herein, stabilizers include, but are not limited to, saccharides, amino acids, polyols (e.g. mannitol, sorbitol, xylitol, dextran, glycerol, arabitol, propylene glycol, polyethylene glycol), cyclodextrines (e.g. hydroxypropyl-β-cyclodextrine, sulfobutyl-ethyl-β-cyclodextrine, β-cyclodextrine), polyethylenglycols (e.g. PEG 3000, PEG 3350, PEG 4000, PEG 6000), albumines (human serum albumin (HSA), bovine serum albumin (BSA)), salts (e.g. sodium chloride (saline), magnesium chloride, calcium chloride), and/or chelators (e.g. EDTA). In any aspect or embodiment described herein, the stabilizer is selected from the group consisting of saccharides, polyols, and amino acids. In any aspect or embodiment described herein, the one or more stabilizers is present in the formulation in an amount of about 10 mM to about 500 mM, particularly in an amount of about 140 to about 250 mM, and more particularly in an amount of about 210 mM to about 240 mM. For example, in any aspect or embodiment described herein, sucrose or trehalose are used as stabilizers in an amount of about 220 mM to about 240 mM.

The term "saccharide" as used herein includes monosaccharides and oligosaccharides. A monosaccharide is a monomeric carbohydrate which is not hydrolysable by acids, including simple sugars and their derivatives, e.g., aminosugars. Saccharides are usually in their D conformation. Examples of monosaccharides include glucose, fructose, galactose, mannose, sorbose, ribose, deoxyribose, neuraminic acid. An oligosaccharide is a carbohydrate consisting of more than one monomeric saccharide unit connected via glycosidic bond(s) either branched or in a linear chain. The monomeric saccharide units within an oligosaccharide can be identical or different. Depending on the number of monomeric saccharide units the oligosaccharide is a di-, tri-, tetra- penta- and so forth saccharide. In contrast to polysaccharides the monosaccharides and oligosaccharides are water soluble. Examples of oligosaccharides include sucrose, trehalose, lactose, maltose and raffinose. Preferred saccharides for use in the present disclosure are sucrose and trehalose (i.e. α,α-D-trehalose), most preferred is sucrose. Trehalose is available as trehalose dihydrate. In any aspect or embodiment described herein, the at least one saccharide(s) can be present in the formulation in an amount of about 10 to about 500 mM, preferably in an amount of about 200 to about 300 mM, more preferably in an amount of about 220 to about 250 mM, particularly an amount of about 220 mM or about 240 mM, most preferably in an amount of about 220 mM.

The term "amino acid" as used herein as a stabilizer refers to a pharmaceutically acceptable organic molecule possessing an amino moiety located at a-position to a carboxylic group. In any aspect or embodiment described herein, the amino acid is one or more of arginine, glycine, ornithine, lysine, histidine, glutamic acid, asparagic acid, isoleucine, leucine, alanine, phenylalanine, tyrosine, tryptophane, methionine, serine, and proline. In any aspect or embodiment described herein, the amino acid employed is the L-form. Basic amino acids, such as arginine, histidine, or lysine, are preferably employed in the form of their inorganic salts (advantageously in the form of the hydrochloric acid salts, i.e. as amino acid hydrochlorides). In any aspect or embodiment descried herein, the amino acid is methionine. In any aspect or embodiment described herein, the amino acid (such as methionine) is used at a concentration of about 5 to about 25 mM or about 10 mM.

In any aspect or embodiment described herein, the stabilizer includes or is one or more lyoprotectant. The term "lyoprotectant" as used herein refers to a pharmaceutically acceptable excipients, which protect the labile active ingredient (e.g. a protein) against destabilizing conditions during the lyophilisation process, subsequent storage and reconstitution. In any aspect or embodiment described herein, the lyoprotectants comprise, but are not limited to, the group consisting of saccharides, polyols (such as e.g. sugar alcohols), and amino acids. In any aspect or embodiment described herein, the one or more lyoprotectant is selected from the group consisting of saccharides such as sucrose, trehalose, lactose, glucose, mannose, maltose, galactose, fructose, sorbose, raffinose, neuraminic acid, amino sugars such as glucosamine, galactosamine, N-methylglucosamine ("Meglumine"), polyols such as mannitol and sorbitol, and amino acids such as arginine and glycine, or mixtures thereof. In any aspect or embodiment described herein, the one or more lyoprotectant is used in an amount of about 10 to 500 mM, preferably in an amount of about 10 to about 300 mM and more preferably in an amount of about 100 to about 300 mM.

In any aspect or embodiment described herein, the stabilizer includes or is one or more antioxidant. The term "antioxidant" as used herein refers to a pharmaceutically acceptable excipient, which prevent oxidation of the active pharmaceutical ingredient. In any aspect or embodiment described herein, the one or more antioxidant comprises, but are not limited to, ascorbic acid, gluthathione, cysteine, methionine, citric acid, and EDTA. In any aspect or embodiment described herein, the one or more antioxidant is used in an amount of about 0.01 to about 100 mM, preferably in an amount of about 5 to about 50 mM and more preferably in an amount of about 5 to about 25 mM.

In any aspect or embodiment described herein, the compositions or formulations described herein further comprise one or more tonicity agents. The term "tonicity agents" as used herein refers to pharmaceutically acceptable excipients used to modulate the tonicity of the formulation. The formulation can be hypotonic, isotonic or hypertonic. Isotonicity in general relates to the osmotic pressure of a solution, usually relative to that of human blood serum (around 250-350 mOsmol/kg). The formulation according to the present disclosure can be hypotonic, isotonic or hypertonic, but will preferably be isotonic. In any aspect or embodiment described herein, an isotonic formulation is liquid or liquid reconstituted from a solid form, e.g. from a lyophilized form, and denotes a solution having the same tonicity as some other solution with which it is compared, such as physiologic salt solution and the blood serum. In any aspect or embodiment described herein, the one or more tonicity agents includes or is selected from sodium chloride, potassium chloride, glycerine and any component from the group of amino acids or sugars, in particular glucose. In any aspect or embodiment described herein, the one or more tonicity agents is used in an amount of about 5 mM to about 500 mM. Within the stabilizers and tonicity agents there is a group of compounds which can function in both ways, i.e. they can at the same time be a stabilizer and a tonicity agent. Examples thereof can be found in the group of sugars, amino acids, polyols, cyclodextrines, polyethyleneglycols and salts. An example for a sugar which can at the same time be a stabilizer and a tonicity agent is trehalose.

The term "polyols" as used herein denotes pharmaceutically acceptable alcohols with more than one hydroxy group. In any aspect or embodiment described herein, the one or more polyols is selected from mannitol, sorbitol, glycerine, dextran, glycerol, arabitol, propylene glycol, polyethylene glycol, and combinations thereof. In any aspect or embodiment described herein, the one or more polyols is used in an amount of about 10 mM to about 500 mM, particularly in an amount of about 10 to about 250 mM and more particularly in an amount of about 200 to about 250 mM.

In any aspect or embodiment described herein, the composition or formulations described herein may further includes adjuvants (such as preservatives, wetting agents, emulsifying agents and dispersing agents). Prevention of presence of microorganisms may be ensured both by sterilization procedures, and by the inclusion of various antibacterial and antifungal agents, e.g., paraben, chlorobutanol, phenol, sorbic acid, and the like. In any aspect or embodiment described herein, the one or more preservative is used in an amount of about 0.001 to about 2% (w/v). In any aspect or embodiment described herein, the one or more preservative is selected from ethanol, benzyl alcohol, phenol, m-cresol, p-chlor-m-cresol, methyl or propyl parabens, benzalkonium chloride.

Advantageously, the present disclosure relates to a pharmaceutical composition or formulation as defined above, which is in the form of a liposome, or nano particles, or in the form of a solution. An advantageous solution is a solution comprising from 1 to 15 %, in particular about 10% of mannitol. The solution should be iso-osmolar.

### Definitions

Unless otherwise defined, all technical and scientific terms used herein have the same meaning as commonly understood by one of ordinary skill in the art to which this disclosure belongs. The terminology used in the description is for describing particular embodiments only and is not intended to be limiting of the present disclosure.

Where a range of values is provided, it is understood that each intervening value, to the tenth of the unit of the lower limit unless the context clearly dictates otherwise, between the upper and lower limit of that range and any other stated or intervening value in that stated range is encompassed within the present disclosure. The upper and lower limits of these smaller ranges which may independently be included in the smaller ranges is also encompassed within the present disclosure, subject to any specifically excluded limit in the stated range. Where the stated range includes one or both of the limits, ranges excluding either both of those included limits are also included in the present disclosure.

As used herein, the following terms may have meanings ascribed to them below, unless specified otherwise. However, it should be understood that other meanings that are known or understood by those having ordinary skill in the art to which the present disclosure belongs are also possible, and within the scope of the present disclosure.

It must be noted that as used herein and in the appended claims, the singular forms "a", "and", and "the" include plural references (i.e., refer to one or to more than one or at least one) to the grammatical object of the article. By way of example, "an element" means one element or more than one element.

The term "about" as it is used herein, in association with numeric values or ranges, reflects the fact that there is a certain level of variation that is recognized and tolerated in the art due to practical and/or theoretical limitations. For example, minor variation is tolerated due to inherent variances in the manner in which certain devices operate and/or measurements are taken. In accordance with the above, the phrase "about" is normally used to encompass values within the standard deviation or standard error.

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e., "one or the other but not both") when preceded by terms of exclusivity, such as "either," "one of," "only one of," or "exactly one of."

In the claims, as well as in the specification above, all transitional phrases such as "comprising," "including," "carrying," "having," "containing," "involving," "holding," "composed of," and the like are to be understood to be open-ended (that is, to mean including but not limited to). It is expressly contemplated that all embodiments, and claims reciting one of the open-ended transitional phrases can be written with any other transitional phrase, which may be more limiting, unless clearly precluded by the context or art. Only the transitional phrases "consisting of" and "consisting essentially of" shall be closed or semi-closed transitional phrases, respectively, as set forth in the United States Patent Office Manual of Patent Examining Procedures, Section 2111.03.

As used herein in the specification and in the claims, the phrase "at least one," in reference to a list of one or more elements, should be understood to mean at least one element selected from anyone or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a nonlimiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, in certain methods described herein that include more than one step or act, the order of the steps or acts of the method is not necessarily limited to the order in which the steps or acts of the method are recited unless the context indicates otherwise.

The term "antibody" as used herein in the specification and claims includes whole antibodies and any antigen binding fragment (e.g., "antigen-binding portion") or single chains thereof. For example, in any aspect or embodiment described herein "antibody" as used herein in the specification and claims refers to a protein or immunoglobulin comprising at least two heavy chains (H chains) and two light chains (L chains) connected or stabilized by disulfide bonds. Each heavy chain is comprised of a heavy chain variable region (V_{H}) and a heavy chain constant region (C_{H}). Each light chain is comprised of a light chain variable region (V_{L}) and a light chain constant region (C_{L}). The heavy chain-constant region comprises three heavy chain-constant domains (C_{H1}, C_{H2}, and C_{H3}) or four heavy chain-constant domains (IgM-type or IgE-type antibodies; C_{H1}, C_{H2}, C_{H3} and C_{H4}) wherein the first constant domain C_{H1} is adjacent to the variable region and may be connected to the second constant domain CH₂ by a hinge region. The light chain-constant region consists only of one constant domain. The variable regions can be further subdivided into regions of hypervariability, termed complementarity determining regions (CDRs), interspersed with regions that are more conserved, termed framework regions (FR), wherein each variable region comprises three CDRs (CDR1, CDR2, and CDR3) and four FRs (FR1, FR2, FR3, and FR4), arranged from amino-terminus to carboxy-terminus in the following order: FR1, CDR1, FR2, CDR2, FR3, CDR3, FR4. The constant regions of the antibodies can mediate the binding of the immunoglobulin to host tissues or factors, including various cells of the immune system (e.g., effector cells) and the first component (C1q) of the classical complement system. The variable regions of the heavy and light chains contain a binding domain that interacts with an antigen. The heavy chain constant regions may be of any type, such as γ-, δ-, α-, µ.- or ε-type heavy chains. In any aspect or embodiment described herein, the heavy chain constant region of the antibody is a γ-chain. Furthermore, the light chain constant region may also be of any type, such as κ- type light chain or λ-type light chain. In any aspect or embodiment, the light chain constant region of the antibody is a κ-chain.

The terms "y- (δ-, α-, µ- or ε-) type heavy chain" and "κ- (λ-) type light chain" refer to antibody heavy chains or antibody light chains, respectively, which have constant region amino acid sequences derived from naturally occurring heavy or light chain constant region amino acid sequences, especially human heavy or light chain constant region amino acid sequences. In particular, the amino acid sequence of the constant domains of a γ-type (especially γ1-type) heavy chain is at least 95%, especially at least 98%, identical to the amino acid sequence of the constant domains of a human y (especially the human γ1) antibody heavy chain. Furthermore, the amino acid sequence of the constant domain of a κ-type light chain is in particular at least 95%, especially at least 98%, identical to the amino acid sequence of the constant domain of the human K antibody light chain. The constant regions of the antibodies may mediate the binding of the immunoglobulin to host tissues or factors, including various cells of the immune system (e.g., effector cells) and the first component (C1q) of the classical complement system. For example, the antibody can be a humanized, human, or chimeric antibody.

The term "isotype" as used herein in the specification and claims refers to the antibody class (e.g., IgG, IgD, IgA, IgM, or IgE) that is encoded by the heavy chain constant region genes (y-, δ-, α-, µ- or ε-heavy chain constant genes, respectively).

The "antigen-binding portion" or "antigen-binding fragment" of an antibody as used herein in the specification and claims usually refers to full length or one or more fragments (e.g., antigen-binding fragment) of an antibody that retains the ability to specifically bind to an antigen. It has been shown that the antigen-binding function of an antibody can be performed by fragments of a full-length antibody. Examples of binding fragments of an antibody include a Fab fragment, a monovalent fragment consisting of the V_{L}, V_{H}, C_{L} and C_{H1} domains; a F(ab)₂ fragment, a bivalent fragment comprising two Fab fragments, each of which binds to the same antigen, linked by a disulfide bridge at the hinge region; a Fd fragment consisting of the V_{H} and C_{H1} domains; a Fv fragment consisting of the V_{L} and V_{H} domains of a single arm of an antibody; and a dAb fragment, which consists of a V_{H} domain.

The term "Fab part" as used herein in the specification and claims refers to a part of the antibody comprising the heavy and light chain variable regions (V_{H} and V_{L}) and the first domains of the heavy and light chain constant regions (C_{H1} and C_{L}). In cases where the antibody does not comprise all of these regions, then the term "Fab part" only refers to those of the regions V_{H}, V_{L}, C_{H1} and C_{L} which are present in the antibody. In certain embodiments, "Fab part" refers to that part of an antibody corresponding to the fragment obtained by digesting a natural antibody with papain which contains the antigen binding activity of the antibody. In particular, the Fab part of an antibody encompasses the antigen binding site or antigen binding ability thereof. For example, the Fab part comprises at least the V_{H} region of the antibody.

The term "Fc part" as used herein in the specification and the claims is a part of the antibody comprising the heavy chain constant regions 2, 3 and--where applicable--4 (C_{H2}, C_{H3} and C_{H4}). In particular, the Fc part comprises two of each of these regions. In cases where the antibody does not comprise all of these regions, then the term "Fc part" only refers to those of the regions O_{H2}, H₃ and C_{H4} which are present in the antibody. Preferably, the Fc part comprises at least the C_{H2} region of the antibody. Preferably, "Fc part" refers to that part of an antibody corresponding to the fragment obtained by digesting a natural antibody with papain which does not contain the antigen binding activity of the antibody. In particular, the Fc part of an antibody is capable of binding to the Fc receptor and thus, e.g. comprises an Fc receptor binding site or an Fc receptor binding ability.

The term "antibody" as used herein in the specification and claims, refer in certain embodiments to a population of antibodies of the same kind. In particular, all antibodies of the population exhibit the features used for defining the antibody. In any aspect or embodiment described herein, all antibodies in the population have the same amino acid sequence.

The term "antibody" as used herein in the specification and claims also includes fragments and derivatives of said antibody. A "fragment or derivative" of an antibody as used herein in the specification and claims is a protein or glycoprotein which is derived from said antibody and is capable of binding to the same antigen, in particular to the same epitope as the antibody. Thus, a fragment or derivative of an antibody herein generally refers to a functional fragment or derivative. In any aspect or embodiment described herein, the fragment or derivative of an antibody comprises a heavy chain variable region. It has been shown that the antigen-binding function of an antibody can be performed by fragments of a full-length antibody or derivatives thereof. Examples of fragments of an antibody include (i) Fab fragments, monovalent fragments consisting of the variable region and the first constant domain of each the heavy and the light chain; (ii) F(ab)₂ fragments, bivalent fragments comprising two Fab fragments linked by a disulfide bridge at the hinge region; (iii) Fd fragments consisting of the variable region and the first constant domain C_{H1} of the heavy chain; (iv) Fv fragments consisting of the heavy chain and light chain variable region of a single arm of an antibody; (v) scFv fragments, Fv fragments consisting of a single polypeptide chain; (vi) (Fv)₂ fragments consisting of two Fv fragments covalently linked together; (vii) a heavy chain variable domain; and (viii) multibodies consisting of a heavy chain variable region and a light chain variable region covalently linked together in such a manner that association of the heavy chain and light chain variable regions can only occur intermolecular but not intramolecular. In any aspect or embodiment described herein, derivatives of an antibody include antibodies that bind to or compete with the same antigen as the parent antibody, but which have a different amino acid sequence than the parent antibody from which it is derived. These antibody fragments and derivatives are obtained using conventional techniques known to those with skill in the art, and the fragments are screened for utility in the same manner as are intact antibodies. The term "chimeric antibody" as used herein in the specification and claims is an antibody having a heavy chain variable region and a light chain variable region from one species linked to a heavy chain constant region and a light chain constant region from a different species, respectively (e.g., combining genetic material from a nonhuman source with genetic material from a human being).

The term "human antibody" as used herein in the specification and claims includes antibodies having variable regions in which both the FR and CDR regions are derived from human germline immunoglobulin sequences. Furthermore, if the antibody contains a constant region, the constant region also is derived from human germline immunoglobulin sequences. The human antibodies of the present disclosure can include amino acid residues not encoded by human germline immunoglobulin sequences (e.g., mutations introduced by random or site-specific mutagenesis *in vitro* or by somatic mutation *in vivo).* However, the term "human antibody" as used herein the specification and claims does not include antibodies in which CDR sequences derived from the germline of another mammalian species have been grafted onto human framework sequences.

The term "mouse antibody" as used herein in the specification and claims includes antibodies having variable regions in which both the FR and CDR regions are derived from mouse germline immunoglobulin sequences. Furthermore, if the antibody contains a constant region, the constant region also is derived from mouse germline immunoglobulin sequences. The mouse antibodies of the present disclosure can include amino acid residues not encoded by mouse germline immunoglobulin sequences (e.g., mutations introduced by random or site-specific mutagenesis *in vitro* or by somatic mutation *in vivo*)*.* However, the term "mouse antibody" as used herein in the specification and claims does not include antibodies in which CDR sequences derived from the germline of another mammalian species have been grafted onto mouse framework sequences.

The term "rabbit antibody" as used herein in the specification and claims includes antibodies having variable regions in which both the FR and CDR regions are derived from mouse germline immunoglobulin sequences. Furthermore, if the antibody contains a constant region, the constant region also is derived from mouse germline immunoglobulin sequences. The mouse antibodies of the disclosure can include amino acid residues not encoded by mouse germline immunoglobulin sequences (e.g., mutations introduced by random or site-specific mutagenesis *in vitro* or by somatic mutation *in vivo*). However, the term "mouse antibody" as used in the specification and claims does not include antibodies in which CDR sequences derived from the germline of another mammalian species have been grafted onto mouse framework sequences.

The term "humanized antibody" as used herein in the specification and claims refers to an antibody from non-human species whose protein sequences have been modified to increase similarity to antibody variants produced naturally in humans, such as an amino acid sequence characteristic of an antibody derived from a non-human has replaced a corresponding position of a human antibody. Examples of the humanized antibody include an antibody having heavy chain CDR1 to CDR3 and light chain CDR1 to CDR3 derived from an antibody prepared from a non-human antibody and in which all other regions comprising respective four framework regions (FRs) of the heavy chain and the light chain are derived from a human antibody. Such an antibody may be referred to as a CDR-grafted antibody. The term "humanized antibody" may include a human chimeric antibody. A "human chimeric antibody" is an antibody based on an antibody derived from a non-human in which a constant region of the antibody derived from a non-human has been replaced with a constant region of a human antibody. For increasing the antibody-dependent cellular cytotoxicity (ADCC) activity of the human chimeric antibody, for example, the subtype of the human antibody used for the constant region can be IgG1.

A "monoclonal antibody" is an antibody produced by a single clone of B lymphocytes from mouse or rabbit or by a cell, e.g., HEK293 cell, into which the light and heavy chain genes of a single antibody have been transfected. Monoclonal antibodies are produced by methods known to those of ordinary skill in the art, for instance by making hybrid antibody-forming cells from a fusion of myeloma cells with immune spleen cells. These fused cells and their progeny are termed "hybridomas." Monoclonal antibodies include humanized monoclonal antibodies.

The term "human monoclonal antibody" as used herein in the specification an claims refers to a monoclonal antibody that has variable regions in which both the FR and CDR regions are derived from human germline immunoglobulin sequences.

The term "isolated antibody" as used herein the specification and claims refers to an antibody that is substantially free of other antibodies having different antigenic specificities (e.g., an isolated bispecific antibody as described herein that specifically binds the protein of interest). Moreover, an isolated antibody can be substantially free of other cellular material and/or chemicals.

A target amino acid sequence is "derived" from or "corresponds" to a reference amino acid sequence if the target amino acid sequence shares a homology or identity over its entire length with a corresponding part of the reference amino acid sequence of at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99%. In any aspect or embodiment described herein, a target amino acid sequence which is "derived" from or "corresponds" to a reference amino acid sequence is 100% homologous, or in particular 100% identical, over its entire length with a corresponding part of the reference amino acid sequence. The "homology" or "identity" of an amino acid sequence or nucleotide sequence is preferably determined according to the present disclosure over the entire length of the reference sequence or over the entire length of the corresponding part of the reference sequence that corresponds to the sequence that homology or identity is defined. An antibody derived from a parent antibody which is defined by one or more amino acid sequences, such as specific CDR sequences or specific variable region sequences, in particular is an antibody having amino acid sequences, such as CDR sequences or variable region sequences, which are at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% homologous or identical, especially identical, to the respective amino acid sequences of the parent antibody. In any aspect or embodiment described herein, the antibody derived from (i.e. derivative of) a parent antibody comprises the same CDR sequences as the parent antibody, but differs in the remaining sequences of the variable regions.

By "homology" is meant two or more nucleic acid or amino acid sequences is partially or completely identical. In any aspect or embodiment described herein, the homologous nucleic acid or amino acid sequence has 30%, 40%, 50%, 60%, 70%, 80%, 90%, or 95% sequence similarity or identity to an nucleic acid encoding the reference nucleic acid or amino acid sequence.

"Homologs" can be naturally occurring or created by artificial synthesis of one or more nucleic acids or polypeptides having related sequences, or by modification of one or more nucleic acid or amino acid to produce related nucleic acid or amino acid sequences. If the homology between two nucleic acid or amino acid sequences is not expressly described, homology can be inferred by a nucleic acid or amino acid comparison between two or more sequences. If the sequences demonstrate some degree of sequence similarity, for example, greater than about 30% at the primary amino acid structure level, it is concluded that they share a common ancestor. For purposes of the present disclosure, genes are homologous if the nucleic acid or amino acid sequences are sufficiently similar to allow recombination and/or hybridization under low stringency conditions. In addition, polypeptides are regarded as homologous if their nucleic acid sequences are sufficiently similar to allow recombination or hybridization under low stringency conditions.

The term "specific binding" as used in the specification and claims preferably means that an agent such as an antibody, binds stronger to a target, such as an epitope for which it is specific, compared to the binding to another target. An agent binds stronger to a first target compared to a second target if it binds to the first target with a dissociation constant (K_{d}) which is lower than the dissociation constant for the second target. Preferably the dissociation constant for the target to which the agent binds specifically is more than 100-fold, 200-fold, 500-fold, or 1000-fold lower than the dissociation constant for the target to which the agent does not bind specifically. Furthermore, the term "specific binding" in particular indicates a binding affinity between the binding partners with an affinity constant Kₐ of at least 10⁶ M⁻¹, preferably at least 10⁷ M⁻¹, more preferably at least 10⁸ M¹. An antibody specific for a certain antigen in particular refers to an antibody that is capable of binding to said antigen with an affinity having a Kₐ of at least 10⁶ M⁻¹, preferably at least 10⁷ M⁻¹, more preferably at least 10⁸ M^{.-1}.

The present disclosure provides, in any aspect or embodiment described herein, an antibody that binds to a protein of interest, wherein a dissociation rate constant K_{d} thereof is not less than a first specified value and an association rate constant Kₐ thereof is not less than a second specified value. The first specified value may be 1 X 10⁻⁴ s⁻¹ or more, 2 X 10⁻⁴ s⁻¹ or more, 3 X 10⁻⁴ s⁻¹ or more, 4 X 10⁻⁴ s⁻¹ or more, 5 X 10⁻⁴ s⁻¹ or more, 6 X 10⁻⁴ s⁻¹ or more, 7 X 10⁻⁴ s⁻¹ or more, or 8 X 10⁻⁴ s⁻¹ or more. The second specified value may be 1 X 10⁻⁴ M⁻¹s⁻¹ or more, 1.5 X 10⁻⁴ M⁻¹s⁻¹ or more, or 2 X 10⁻⁴ M⁻¹s⁻¹ or more. K_{D} of the antibody may be 1 nM to 100 nM, 10 nM to 50 nM, 20 nM to 40 nM, or 30 nM to 40 nM.

Combinations of the first specified value and the second specified value are as follows: the second specified value may be 1 X 10⁻⁴ or more and the first specified value may be 1.times.10⁻⁴ s⁻¹ or more; the second specified value may be 1.5 X 10⁻⁴ M⁻¹s⁻¹ or more and the first specified value may be 2 X 10⁻⁴ s⁻¹ or more; or the second specified value may be 2 X 10⁻⁴ M⁻¹s⁻¹ or more and the first specified value may be 3 X 10⁻⁴ s⁻¹ or more. The upper limit of Kₐ and K_{d} may be within the range of Kₐ and K_{d} of antibodies obtained as described herein.

The present disclosure provides an antibody that binds to a target protein or protein of interest, wherein a dissociation rate constant K_{d} thereof is 5 X 10⁻⁴ s⁻¹ or more and an association rate constant Kₐ thereof is 1 X 10⁻⁴ M⁻¹s⁻¹ or more. The dissociation rate constant K_{d} may be, for example, 1 X 10⁻⁴ s⁻¹ or more, 2 X 10⁻⁴ s⁻¹ or more, 3 X 10⁻⁴ s⁻¹ or more, 4 X 10⁻⁴ s⁻¹ or more, 5 X 10⁻⁴ s⁻¹ or more, 6 X 10⁻⁴ s⁻¹ or more, 7 X 10⁻⁴ s⁻¹ or more, or 8 X 10⁻⁴ s⁻¹ or more. The association rate constant Kₐ may be 1 X 10⁻⁴ M⁻¹s⁻¹ or more, 1.5 X 10⁻⁴ M⁻¹s⁻¹ or more, or 2 X 10⁻⁴ M⁻¹s⁻¹ or more. K_{D} may be 1 nM to 100 nM, 10 nM to 50 nM, 20 nM to 40 nM, or 30 nM to 40 nM. Particularly, combinations of Kₐ and K_{d} are as follows: Ka may be 1 X 10⁻⁴ M⁻¹s⁻¹ or more and K_{d} may be 1 X 10⁻⁴ s⁻¹ or more; Kₐ may be 1.5 X 10⁻⁴ M⁻¹s⁻¹ or more and K_{d} may be 2 X 10⁻⁴ s⁻¹ or more; or Kₐ may be 2 X 10⁻⁴ M⁻¹s⁻¹ or more and K_{d} may be 3 X 10⁻⁴ s⁻¹ or more. The antibody having the association rate constant and the dissociation rate constant binds to the protein of interest rapidly and also dissociates from the protein of interest rapidly. The upper limit of Kₐ and K_{d} may be within the range of Kₐ and K_{d} of antibodies obtained by immunizing an animal.

Each of the association rate constant Kₐ and the dissociation rate constant K_{d} of an antibody can be determined, for example, by surface plasmon resonance (SPR) measurement. SPR measurement for binding between an antibody and an antigen is well known and those skilled in the art will be able to calculate the association rate constant Kₐ and the dissociation rate constant K_{d} of the antibody based on a well-known technique. In SPR measurement, the association rate constant can be calculated from variation of RU in a phase in which an analyte is flowed at a fixed concentration (association phase) and then, the dissociation constant can be calculated from variation of RU in a phase in which running buffer is flowed (dissociation phase). Measurement can be performed by using single-cycle kinetics. Analysis can be performed by bivalent analysis. Curve fitting of an approximate curve to a measured SPR sensorgram can be performed by using a kinetic titration 1:1 interaction model. For details of curve fitting, one can see Karlsson, R., Katsamba, P. S., Nordin, H., Pol, E. and Myszka, D. G. (2006). "Analyzing a kinetic titration series using affinity biosensors." Anal. Biochem. 349 (1): 136-47. Additionally, assessment of the association rate constant Kₐ and the dissociation rate constant K_{d} of an antibody can be performed using surface-based (heterogeneous) methods including SPR, biolayer interferometry (BLI) and enzyme linked immunosorbent assays (ELISA). Schuck 1997; Gauglitz 2008; Friguet et al. 1985.

The association rate constant Kₐ and the dissociation rate constant K_{d} of an antibody can also be determined, for example, by using an SPR instrument, such as Biacore^{™} commercially available from GE Healthcare, according to the manufacturer's manual. For example, the SPR measurement instrument also includes a program for determining Kₐ and K_{d} and can calculate Kₐ and K_{d} from an SPR sensorgram. For example, an SPR sensorgram obtained by a Biacore^{™} instrument can be subjected to analysis in which Biacore T200 evaluation software is used and a bivalent analyte model is adopted as a fitting model, thereby deriving a fitting curve, from which Kₐ, K_{d}, and KD as kinetics parameters of an antibody or an ADC can be calculated.

In any aspect or embodiment described herein, a competitive assay can be used to test whether antibodies have binding properties similar to each other. An antibody that competes with a certain antibody for binding to an antigen thereof can be identified for example by a competitive assay well known to those skilled in the art. When an antibody can block binding of a desired antibody to an antigen thereof, for example, by at least 20%, preferably at least 20 to 50%, further preferably at least 50%, more preferably 60%, more preferably 70%, more preferably 80%, and especially preferably 90% or more, in the competitive assay, the antibody can be identified as an antibody that competes for binding to the same antigen. In any aspect or embodiment described herein, a competitive antibody can be identified by a cross-blocking assay or a competitive ELISA assay. In the cross-blocking assay, an antigen is coated onto, for example, a microtiter plate, and a competitive antibody entity as a candidate is added thereto and incubated to allow binding between the antigen and the candidate antibody to form. Subsequently, the desired antibody is labelled, then added additionally to the well, incubated, and washed. One can determine whether the candidate antibody competed or not by quantifying the amount of the desired antibody that is bound. When competition exists, the amount of the label remaining in the well should be decreased.

Generally, in the competitive assay, the fact that a first antibody causes dissociation of binding of a second antibody to an antigen does not always mean that the second antibody causes dissociation of binding of the first antibody to the antigen. This can be easily understood by imagining a case where the first antibody shows extremely strong binding to the antigen compared to the second antibody. Identification of an antibody having a similar binding property may be achieved by confirming that the first antibody causes dissociation of binding of the second antibody to an antigen and the second antibody causes dissociation of binding of the first antibody to the antigen. Herein, such a competitive state is referred to as "the first antibody and the second antibody mutually compete with each other for binding to an antigen."

The terms "co-administration" and "co-administering" or "combination therapy" refer to both concurrent administration (administration of two or more therapeutic agents at the same time) and time varied administration (administration of one or more therapeutic agents at a time different from that of the administration of an additional therapeutic agent or agents), as long as the therapeutic agents are present in the patient to some extent, preferably at effective amounts, at the same time. In any aspect or embodiment described herein, one or more of the present antibodies or antigen-binding fragments thereof described herein, are coadministered in combination with at least one additional bioactive agent, especially including an anticancer agent. In any aspect or embodiment described herein, the co-administration of compounds results in synergistic activity and/or therapy, including anticancer activity.

The term "effective amount/dose," "pharmaceutically effective amount/dose," "pharmaceutically effective amount/dose," or "therapeutically effective amount/dose" can mean, but is in no way limited to, that amount/dose of the active pharmaceutical ingredient sufficient to prevent, inhibit the occurrence, ameliorate, delay or treat (alleviate a symptom to some extent, preferably all) the symptoms of a condition, disorder or disease state. The effective amount depends on the type of disease, the composition used, the route of administration, the type of mammal being treated, the physical characteristics of the specific mammal under consideration, concurrent medication, and other factors which those skilled in the medical arts will recognize. Generally, an amount between 0.1 mg/kg and 1000 mg/kg body weight/day of active ingredients is administered dependent upon potency of the agent. Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds that exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects. The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the present disclosure, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

The term "pharmacological composition," "therapeutic composition," "therapeutic formulation," or "pharmaceutically acceptable formulation" can mean, but is in no way limited to, a composition or formulation that allows for the effective distribution of an agent provided by the present disclosure, which is in a form suitable for administration to the physical location most suitable for their desired activity, e.g., systemic administration.

The term "pharmaceutically acceptable" or "pharmacologically acceptable" can mean, but is in no way limited to, entities and compositions that do not produce an adverse, allergic or other untoward reaction when administered to an animal, or a human, as appropriate.

The term "pharmaceutically acceptable carrier" or "pharmacologically acceptable carrier" can mean, but is in no way limited to, any and all solvents, dispersion media, coatings, antibacterial and antifungal agents, isotonic and absorption delaying agents, and the like, compatible with pharmaceutical administration. Suitable carriers are described in the most recent edition of Remington's Pharmaceutical Sciences, a standard reference text in the field, which is incorporated herein by reference. Preferred examples of such carriers or diluents include, but are not limited to, water, saline, finger's solutions, dextrose solution, and 5% human serum albumin. Liposomes and non-aqueous vehicles such as fixed oils may also be used. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

The term "systemic administration" refers to a route of administration that is, e.g., enteral or parenteral, and results in the systemic distribution of an agent leading to systemic absorption or accumulation of drugs in the blood stream followed by distribution throughout the entire body. Suitable forms, in part, depend upon the use or the route of entry, for example oral, transdermal, or by injection. Such forms should not prevent the composition or formulation from reaching a target cell (i.e., a cell to which the negatively charged polymer is desired to be delivered to). For example, pharmacological compositions injected into the blood stream should be soluble. Other factors are known in the art, and include considerations such as toxicity and forms which prevent the composition or formulation from exerting its effect. Administration routes which lead to systemic absorption include, without limitations: intravenous, subcutaneous, intraperitoneal, inhalation, oral, intrapulmonary, and intramuscular. The rate of entry of a drug into the circulation has been shown to be a function of molecular weight or size. The use of a liposome or other drug carrier comprising the compounds of the present disclosure can potentially localize the drug, for example, in certain tissue types, such as the tissues of the reticular endothelial system (RES). A liposome formulation which can facilitate the association of drug with the surface of cells, such as, lymphocytes and macrophages is also useful.

The term "conservative substitution" or "conservative mutation" as used herein in the specification and claims refers to replacement of an amino acid by an amino acid of similar structure (such as size) and characteristics or chemical nature, such as where a hydrophobic amino acid is replaced by another hydrophobic amino acid (e.g., replacing a leucine with an isoleucine). In studies of sequence variations in families of naturally occurring homologous proteins, certain amino acid substitutions are more often tolerated than others, and these are often show correlation with similarities in size, charge, polarity, and hydrophobicity between the original amino acid and its replacement, and such is the basis for defining "conservative substitution" or "conservative mutation". In any aspect or embodiment described herein the term "conservative mutations" or "conservative substitutions" can refer to the substitution, deletion or addition of nucleic acids that alter, add or delete a single amino acid or a small number of amino acids in a coding sequence where the nucleic acid alterations result in the substitution of a chemically similar amino acid. Amino acids that may serve as conservative substitutions for each other include the following: Basic: Arginine (R), Lysine (K), Histidine (H); Acidic: Aspartic acid (D), Glutamic acid (E), Asparagine (N), Glutamine (Q); hydrophilic: Glycine (G), Alanine (A), Valine (V), Leucine (L), Isoleucine (I); Hydrophobic: Phenylalanine (F), Tyrosine (Y), Tryptophan (W); Sulfur-containing: Methionine (M), Cysteine (C). In addition, sequences that differ by conservative variations are generally homologous.

The term "patient" or "subject" is used throughout the specification to describe an animal, preferably a human or a domesticated animal, to whom treatment, including prophylactic treatment, with the compositions according to the present disclosure is provided. For treatment of those infections, conditions or disease states which are specific for a specific animal such as a human patient, the term patient refers to that specific animal, including a domesticated animal such as a dog or cat or a farm animal such as a horse, cow, sheep, etc. In general, in the present disclosure, the term patient refers to a human patient unless otherwise stated or implied from the context of the use of the term.

The term "therapeutically effective amount or dose" includes a dose of a drug that is capable of achieving a therapeutic effect in a subject in need thereof. For example, a therapeutically effective amount of a drug can be the amount that is capable of preventing or relieving one or more symptoms associated with a disease or disorder, e.g., tissue injury or muscle-related disease or disorder. The exact amount can be ascertainable by one skilled in the art using known techniques (see, e.g., Lieberman, Pharmaceutical Dosage Forms (vols. 1-3, 1992); Lloyd, The Art, Science and Technology of Pharmaceutical Compounding (1999); Pickar, Dosage Calculations (1999); and Remington: The Science and Practice of Pharmacy, 20th Edition, 2003, Gennaro, Ed., Lippincott, Williams & Wilkins).

A kit is any manufacture (e.g. a package or container) comprising at least one reagent, e.g. a probe, for specifically detecting a marker of the present disclosure. The manufacture may be promoted, distributed, or sold as a unit for performing the methods of the present disclosure. The reagents included in such a kit comprise antibodies for use in treating a disease and/or disorder and/or detecting/binding the protein of interest. In addition, the kits of the present disclosure may preferably contain instructions which describe the therapeutic use of the antibodies and/or a suitable detection assay utilizing the antibodies. Such kits can be conveniently used, e.g., in clinical settings, to diagnose or treating patients **exhibiting** symptoms of a disease and/or disorder.

### EXAMPLES

### EXAMPLE 1.

**Media and Solutions.** All solutions were prepared using ultrapure water that was prepared by purifying deionized water to attain a sensitivity of 18 MΩ-cm at 25°C. All reagents were prepared and stored at room temperature unless indicated otherwise.

Rich Broth: deionized water, to 950 mL, Tryptone, 10 g, NaCl, 5 g, Yeast extract, 5 g. The above reagents were combined and mixed until the solutes were dissolved. The volume of the solution was adjusted to 1 L with deionized water and autoclaved. Before use, 2 grams of glucose was added.

Column Buffer: 20 mM Tris-HCl, pH 7.4, 200 mM NaCl, 1 mM, ethylenediaminetetraacetic acid (EDTA), 1 mM dithiothreitol (DTT).

Elution Buffer: column buffer with maltose to 10 mM (final).

10x Tris Buffered Saline (TBS): 1.5 M Sodium Chloride, 26.8 mM Potassium Chloride, and 0.25 M Tris/Tris-HCl, adjusted to pH 7.5.

1x TBS-TWEEN^{®} 20: 1x TBS with final concentration of 0.1% TWEEN^{®} 20.

3% Milk-TBST (MTBST): 1x TBST with 3% nonfat dry milk powder.

5% Bovine Serum Albumin-TBST (BSA-TBST): 1x TBST with 5% bovine serum albumin.

10x Sortase Buffer: 200 mM Tris-HCl, 1.5 M NaCl, 50 mM CaCl₂, 2 mM beta-mercaptoethanol prepared in sterile water.

2x Laemmeli + dithiothreitol (DTT): 2x Laemmli sample buffer (BioRad; Hercules, California), 200 mM DTT.

1x Phosphate Buffered Saline-TWEEN^{®} 20 (PBST):1x Phosphate Buffered Saline (PBS) with final concentration of 0.1% TWEEN^{®} 20.

**Methods.** All procedures were carried out at room temperature unless otherwise specified.

Construction of Sortase-acceptor plasmid. To begin construction of a sortase-acceptor plasmid (pAT27 or equivalent), the commercially available plasmid pMAL-c6t was purchased from New England BioLabs^{®} (catalog #: N0378S; Ipswich, Massachusetts), The pMAL-c6T vector is a replacement for pMAL^{™}-c5X Vector (NEB #N8108, Ipswich, Massachusetts). Like the pMAL-c5x vector, the pMAL-c6T vector is designed to produce maltose-binding protein (MBP) fusions in the cytoplasm. The maltose-binding protein (MBP) has been engineered for tighter binding to amylose resin. The vector expresses an N-terminal His-tagged malE gene followed by a multiple cloning site containing a *Tobacco Etch Virus* (TEV) protease recognition sequence, allowing MBP to be cleaved from the protein of interest after purification. A sortase conjugation site (LPETG, SEQ ID NO:5, encoded in the plasmid DNA as TTACCGGAAACTGGT, SEQ ID NO:6) with a preceding Gly₄Ser peptide linker (GGGGS, SEQ ID NO:7, encoded in the DNA as GGTGGCGGTGGCTCG, SEQ ID NO:8) were cloned into the parent vector in-frame with the MBP into the multiple cloning site using *EcoRI* and *NotI.* The resulting plasmid pAT27 expresses a modified maltose binding protein (modMBP) containing the sortase conjugation site in its C-terminus (Figure 6). The recombinant vector was transformed into NEBExpress^{®} Competent *E. coli* cells (New England BioLabs^{®} Catalog # C2523H; Ipswich, Massachusetts), frozen in 15% glycerol stock solution, and stored at -80 °C. Purified plasmid DNA was stored at -20 °C in Tris-ethylenediamenetetraacetic acid (EDTA) solution.

### Preparation of Modified Maltose-binding Protein (modMBP) with a C-terminal Sortase Site Inoculant.

Five mL Luria-Bertani (LB) media supplemented with ampicillin (final concentration of 100 µg mL⁻¹) in a 14 mL snapcap culture tube was inoculated with a scraping of a glycerol stock of the sortase acceptor plasmid in NEBExpress^{®} *Competent E. coli* cells. This plasmid contains a modified maltose-binding protein (modMBP) with a C-terminal sortase site. The modified maltose-binding protein (modMBP) was expressed in NEBExpress^{®} Competent *E*. *coli* cells (New England BioLabs^{®} Catalog # C2523H; Ipswich, Massachusetts). Genotype: *fhuA2 [lon] ompT gal sulA11 R(mcr-73::miniTn10-*TetS*)2 [dcm] R(zgb-210::Tn10-*TetS*) endAl delta(mcrC-mrr)114::IS10.* See Figure 6 for plasmid details. The cells were incubated with shaking at 37 °C overnight.

### Expression of Modified Maltose-binding Protein (modMBP) with a C-terminal Sortase Site.

Seventy-five mL of Rich Broth media supplemented with ampicillin (final concentration of 100 µg mL⁻¹) in a 250 mL baffled glass flask was inoculated with 750 µL of the overnight culture, and incubate with shaking at 37 °C until an OD₆₀₀ = 0.5 was reached. OD₆₀₀ was determined using a spectrophotometer. Fifteen µL of 1M isopropyl β-D-1-thiogalactopyranoside (IPTG) stock was added to each flask for a final concentration of 0.3 mM, and the false were incubated with shaking at 37 °C for an additional 2 hours. The culture was poured into one 250 mL centrifuge tube, and spun at 5,000 x g for 20 minutes at 4 °C. Supernatant was discarded and pellet frozen overnight at -80 °C.

### Purification of Modified Maltose-binding Protein (modMBP) with a C-terminal Sortase Site.

The bacterial pellets were thawed at room temperature and two mL of B-PER^{™} II Reagent Bacterial Protein Extract Reagent (ThermoFisher Scientific, Waltham Massachusetts) or up to 4 mL per gram of pellet was added to the centrifuge tube, and incubated for 15 minutes at room temperature on a three-dimensional shaker. The sample was transferred to a 45 mL centrifuge tube and placed on ice. The samples were centrifuged at 15,000 x g for 15 minutes at 4 °C to clarify the samples.

Two mL of amylose resin (New England BioLabs^{®}; Ipswich, Massachusetts) was added to each column utilized and allowed to drain via gravity. Each column was washed once with 2 mL of ultrapure water, followed by 2 mL of column buffer. The clarified sample was loaded onto the washed column by pouring, being careful to not disturb the cell pellet, and allowed to flow by gravity. The pellet was discarded. The flow through was collected in a clean tube and poured back over column a second time without collecting the flow through. Each column was washed with 12 mL column buffer.

A new sterile tube was placed under the column. One mL of elution buffer was added 3 times with each aliquot of elution buffer completely through the column before the next 1 mL was added. Each elution fraction was collected in a separate tube. The eluted protein was placed on ice and glycerol added to achieve a final concentration of 15% (333 µL of 45% glycerol stock per 1 mL eluate). The amount of protein was quantitated in the sample using a BioTek plate reader (Winooski, Vermont), or an equivalent, with elution buffer with 15% glycerol used at the blank. The eluted modified maltose-binding protein (modMBP) with a C-terminal Sortase Site was stored at 4 °C for short term or flash frozen in liquid nitrogen and store at -80 °C for long term storage.

### Modified Maltose-Binding Protein Conjugation to Peptide.

### Conjugation Controls.

The presence of the hemagglutinin (HA) tag (YPYDVPDYA; SEQ ID NO:9) in a conjugated reaction and the absence of HA tag in the unconjugated sample was confirmed.

Positive control: anti-HA (Abcam, Cambridge, United Kingdom) that recognizes the C-terminal HA tag on the peptide was used as a secondary antibody in a separate blot to confirm that the conjugation reaction was successful.

Negative control: unconjugated modified maltose-binding protein (modMBP) with a C-terminal Sortase Site was loaded in one lane per blot to confirm that the primary antibody binding was specific to conjugated peptide and not to the modified maltose-binding protein (modMBP) with a C-terminal Sortase Site.

### Peptide design.

Two peptides were designed and ordered per target being tested: (i) one peptide was a modified peptide containing the N-terminal sortase compatible site and a C-terminal HA tag, and (ii) the second peptide was a non-modified version containing both an N-terminal sortase compatible site and a C-terminal HA tag. The peptides were resuspended in dimethyl sulfoxide (DMSO) to a concentration of 10 mg mL⁻¹. An exemplary peptide set for the target AKT1_473 (a post-translational modification phospho-site) consists of the following two peptides (sortase compatible site, target sequence, HA tag): Phospho_akt1_473_HA: GGGSGSS ERRPHFPQF{pSer}YSASGTA YPYDVPDYA (SEQ ID NO:10) and Non-Phospo_akt1_473_HA: GGGSGSS ERRPHFPQFSYSASGTA YPYDVPDYA (SEQ ID NO:11.

### Modified Maltose Binding Protein Conjugation to Peptides.

Reactions were arranged in sets of three in a 96-well deep-well plate: (i) unconjugated modified maltose-binding protein (modMBP) with a C-terminal Sortase Site negative control, (ii) modified maltose-binding protein (modMBP) conjugated to the modified peptide, and (iii) modified maltose-binding protein (modMBP) conjugated to the non-modified peptide.

For wells containing unconjugated modified maltose-binding protein (modMBP) with a C-terminal Sortase Site, the following were mixed for each well: 10x Sortase buffer (11 µL), modified maltose-binding protein (modMBP) (0.145 mg), and 1x PBS to a final volume of 113 µL.

For wells containing modified maltose-binding protein (modMBP) conjugated to the modified peptide, the following were mixed for each well: modified peptide, 4 µL of a 10 mg mL⁻¹ stock (in DMSO), 10x Sortase Buffer (11 µL), modified maltose-binding protein (modMBP) (0.145 mg), Sortase Enzyme (1.5 µL) (Moradec LLC, San Diego, California), and 1x PBS to a final well volume of 113 µL.

For wells containing modified maltose-binding protein (modMBP) conjugated to the non-modified peptide, the following were mixed for each well: non-modified peptide, 4 µL of a 10 mg mL⁻¹ stock (in DMSO), 10x Sortase Buffer (11 µL), modified maltose-binding protein (modMBP) (0.145 mg), Sortase Enzyme (1.5 µL), and 1x PBS to a final well volume of 113 µL.

Plate tape was used to seal the reactions and the plates were incubate with shaking at 250 rotations per minute at 37 °C for 2 hours. After the incubation, the plate was centrifuged for 5 minutes at 2,100 x g to locate the reaction mixture at the bottom of the wells.

One-hundred and thirteen µL of 2x Laemmli + DTT buffer was added to each well, and each mixture transferred to a separate 1.7 ml centrifuge tube. The tubes containing conjugation reactions were incubated at 95 °C for 10 minutes. The reaction was then loaded in a sodium dodecyl sulfate-polyacrylamide (SDS-PAGE) gel for Western blot analysis.

### Antibody Validation Using Western Blot.

### Protein electrophoresis and membrane transfer.

For each antibody tested the following lanes were used: (1) 5 µl of a protein standard (Precision Plus from BioRad); (2) up to 15 µL (or 5 µg) of unconjugated modified maltose-binding protein (modMBP) with a C-terminal Sortase Site per lane; (3) up to 15 µL (or 5 µg) of modified peptide conjugated modified maltose-binding protein (modMBP)) per lane; (4) up to 15 µL (or 5 µg) of non-modified peptide conjugated modified maltose-binding protein (modMBP)) per lane. Each gel contained one control with 15 µL of modified maltose-binding protein (modMBP) conjugated to an irrelevant modified-HA peptide that can confirm that binding of the primary antibody is specific to the target peptide. The gels were run until the dye front reached the bottom of the gel.

The proteins in the gel were transferred to a nitrocellulose membrane according to the Western blot supplier's protocol (BioRad Protocol 10019593). Once the transfer was complete, the membranes were cut between the molecular weight standard lanes to generate separate blots for each antibody tested. The membranes were placed in separate compartments in a tray and wash with 1x TBS for 5 minutes on a platform shaker with gentle shaking. The 1x TBS was discarded, and a sufficient amount of 3% MTBST was added to cover each blot. The blots were incubated at room temperature for 1 hour on a platform shaker with gentle shaking to block the membranes.

The blocking solution was discarded, and the membranes were washed three times with 1x TBST, each wash included a 5 minute incubation at room temperature on a platform shaker with gentle shaking. The 1x TBST was removed from blots, and primary (tested) antibody added to the corresponding blot(s) at a concentration of 1 µg mL⁻¹ (or according to vendor recommendations) in 5% BSA-TBST. For secondary only or anti-HA blots, 5% BSA-TBST was added instead of the primary (tested) antibody. The blots were incubated overnight at 4 °C on a platform shaker with gentle shaking.

### Developing and exposing the membrane.

After the overnight incubation, the solution was discarded, and the membranes washed three time with 1x TBST, each wash included a 5-minute incubation at room temperature on a platform shaker with gentle shaking. The 1x TBST was removed from the blots, and secondary antibody diluted 1:5,000 in 3% MTBST was added to the blots. The blots were incubated at room temperature for 1 hour on a platform shaker with gentle shaking.

The secondary antibody was removed from the blots, and the membranes were washed three times with 1x TBST, each wash included a 5-minute incubation at room temperature on a platform shaker with gentle shaking.

The membranes were developed with Clarity Western Enhanced Chemiluminescence (ECL) Substrate (BioRad, Hercules, California) or equivalent for at least 10 seconds. A sufficient amount of the Clarity Western Enhanced Chemiluminescence (ECL) Substrate to cover the surface of the membrane was added to each blot. The membranes were imaged.

### Interpretation of data.

The expected band size of the modified maltose-binding protein (modMBP) is 42 kDa (Figure 4A). As an example, a phospho-specific antibody should only bind the phospho-conjugated modified maltose-binding protein (modMBP). Phospho-independent antibodies should bind to both phospho- and nonphospho-conjugated modified maltose-binding protein (modMBP). Non-phospho specific antibodies should bind only to the nonphospho-conjugated modified maltose-binding protein (modMBP). No antibodies should bind to the unconjugated modified maltose-binding protein (modMBP).

### Construction of Genetically Encoded Target Sequence Plasmid.

To begin construction of an expression plasmid comprising a maltose-binding protein linked to a target sequence (pAT28), the commercially available plasmid pMAL-c6T was purchased from New England BioLabs^{®} (catalog #: N8108S; Ipswich, Massachusetts). A sequence of 10 to 14 amino acids from the target of interest preceded by a GSGS linker was cloned into the parent vector in-frame with the maltose-binding protein (MBP) into the multiple cloning site using *NotI* and *EcoRI.* An example of two expression plasmids for the membrane-proximal external region (MPER) target found in the human immunodeficiency virus HIV consists of the following (pMAL-c6T sequence, GS linker, target sequence):
MPER-Sundae-D : MLMGGR GSGS EQELLEL**D**KWASLW (SEQ ID NO:12)
MPER-Sundae-E: MLMGGR GSGS EQELLEL**E**KWASLW (SEQ ID NO: 13)

The resulting plasmid pAT28 expresses a modified maltose-binding protein (modMBP) containing a short sequence of the target of interest at its C-terminus (also referred to as a modified maltose-binding protein (modMBP) containing or with a target sequence, modified maltose-binding protein (modMBP)-target sequence, or "Sundae"). In order to further probe antibody binding to a specific residue in a target sequence, a set of 20 plasmids that express modified maltose-binding protein (modMBP) containing a target sequence was constructed, each with a different amino acid at the site of interest (Figure 4B). The vectors were transformed into NEBExpress^{®} Competent *E. coli* cells (New England BioLabs^{®} Catalog # C2523H; Ipswich, Massachusetts), frozen in 15% glycerol stock solution, and stored at -80 °C. Purified plasmid DNA is stored at -20 °C in Tris-ethylenediamenetetraacetic acid (EDTA) solution.

### Preparation of Inoculant of Modified Maltose-binding Protein (modMBP) with a target sequence.

Five mL Luria-Bertani (LB) media supplemented with ampicillin (final concentration of 100 µg mL⁻¹) in a 14 mL snapcap culture tube was inoculated with a scraping of a glycerol stock of each modified maltose-binding protein (modMBP) with a target sequence plasmid (or Sundae plasmid) in NEBExpress^{®} Competent *E. coli* cells. The modified maltose-binding protein (modMBP) with a target sequence was expressed in NEBExpress^{®} Competent *E. coli* cells (New England BioLabs^{®} Catalog # C2523H; Ipswich, Massachusetts). Genotype: *fhuA2 [lon] ompT gal sulA11 R(mcr-73::miniTnl0--TetS)2 [dcm] R(zgb-210::Tnl0--TetS) endAl delta(mcrC-mrr)114::IS10.* The cells were incubated with shaking at 37 °C overnight.

### Expression of Modified Maltose-binding Protein (modMBP) with a target sequence.

Seventy-five mL of Rich Broth media supplemented with ampicillin (final concentration of 100 µg mL⁻¹) in a 250 mL baffled glass flask was inoculated with 750 µL of each of the overnight culture, and incubate with shaking at 37 °C until an OD₆₀₀ = 0.5 was reached. OD₆₀₀ was determined using a spectrophotometer. Fifteen µL of 1M isopropyl β-D-1-thiogalactopyranoside (IPTG) stock was added to each flask for a final concentration of 0.3 mM, and the false were incubated with shaking at 37 °C for an additional 2 hours. Each culture was poured into one 250 mL centrifuge tube, and spun at 5,000 x g for 20 minutes at 4 °C. Supernatant was discarded and pellet frozen overnight at -80 °C.

### Purification of Modified Maltose-binding protein (modMBP) with a target sequence.

The bacterial pellets were thawed at room temperature and two mL of B-PER^{™} II Reagent Bacterial Protein Extract Reagent (ThermoFisher Scientific, Waltham Massachusetts) or up to 4 mL per gram of pellet was added to the centrifuge tube, and incubated for 15 minutes at room temperature on a three-dimensional shaker. Each sample was transferred to a 45 mL centrifuge tube and placed on ice. The samples were centrifuged at 15,000 x g for 15 minutes at 4 °C to clarify the samples.

Two mL of amylose resin (New England BioLabs^{®}; Ipswich, Massachusetts) was added to each column utilized and allowed to drain via gravity. Each column was washed once with 2 mL of ultrapure water, followed by 2 mL of column buffer. Each clarified sample was individually loaded onto a washed column by pouring, being careful to not disturb the cell pellet, and allowed to flow by gravity. The pellet was discarded after pouring off the supernatant. The flow through was collected in a clean tube and poured back over column a second time without collecting the flow through. Each column was washed with 12 mL of column buffer.

A new sterile tube was placed under the column. One mL of elution buffer was added 3 times with each aliquot of elution buffer completely through the column before the next 1 mL was added. Each elution fraction was collected in a separate tube. The eluted protein was placed on ice and glycerol added to achieve a final concentration of 15% (333 µL of 45% glycerol stock per 1 mL eluate). The amount of protein was quantitated in the sample using a BioTek plate reader (Winooski, Vermont), or an equivalent, with elution buffer with 15% glycerol used at the blank. The eluted modified maltose-binding protein (modMBP) with a target sequence was stored at 4 °C for short term or flash frozen in liquid nitrogen and store at -80 °C for long term storage.

### Antibody Validation Using Modified Maltose-binding Protein (modMBP) with a target sequence enzyme-linked immunosorbent assay (ELISA).

The wells of a 96-well microtiter plate were coated with 50 µL of the modified maltose-binding Protein (modMBP) with a target sequence (antigen) diluted to 1 µg mL⁻¹ in 1x PBS. The plates were sealed and incubated overnight at 4°C. After the incubation, the plates were washed 3 times with 1x PBS and each well blocked the wells with 50 µL of 2% BSA diluted in 1x PBS. The plates were Incubated for 1 hour, and then washed 3 times with 1x PBS.

Primary antibody (50 µL) was added to each well with a 4-fold dilution in 2% BSA that began at 30 µg mL⁻¹. The plates were incubated for 1 hour, and then washed 3 times with 1x PBST. Anti-Protein A horseradish peroxidase (HRP) conjugated antibody was added to each well (50 µL at a 1:5,000 dilution). The plates were incubated for 1 hour, and then washed 3 times with 1x PBST. Room temperature 3,3',5,5' tetramethylbenzidine (TMB) was added (50 µL) to each well and allowed to develop for 5 to 10 minutes. The reaction was quenched with the additional of 50 µL of 0.16 M H₂SO₄ to each well. Absorbance at 450 nm was read on a plate reader.

**Discussion.** To develop reproducible results, it is critical that all reagents used in an experiment be validated in an alternative or independent method. Here, two such independent methods for determining the specificity of antibodies: (1) maltose-binding protein (MBP) conjugated to the modified or non-modified peptide with a C-terminal HA tag (referred to as "MILKSHAKE"), which can be used to validate the liability and specificity of antibodies directed against post-translationally-modified epitopes, and (2) a set of maltose-binding proteins (MBPs) containing a target sequence, wherein each target sequence in the set has a different amino acid at the site of interest (referred to herein as "Sundae"), which is a more complete alanine-like scanning method that can be used to better understand the binding and bioactivity of specific residues of a protein. Both methods were applied to the interaction between an antibody and its antigen.

A current cause for concern in the scientific literature is the poor reproducibility of published experimental results because of a lack of rigor in reagent validation and inter-experimental consistency. Even beyond affinity reagent-antigen binding, the reproducibility issue drastically increases in complexity when validating antibodies against post-translationally modified (PTM) targets. Often antibodies are sold as being post-translational modification specific, however when tested under the recommended protocol appear to have some affinity to the non-modified state of the epitope. Thus, a real difficulty in validating any anti-post-translationally modified protein antibodies is access to a validated antigen or cell lysate. It is especially difficult to generate cell lysates and purified antigen wherein the modified amino acid in the protein is present at either 100% (fully modified) or 0% (fully non-modified) in the sample. Even when a particularly specific and targeted treatment is known for a particular post-translational modification, other variables such as batch to batch variation, cell health, modifying-enzyme activity, storage conditions, and researcher technique can persist to allow for errors in an appropriate validation.

Alanine scanning is a technique that is often used to determine the contribution of a specific amino acid residue to the stability or function of a given protein. Alanine is used because it is non-bulky and chemically inert. The methyl group of alanine can be used to mimic the secondary structure preferences that the other amino acids possess. Sometimes bulky amino acids, such as the branched chain amino acids valine and leucine, are used where the conservation of the size of mutated residues is needed.

Alanine scanning is further used to determine whether a specific amino acid residue plays a significant role in bioactivity by replacing the naturally occurring amino acid with alanine. This can often be performed without the requirement for highly purified protein and often uses the method of site-directed mutagenesis to change the residues in question to alanine. Once the change is made, the protein can be retested for bioactivity with the alanine replaced. Often these tests can yield a quantitative measurement. However, one limitation to alanine scanning is that it only reveals whether alanine as a replacement at a specific site retains bioactivity. It reveals nothing about the effect of the other 18 amino acids (i.e., the set of 20 naturally occurring (i.e., proteinogenic) amino acids minus the native and alanine residues). So a true picture of the importance of a specific residue for interaction or bioactivity cannot be painted if only alanine is used for scanning (Figure 4B).

As such, the most reliable method for validation of antibodies, including anti-post-translational modification-specific antibodies, is one in which each antigen sample contains the target sequence with either 100% modification or 100% non-modification at the residue or epitope of interest. The protocol that utilizes modified maltose-binding protein (MBP) conjugated to the modified or non-modified peptide with a C-terminal hemagglutinin tag (also referred to herein as the MILKSHAKE protocol) provides that certainty for a Western blot assay. This protocol uses a maltose binding protein (MBP) and the sortase enzyme to produce surrogate proteins containing the modified or non-modified epitope sequence (Figure 5). These proteins are then used in Western blots to evaluate the post-translation modification specificity of antibodies.

To determine more completely the binding contribution of a specific amino acid at a specific site, a protocol was developed that utilizes a set of maltose-binding Protein (MBP) with a target sequence, each of the set has a different amino acid at the site of interest of the target sequence (also referred to herein as the "Sundae" protocol because we are using multiple 'flavors' of amino acids at the site of interest, not just alanine). Using this protocol, the targeted site is replaced with up to 20 different amino acids (Figure 6) and retested for bioactivity. This method is able to distinguish varying reactivity to each amino acid in enzyme-linked immunosorbent assay (ELISA) (Figure 7).

### EXAMPLE 2.

**Summary of Example 2.** Knowing that an antibody's sensitivity and specificity in a desired application is accurate is crucial for reliable data collection and thus, antibody development. There is great concern about the reliability and specificity of antibodies, particularly when applied to post-translationally modified proteins. Two validation methods are described herein that use surrogate proteins in western blot analysis and enzyme-linked immunosorbent assay (ELISA) analysis. The first method, which is referred to herein as "MILKSHAKE" is a modified maltose-binding protein that is enzymatically conjugated to a peptide from the chosen target, which is either modified or non-modified at the residue of interest. The certainty of the residue's modification status can be used to confirm antibody specificity to the target's post-translational modification (PTM) status. A second method described uses a surrogate protein set, which is referred to herein as "Sundae". Sundae consists of a modified maltose-binding protein set with a genetically encoded target sequence, each of which contains a single amino acid substitution at one position of interest. With Sundae, antibodies can be evaluated for binding specificities to all twenty amino acids at a single position. Antibody specificity can be determined at a single-residue resolution by combining the two methods. These data aid in evaluation of off-target effects for research-use-only antibodies and therapeutic antibodies.

**Introduction.** Antibodies are an essential tool in biomedical research for the detection and quantification of target proteins. Post-translational modifications (PTMs), such as phosphorylation, acetylation, and glycosylation, are critical for regulating protein function and are involved in many disease processes. Post-translational modifications can make it challenging to develop specific antibodies that differentiate between the modified or the native isoform of the protein, leading to false positive or negative results. There is great concern about the reliability and specificity of antibodies, particularly when applied to post-translationally modified proteins.

To help address challenges observed with validating antibodies, two antibody validation methods are described and utilized herein: a Western blot method (referred to herein as "MILKSHAKE") focuses on post-translational modification-specific epitopes and an enzyme-linked immunosorbent assay (referred to herein as "Sundae") thoroughly explores the antibody-antigen interface at a one-residue resolution.

In the western blot method, a maltose-binding protein (MBP), such as modified maltose binding protein (modMBP), is fused or conjugated to a peptide containing the moiety of interest using, such as sortase A conjugation. The resulting protein is used in western blot to validate the specificity of antibodies to the modified or non-modified epitope. This method works with other types of post-translational modifications as well. This example focuses on phosphoproteins, which account for the majority of antibodies directed against post-translational modifications that are cited in literature. Specific binding to the maltose-binding protein (MPB) conjugated peptide in western blot correlates with binding to full length target proteins found in treated cell lysates. In addition, comparing the binding of an antibody to the MBP conjugated peptide (or MILKSHAKE protein) and to a treated cell lysate in western blot can verify the quality of the lysate treatment itself.

The enzyme-linked immunosorbent assay method, or Sundae method, is a superior means of understanding the paratope-epitope interaction at one-residue resolution as compared with known, simpler methods, such as alanine scanning. Alanine scanning is a technique that is often used to test the contribution of a specific amino acid residue to the function of a given protein. Alanine is used because it is composed of the shortest sidechain among the 20 proteinogenic (or naturally occurring) amino acids, showing the least electronic potential, and chemically inert for contacting with other residues. The alanine methyl group can be used to mimic the secondary structure preferences that the other amino acids possess. Sometimes bulky amino acids, such as the branched chain amino acids valine and leucine, are used where the size conservation of mutated residues is needed. Often these tests can yield a quantitative measurement. However, one limitation to alanine scanning that is rarely discussed and underappreciated is that it only reveals whether alanine as a replacement at a specific site retains or loses bioactivity. It reveals nothing about the effect of the other 18 amino acids (i.e., the set of 20 naturally occurring or proteinogenic amino acids minus the native and alanine residues).

The enzyme-linked immunosorbent assay method consists of a maltose-binding protein (MBP), such as a modified maltose binding protein (modMBP), containing a sequence of interest genetically encoded in the C-terminus. The Sundae term stems from the fact that multiple "flavors" of amino acids are being used at the position of interest on the epitope being tested and fused to the maltose-binding protein. Twenty different peptides ('flavors') fused with the maltose-binding protein (MBP) are produced, each peptide has the same residue replaced with a different amino acid. The resulting proteins can be used in an enzyme-linked immunosorbent assay (ELISA) to validate the binding profile of an antibody to each amino acid substitution. To demonstrate the power of the enzyme-linked immunosorbent assay (ELISA) method, this example focuses on evaluating the binding specificity profile of 2F5, a broadly neutralizing monoclonal antibody (bnAb) that targets Human Immunodeficiency Virus (HIV).

**Materials and Methods.** Enzymes sortase, *Not I,* and *EcoRI,* ligase were purchased from the manufacturer as indicated and used under the conditions specified.

Constructions of Sortase-acceptor Plasmid. A plasmid expressing a modified maltose binding protein (modMBP) was constructed by inserting a C-terminal sortase conjugation site with a preceding Gly₄Ser (G₄S) linker into the maltose binding protein expression vector pMAL-c6T (New England BioLabs^{®}, Ipswich, Massachusetts) (inserted sequence: GGTGGCGGTGGCTCGTTACCGGAAACTGGT (SEQ ID NO: 14)) using the *Not I* and *EcoR I* restriction sites.

Plasmid DNA construction-Sundae. A plasmid expressing modified maltose binding protein (modMBP) was constructed by inserting a sequence from the target of interest (10-14 amino acids) preceded by a (GlySer)₂ linker into the maltose binding protein expression vector pMAL-c6T using the *Not* I (New England BioLabs^{®}; Ipswich, Massachusetts) and *EcoR* I (New England BioLabs^{®}; Ipswich, Massachusetts) restriction sites.

Protein Purification. modMBP was purified from NEBExpress^{®} Competent *E. coli* cells (New England BioLabs^{®}; Ipswich, Massachusetts) cultures. Cultures were grown at 37 °C until an OD₆₀₀ = 0.5 was reached. Cultures were then induced with 0.2 mM isopropyl β-D-1-thiogalactopyranoside (IPTG) (AmericanBio, Inc.; Canton, Massachusetts) for an additional 1 hour. Cultures were centrifuged at 4,000 x g for 20 minutes at 4 °C. Pellets were frozen overnight at -80 °C. The following day the cells were thawed at room temperature and lysed using 2 mL of B-PER^{™} II Reagent Bacterial Protein Extract Reagent (ThermoFisher Scientific, Waltham Massachusetts). Protein was purified via gravity flow using amylose resin (New England BioLabs^{®}; Ipswich, Massachusetts) for immobilization and 10 mM maltose (MilliporeSigma Burlington, Massachusetts) for elution. Protein was stored at 4 °C for short term and -80 °C for long term in 15% glycerol.

Peptide Design. Peptides (Biopeptek Pharmaceuticals LLC, Malvern, Pennsylvania) contain an N-terminal sortase conjugation site (GlyGlyGlySerGlySerSer [GGGSGSS]; SEQ ID NO:3), a target-specific sequence (either phosphorylated or non-phosphorylated at a single amino acid), and a C-terminal hemagglutinin (HA) tag (TyrProTyrAspValProAspTyrAla [YPYDVPDYA]; SEQ ID NO:4).

Sortase Conjugation Reaction. A master mix was prepared for the number of reactions required and aliquoted into the wells of a 96-well deep well plate, wherein each reaction contained: 0.145 mg purified modMBP, 10x Sortase Buffer (200 mM Tris-HCl, 1.5 M NaCl, 50 mM CaCl₂, 2 mM beta-mercaptoethanol), 0.04 mg target peptide, 3 µg sortase enzyme (Moradec, San Diego, California), and 1x phosphate buffered saline (PBS) to a final volume of 113 µL. One well without target peptide or sortase enzyme was used as a negative unconjugated control. The plate was incubated with shaking at 250 rotations per minute at 37 °C for 2 hours. Reactions were then mixed with equal volumes of 2x Laemmli Sample Buffer (Bio-Rad Laboratories; Hercules, California) containing 200 mM dithiothreitol (AmericanBio, Inc.; Canton, Massachusetts) and denatured at 95 °C for 10 minutes.

Preparation of Cell Lysates. Five mL of HEK293-F cells (ThermoFisher Scientific, Waltham, Massachusetts) at 1 x 10⁶ cells per mL were pelleted by centrifugation at 2,500 x g for 5 minutes at room temperature and washed twice with 10 mL chilled 1x PBS. The pellet was resuspended in 1 mL lysis buffer-radioimmunoprecipitation assay (RIPA) buffer (MilliporeSigma; Burlington, Massachusetts) containing 3.2 mM sodium orthovanadate (New England BioLabs^{®}; Ipswich, Massachusetts) and 10 µg each leupeptin, pepstatin, aprotinin and chymostatin (MilliporeSigma). The lysate was transferred to a microcentrifuge tube and incubated on ice for 15 minutes with shaking. The resulting lysate was centrifuged at 14,000 x g for 15 minutes at 4 °C. Supernatant was collected and quantified using an A280 microplate reader. Lysates were then mixed with equal volume of 2x Laemmli Sample Buffer (Bio-Rad Laboratories; Hercules, California) containing 200 mM dithiothreitol (AmericanBio, Inc; Canton, Massachusetts) and denatured at 95 °C for 10 minutes.

Polyacrylamide gel electrophoresis. Each lane of a 4-20% polyacrylamide gel (Bio-Rad Laboratories; Hercules, California) was loaded with modMBP conjugated to a target peptide and where appropriate prepared HEK293 cell lysate. In particular, 5 µL of ladder was used, 3 µg of maltose-binding protein was used for the AKT and STAT3 low stringency ("vanilla") blots, 10 µg of Milkshake added to ERK1 and JNK1 vanilla blots, and for high stringency ("strawberry") blots, 0.5 µg of milkshake and 15 µg of HEK293 cell lysate used. Gels were run for 40 minutes at 180 volts in sodium dodecyl sulfate (SDS) buffer.

Western blot. After electrophoresis, proteins were transferred to nitrocellulose membranes using the Trans-Blot Turbo system (Bio-Rad Laboratories; Hercules, California). Membranes were washed with 1x Tris Buffered Saline (TBS) for 5 minutes at room temperature. Membranes were incubated in blocking buffer (3% Milk-Tris Buffered Saline with 0.1% TWEEN^{®} 20 (TBST)) for 1 hour at room temperature followed by washing three times for 5 minutes, each with 1x Tris Buffered Saline with 0.1% TWEEN^{®} 20 (TBST). Primary antibodies were diluted according to vendor recommended concentrations for each individual antibody (unless otherwise specified) in 5% bovine serum albumin-TBST (BSA-TBST) as shown in Tables 1-4 below, and incubated overnight at 4 °C with shaking. The following day, membranes were washed with 1x TBST three times, each for 5 minutes. Secondary horseradish peroxidase (HRP)-conjugated antibodies were diluted 1:5,000 (see Tables 1-4 below) in 3% Milk-TBST and incubated at room temperature for 1 hour. Membranes were washed with 1x TBST three times at room temperature, each for 5 minutes. Blots were visualized using Clarity Western Enhanced Chemiluminescence (ECL) Substrate (BioRad, Hercules, California) with exposure times ranging from 0.4 to 16 seconds.

**Table 1. Antibodies utilized in AKT (Ser473) Western blot analysis in Figures 9A, 10A, and 10A**

| | **AKT Primary Antibody Vendor (dilution or concentration)** | **Secondary Antibody (same vendor as primary)** |
|---|---|---|
| **Vendor A** | Cell Signal Technology, Catalog No. 9271; Danvers, Massachusetts (1:1,000) | Anti-rabbit HRP |
| **Vendor B** | Rockland Immunochemicals, Inc., Catalog No. 200-301-268; Royersford, Pennsylvania (1:20,000) | Anti-mouse HRP |
| **Vendor C** | Santa Cruz Biotechnology, Catalog No. sc293125; Dallas, Texas (1:200) | Anti-mouse HRP |
| **Vendor D** | Novus Biologicals, Catalog No. AF887; Littleton, Colorado (0.1 µg/mL) | Anti-rabbit HRP |

**Table 2. Antibodies utilized in Stat3 (Tyr705) Western blot analysis in Figures 9B, 10B, and 11B**

| | **STAT3 Primary Antibody Vendor (dilution)** | **Secondary Antibody (same vendor as primary)** |
|---|---|---|
| **Vendor A** | Cell Signal Technology, Catalog No. 9145; Danvers, Massachusetts | Anti-rabbit HRP |
| | (1:2,000) | |
| **Vendor C** | Santa Cruz Biotechnology, Catalog No. sc-8059; Dallas, Texas (1:1000) | Anti-mouse HRP |
| **Vendor D** | Novus Biologicals, Catalog No. NBP2-61588; Littleton, Colorado (1:2,000) | Anti-rabbit HRP |
| **Vendor E** | BioLegend, Catalog No. 651001; San Diego, California (1:1,000) | Anti-mouse HRP |

**Table 3. Antibodies utilized in ERK1 (Thr202/Tyr204) Western blot analysis Figures 9C, 10C, and 11C**

| | **ERK1 Primary Antibody Vendor (dilution)** | **Secondary Antibody (same vendor as primary)** |
|---|---|---|
| **Vendor A** | Cell Signal Technology, Catalog No. 9101; Danvers, Massachusetts (1:1,000) | Anti-rabbit HRP |
| **Vendor D** | Novus Biologicals, Catalog No. NB500-141; Littleton, Colorado (1:2,000) | Anti-rabbit HRP |
| **Vendor F** | Thermo Fisher Scientific, Catalog No. 36-8800; Waltham, Massachusetts (1:1,000) | Anti-rabbit HRP |
| **Vendor G** | Sigma, Catalog No. E7028; Saint Louis, Missouri (1:1,000) | Anti-rabbit HRP |

**Table 4. Antibodies utilized in JNK1 (Thr183/Tyr185) Western blot analysis Figures 9D, 10D, and 11D**

| | **JNK1 Primary Antibody Vendor (dilution or concentration)** | **Secondary Antibody (same vendor as primary)** |
|---|---|---|
| **Vendor A** | Cell Signal Technology, Catalog No. 9251; Danvers, Massachusetts (1:1,000) | Anti-rabbit HRP |
| **Vendor C** | Santa Cruz Biotechnology, Catalog No. sc-293136; Dallas, Texas (1:1000) | Anti-mouse HRP |
| **Vendor F** | Thermo Fisher Scientific, Catalog No. 44-682G; Waltham, Massachussetts (1:1,000) | Anti-rabbit HRP |
| **Vendor G** | Sigma, Catalog No. 07-175; Saint Louis, Missouri (2 µg/mL) | Anti-rabbit HRP |

Enzyme-linked Immunosorbent Assay (ELISA). Wells of a 96-well microtiter plate were coated with modified maltose-binding Protein (modMBP) with a target sequence (antigen) and blocked with 2% BSA. Primary antibody 2F5 (Polymun Scientific; Klosterneuburg, Austria) or rabbit-derived IgG was diluted in 2% BSA, added to the wells, and incubated for 1 hour. Wells were washed three times with 1x Phosphate Buffered Saline (PBS) with final concentration of 0.1% TWEEN^{®} 20 (PBST). Secondary antibody anti-Protein A-HRP (ThermoFisher Scientific, Waltham, Massachusetts) or goat anti-rabbit HRP (Jackson ImmunoResearch, West Grove, Pennsylvania) was diluted 1:5,000 and incubated for 1 hour. Wells were washed again 3x with PBST. Wells were developed with 3,3',5,5' tetramethylbenzidine (TMB) (ThermoFisher Scientific, Waltham, Massachusetts), and the reaction was quenched with 0.16 M sulfuric acid. Plate absorbance was read in a microplate reader at 450 nm.

### Results.

Surrogate proteins used for Western and ELISA antibody validation. Sortase A can accept substrates of varying structure. The first system described herein utilized a maltose-binding protein, such as a modified maltose binding protein (modMBP), with a sortase acceptor site that is conjugated or fused to a peptide that contains a moiety of interest and a sortase donor site. The resulting protein, which is referred to herein as MILKSHAKE, conjugated maltose-binding protein (MBP), or maltose-binding protein (MPB) conjugated peptide, can be used in western blot to validate the specificity of post-translation modifications specific binders, such as phospho-specific binders, to the modified or non-modified epitope of interest (Figure 8A). Positive binding to the maltose-binding protein conjugated to the peptide in a western blot correlates with binding to full length target proteins found in treated cell lysates. the maltose-binding protein (MBP) conjugated to the peptide of interest (also referred to herein as the conjugated maltose-binding protein) can be utilized in two different experiment types: western blots in which lanes are loaded only with the conjugated maltose-binding protein (MBP) as a test of the antibody's specificity (also referred to herein as "low stringency" or "vanilla") and western blots in which lanes are loaded with a mixture of conjugated maltose-binding protein (MBP) and HEK293 cell lysate as a more stringent test of the antibody's sensitivity (referred to herein as, "high stringency" or "strawberry").

The maltose-binding protein (MBP) fused peptide, or Sundae proteins, contain a genetically-encoded epitope target sequence fused to the C-terminus of maltose-binding protein, such as a modified maltose-binding protein (modMBP). In each maltose-binding protein (MBP) fused peptide, one residue of the peptide has been replaced by all 20 naturally occurring or proteinogenic amino acids, thereby resulting in a full set of twenty maltose-binding protein (MBP) fused peptide proteins (Figure 8B) per single amino acid targeted site. These surrogate proteins are used in single-well or titration enzyme-linked immunosorbent assay (ELISA) to determine binding affinity of antibodies when the single residue is replaced. The maltose-binding protein (MBP) fused peptide proteins shed light on which residues or what physical and chemical attributes of each residue are important for antibody binding.

Low Stringency ("Vanilla") Western Blot Method Demonstrates Antibody binding specificity to Post-translational Modifications. Using a modified maltose-binding protein (modMBP), wherein the maltose-binding protein has been engineered for tighter binding to amylose resin, and specifically designed peptides, a modified maltose-binding protein (MBP) conjugated to a peptide with a phosphorylated (single or dual depending on the target) or non-phosphorylated epitope from a desired protein. With antibodies from multiple vendors, binding was detected to the phospho-peptide conjugated to the modified maltose-binding protein versus non-phospho-peptide conjugated to the modified maltose-binding protein, and not to unconjugated modified maltose-binding protein (MBP) (Figure 9A-9D). The antibodies chosen to be examined were based upon recent antibody market data, which identified them as being among the most cited phospho-specific antibodies. These include antibodies important in cancer and diabetes research, which bind phospho-AKT (Ser473), phospho-Stat3 (Tyr705), phospho-ERK1 (Thr202/Tyr204), and phospho-JNK1 (Thr183/Tyr185). Their prevalence in published research further supports the need to properly validate these antibodies, which are sold as phospho-specific to these sites. While antibodies from top selling vendors were chosen, the company names have not been disclosed (e.g., Vendor A) mainly because the results disclosed herein are meant to illustrate the methods of the present disclosure, not as a conclusive investigation of the quality of manufacturers' antibodies after testing of a single lot. There may also be lot-to-lot variation or a performance difference if used under a different set of conditions. Most of the antibodies tested bind as expected to the phospho-peptide conjugated modified maltose-binding protein and not to the non-phospho-peptide conjugated modified maltose-binding protein (13 out of 16 antibodies tested. However, three antibodies did show some reactivity to the non-phosphorylated conjugated modified maltose-binding protein (see Figures 9A and 9D, white arrows).

High Stringency ("Strawberry") Western Blot Method Demonstrates Specificity and Sensitivity in a Cell lysate Background. Figures 10A-10D demonstrates the range of sensitivity of post-translational modification specific antibodies for their peptide target conjugated to modified maltose-binding protein when the surrogate protein is presented in a cell lysate background. In high stringency version of the Western blot method, visualization of some antibodies binding to the true target protein present in the HEK293 cell lysate was observed as well as non-specific binding to other cellular proteins in an untreated HEK293 cell lysate. For example, the AKT protein has an expected size of approximately 60 kDa. Bands corresponding to this size can be seen in all four panels of Figure 10A.

Some vendors' antibodies react to a variety of other cellular proteins whereas antibodies from competitors are highly specific (Figures 10A-10D). For example, each of the four antibodies that bind phospho-AKT (Serine 473) bind the phosphorylated peptide conjugated to the modified maltose-binding protein and not the non-phosphorylated peptide conjugated to modified maltose-binding protein with high stringency method. However, the antibodies from Vendor C shows reactivity to a variety of cellular proteins (Figure 10A, white arrows), most of which are not the expected size of the AKT protein itself (approximately 60 kDa). In addition, antibodies from Vendor G for phospho-JNK1 (Thr183/Tyr185) and for phospho-ERK1 (Thr202/Tyr204) show reactivity to other bands that do not correlate with the true sizes of those proteins as well (Figures 10C and 10D, white arrows).

High Stringency (Strawberry) Western Blot Method is a Superior Validation Method for Dual post-Translational Modified Sites Compared to Treated Cell Lysates. For validation of antibodies to be used in western analysis, the most commonly used antigen materials are mammalian cell lysates which have undergone treatment (e.g., chemical exposure, ultraviolet radiation, etc.) to induce or reduce modification of residues on a protein of interest. This material can be extremely challenging to reproduce reliably. Furthermore, many proteins which undergo phosphorylation contain dual phospho sites, which are typically in very close proximity in the amino acid sequence, thereby further complicating the validation process of antibodies. Using only a treated cell lysate for antibody validation leads to uncertainty as to which site or sites the antibody is binding.

In Figures 11A-11D, commercially available treated lysates (Cell Signaling Technology, Danvers, Massachusetts) were used to test phospho-specific antibodies and the results compared to the results from the high stringency western analysis method shown in (Figures 10A-10D). For some targets, the data are compatible. For phospho-AKT (Ser473), antibodies from Vendor A and Vendor D are phospho-specific in both assays and have very limited off-target binding. Antibodies from Vendor B and Vendor C demonstrate non-specific binding to other cellular proteins in both the high stringency western blot method and the treated cell lysates (Figures 10A and 10A).

All four phospho-Stat3 (Tyr705) antibodies tested are phospho specific and demonstrate limited off target binding in both assays (Figures 12B and 13B). The weakest binder (Vendor E) to phospho-Stat3 (Tyr705) in treated lysates was also confirmed as the weakest binder in the high stringency western blot method MILKSHAKE. It is noted that the methods described herein could be used to quantify the lower limit of detection by comparing to a quantified standard/material.

The targets containing dual phosphorylation sites tell a different story. In treated cell lysates, all four vendor antibodies for phospho-ERK1 (Thr202/Tyr204) appear to correctly recognize only the phosphorylated cell condition (Figure 11C). However, when those same antibodies were tested in the high stringency western blot method, those from Vendor F and Vendor D are not able to recognize the peptide conjugated to modified maltose-binding protein on which only Tyr204 is phosphorylated (Figure 10C, black arrows). These results demonstrates that these two antibodies are only able to bind when Thr202 is phosphorylated.

Antibodies to phosphor-JNK1 (Thr183/Tyr185) show Vendor-specific results. The antibody from Vendor F does not bind in the treated lysate even after a prolonged exposure (Figure 11D). Antibodies from Vendor A, Vendor C, and Vendor G appear phospho-specific in treated lysates for one or both of the expected sizes of the JNK1 protein, 46 and 54 kDa (Figure 11D). However, in the high stringency Western blot method these antibodies bind in three distinct ways: (1) to phospho-Tyr185 only (Vendor A and Vendor G), (2) to phospho-Thr183 only (Vendor F), or (3) to both phosphor-Thr183 and phosphor-Tyr185, albeit very weakly (Vendor C, Figure 10D). In addition, off-target binding can be observed in both assays for Vendor G's antibody to phosphor-JNK1 (Thr183/Tyr185) (Figures 10D and 11D).

Peptide Fused Maltose-binding Protein Enzyme-linked Immunosorbent Assay (ELISA) Data Demonstrate Varied Binding Profiles For Residue-specific Antibodies. The epitope for 2F5, a human broadly neutralizing monoclonal antibody (bnAb), is located on the Human Immunodeficiency Virus (HIV) envelope glycoprotein 41 (gp41), specifically on a region of the protein known as the membrane-proximal external region (MPER). The 2F5 epitope is composed of a conserved linear sequence of amino acids in MPER, which includes the sequence 662-ELDKWA-667 (SEQ ID NO:15). This region of the HIV envelope protein is involved in the fusion of the virus with host cells during infection. Thus making this region an important target for neutralizing antibodies, such as 2F5. By binding to this region, 2F5 blocks the fusion of the virus with host cells, thereby preventing infection. Previous research has shown that the aspartic acid at the 664 position is important for 2F5 binding.

To test 2F5 binding, a set of 20 single-site variant peptides were prepared, each with a different amino acid substituted at the D664 position (See Figure 8B). As expected, 2F5 binds strongly to the gp41 protein, a D664 peptide, and the Sundae-D protein, all of which contain the MPER sequence with an aspartic acid (D) at the 664 position (Figures 12A and 12C). These data confirm the importance of aspartic acid (D) at this site. The binding of three IgGs generated from rabbits immunized with the MPER sequence using the site-directed antigen binding region development/generation (also referred to herein as Epivolve technology) were tested. Two of these IgGs (Antibody 2 (Ab2) and Antibody 3 (Ab3)) show strong binding only when an aspartic acid (D) is present at the 664 position in the Sundae-D protein (Figures 12B, 12E, and 12F). However, Antibody 1 (Ab1) is able to bind when a variety of other amino acids are present at the 664 position, notably alanine, asparagine, and proline (Figure 12D).

**Discussion.** It is essential to develop reliable and specific antibodies that can detect and quantify proteins, such as post-translationally modified proteins, accurately. The western blot and enzyme-linked immunosorbent assay methods described herein provide a standardized and comprehensive approach to antibody validation, enabling the generation of more accurate and reproducible results and advance the understanding of complex biological systems.

Example 2 found that 13 out of 16 (81%) top selling antibodies for post-translational modifications tested performed as expected by binding the phosphorylated residue and not the non-phosphorylated residue in the low stringency western blot method. However, the remaining 3 antibodies (19%) tested, which bind the non-phosphorylated peptide conjugated to the modified maltose-binding protein, are cause for concern. This data demonstrates that even among commonly used vendors the quality of antibodies on offer are not always comparable or even valid. Researchers with access to data for antibodies for post-translation modifications prepare by the western blot method described herein could make a much more informed decision when selecting an antibody for their experiments.

Dual phospho-sites have an even higher bar for validation. Traditional treated lysates as a method for post-translational modification validation are not specific enough for dual or multi-post-translational modification sites because the treatments may affect each site unequally. With the western blot method of the present disclosure, the modification status of each individual residue is certain, and the binding specificity is more clearly defined. Out of the 8 dual phospho site antibodies tested, 5 antibodies (62%) were identified that appear dual-phospho-specific in a treated lysate experiment but only truly bind one (12%) phospho site in the high stringency western blot method. Being able to validate antibodies to this level of specificity may enable researchers to conduct more rigorous experiments, using multiple antibodies for these dual sites and dissect exactly which residues' modifications are important for their study. The high stringency method described herein makes this possible, even when those modified sites are just one residue apart.

The high stringency data goes a step further than post-translational modification specificity and reveals which antibodies may have reactivity to other cellular proteins. The two antibodies available from Vendor G for phospho-JNK1 (Thr183/Tyr185) and for phospho-ERK1 (Thr202/Tyr204) demonstrate that an antibody may be specific for a post-translational modification, but can also show off-target binding. The cell lysate used in the high stringency method could also be expanded to include other cell types besides HEK293. This option would enable researchers to capture the full range of potential off-target binding in the cell types they plan to use in their experiments.

Antibodies for phospho-AKT (Ser 473) from Vendor B and Vendor C showed binding to the non-phospho peptide conjugated to the modified maltose-binding protein in the lost stringency experiment (Figure 9A). However, these same antibodies have significantly reduced binding to the non-phospho peptide conjugated to the modified maltose-binding protein when they are tested in a lysate background (high stringency) where many more epitopes, including the AKT protein itself, are present (Figure 10A). The results of both the low stringency and high stringency versions may help inform antibody selection depending on the type of analyte and how much target protein might be present in the planned experiment. It should be noted that this method will also work with other types of post-translational modifications, including as examples: acetylation, methylation and glycosylation. An additional advantage of sortase is that it can be used to attach small molecules to proteins.

The enzyme-linked immunosorbent assay method described herein can be used to better understand the relationship of the antibody paratope to the immunogen epitope. The method replaces the targeted site (i.e., a single amino acid) with up to 20 different amino acids and allows for the testing of each mutated site for bioactivity. The data for the broadly neutralizing 2F5 shown here correlates with previous 2F5 research, which identifies the aspartic acid (D) at position 664 as crucial for binding. Another antibody (Antibody 1 (Ab1)), which relies less heavily on aspartic acid (D) and that is capable of binding to other residues at that same position. These residues include alanine (A), proline (P), and asparagine (N), each of which have side chains that differ from the native aspartic acid (D) both in size and charge. This method can be used to examine/determine an antibody's binding profile for each residue along the target. This type of experiment would be especially helpful for identifying "pan-variant" antibodies, which can bind multiple amino acids at the same site. This data would also be helpful to choose therapeutic and diagnostic antibodies with reduced "off-target" effects which stem from binding to very similar sequences in non-target proteins.

### LISTING OF SEQUENCES

SEQ ID NO:1 - EQELLELDKWASLW
SEQ ID NO:2 - EQELLELXKWASLW
SEQ ID NO:3 - GGGSGSS
SEQ ID NO:4 - YPYDVPDYA
SEQ ID NO:5 - LPETG
SEQ ID NO:6 - TTACCGGAAACTGGT
SEQ ID NO:7 - GGGGS
SEQ ID NO:8 - GGTGGCGGTGGCTCG
SEQ ID NO:9 - YPYDVPDYA
SEQ ID NO:10 - GGGSGSSERRPHFPQF[pSer]YSASGTAYPYDVPDYA
SEQ ID NO:11 - GGGSGSSERRPHFPQFSYSASGTAYPYDVPDYA
SEQ ID NO: 12 - MLMGGRGSGSEQELLELDKWASLW
SEQ ID NO: 13 - MLMGGRGSGSEQELLELEKW ASL W
SEQ ID NO: 14 - GGTGGCGGTGGCTCGTTACCGGAAACTGGT
SEQ ID NO: 15 - ELDKWA
SEQ ID NO:16 - GSGS
SEQ ID NO: 17 - GSGSG
SEQ ID NO:18 - GSGSGS
SEQ ID NO: 19 - QNQQEKNEQELLELDKWASLWNWFNITNWLWYIK (FIG. 1)

### OTHER EMBODIMENTS

The preceding detailed description of the present disclosure is provided to aid those skilled in the art in practicing the present disclosure. Those of ordinary skill in the art may make modifications and variations in the embodiments described herein without departing from the spirit or scope of the present disclosure. Although any methods and materials similar or equivalent to those described herein can also be used in the practice or testing of the present disclosure, the preferred methods and materials are now described. Unless otherwise defined, all technical and scientific terms used herein have the same meaning as commonly understood by one of ordinary skill in the art to which this disclosure belongs. The terminology used in the description of the present disclosure herein is for describing particular embodiments only and is not intended to be limiting of the present disclosure. All publications, patent applications, patents, figures and other references mentioned herein are expressly incorporated by reference in their entirety.

## Claims

1. A method of producing a homogenous antigen-binding protein , the method comprising:
cloning from a single B-cell or B-lymphocyte (I) at least one immunoglobulin (Ig) heavy chain or an antigen-binding fragment thereof and (II) at least one immunoglobulin (Ig) light chain or an antigen-binding fragment thereof;
producing at least one antigen-binding protein containing host cell that expresses one or more of a single antigen-binding protein comprising one of the at least one immunoglobulin (Ig) heavy chain or an antigen-binding fragment thereof and one of the at least one immunoglobulin (Ig) light chain or an antigen-binding fragment thereof;
screening or examining each of the at least one antigen-binding protein containing host cell for specificity, avidity, and/or affinity of the antigen-binding protein for a protein or peptide epitope of interest to find or select at least one antigen-binding protein; and
expressing the at least one antigen-binding protein in a eukaryotic cell.

2. The method of claim 1, wherein cloning from a single B-cell or B-lymphocyte (I) at least one immunoglobulin (Ig) heavy chain and (II) at least one immunoglobulin (Ig) light chain comprises:
(a) cloning the at least one immunoglobulin (Ig) heavy chain or an antigen-binding fragment or portion thereof into a first expression vector to produce at least one heavy chain expression vector or construct;
(b) cloning the at least one immunoglobulin (Ig) light chain or an antigen-binding fragment or portion thereof into a second expression vector to produce at least one light chain expression vector or construct; or
(c) a combination thereof.

3. The method of claim 2, further comprises:
isolating and/or extracting the at least one heavy chain expression vector;
isolating and/or extracting the at least one light chain expression vector; or
a combination thereof.

4. The method of any one of claims 1-3, wherein:
producing at least one antigen-binding protein containing host cell that expresses one or more of a single antigen-binding protein comprising pairing each of the plurality of immunoglobulin (Ig) heavy chain or an antigen-binding fragment thereof with each of the plurality of immunoglobulin (Ig) light chain or an antigen-binding fragment thereof to produce a plurality of antigen-binding protein containing host cell that expresses a single antigen-binding protein;
expressing the at least one antigen-binding protein further comprises amplifying the expression vector or expression vectors in a bacterial cell;
the method further comprises isolating and/or extracting the expression vector or expression vectors for each of the at least one antigen-binding protein from the transformed bacterial cell;
expressing the at least one antigen-binding protein further comprises transfecting the eukaryotic cell with the expression vector or expression vectors;
(i) the first expression vector and the second expression vector have the same sequence or (ii) the first expression vector and the second expression vector have different sequences; or
a combination thereof.

5. The method of any one of claims 1-4, wherein producing at least one antigen-binding protein containing host cell comprises:
transforming each of the at least one heavy chain expression vector into an individual host cell to produce at least one heavy chain containing host cell;
transforming each of the at least one light chain expression vector into an individual host cell to produce at least one light chain containing host cell; and
mating one of the at least one heavy chain containing host cell and one of the at least one light chain containing host cell, and optionally integrating the heavy chain expression vector and the light chain expression vector in the at least one antigen-binding protein containing host cell.

6. The method of any one of claims 1-5, further comprising prior to cloning from a single B-cell or B-lymphocyte, selecting and/or isolating a B-cell or B-lymphocyte expressing CD19 and that binds the protein or peptide epitope of interest, preferably a naive B cell, a memory B-cell, or a plasma cell that binds the protein or peptide epitope of interest.

7. The method of any one of claims 1-6, wherein screening or examining each of the at least one antigen-binding protein containing host cell for the specificity, avidity, and/or affinity of the antigen-binding protein comprises:
providing a set of fusion proteins that comprises two or more fusion proteins, wherein:
each fusion protein includes a maltose-binding protein, or amylose-binding derivative thereof, fuses directly to or via a linker to a target sequence of about 8 amino acids to 18 amino acids from the protein or peptide epitope of interest; and
each target sequence of the set of fusion proteins has a different proteinogenic amino acid at a site of interest in the target sequence, including (i) one that has the native amino acid of the target sequence or (ii) one or more has a native amino acid when the site of interest is a variant site; and
examining or detecting which of the fusion proteins that the antigen-binding protein binds.

8. The method of claim 7, wherein:
detecting is performed via an enzyme-linked immunosorbent assay, and each fusion protein is a different antigen of the enzyme-linked immunosorbent assay;
the maltose-binding protein derivative has been engineered/modified to provide tighter binding to amylose resin;
the linker is a protein linker;
the linker is a protein linker, wherein the protein linker includes or consists of about 1 to about 10 amino acids;
the target sequence is linked to the C-terminus of the maltose-binding protein;
the set of fusion proteins includes a fusion protein comprising a target sequence for two or more variant of the protein or peptide epitope of interest, preferably (i) the antigen-binding protein binds to a specific variant of the protein or peptide epitope of interest, or (ii) the antigen-binding protein binds to a specific set of variants of the protein or peptide epitope of interest; or
a combination thereof.

9. The method of claim 7 or 8, wherein:
the antigen-binding protein binds to only a target sequence with the native amino acid at the site of interest;
the method further comprising expressing each fusion protein via an expression vector or construct that expresses the fusion protein;
the method further comprising purifying each fusion protein from a cell in which it was expressed; or
a combination thereof.

10. The method of claim 9, wherein purifying comprises, for one or more of the fusion proteins:
adding a cell lysate containing the fusion protein to a gravity flow column comprising amylose resin;
optionally washing the gravity flow column after the cell lysate is added;
eluting and/or collecting the fusion protein; or
a combination thereof.

11. The method of any one of claims 7-10, further comprising determining or quantifying the lower limit of detection of the antigen-binding protein by examining the protein or peptide epitope of interest at a plurality of concentrations.

12. The method of any one of claims 1-11, further comprising generating the B-cell or B-lymphocyte.

13. The method of claim 12, wherein generating the B-cell or B-lymphocyte comprises:
a. immunizing an animal at least once with a modified peptide having an amino acid sequence of about 8 to about 20 amino acids identical to an amino acid sequence of the protein or peptide epitope of interest that includes the amino acids of the target sequence, except wherein one internal amino acid has been substituted with a non-native amino acid (nnAA); and
b. boosting the animal at least once with a first unmodified peptide comprising a core amino acid sequence identical to the modified peptide of step (a), except wherein the nnAA has been substituted with the native amino acid (nAA), a first N-terminal amino acid sequence that is not native to the protein or peptide epitope of interest, and a first C-terminal amino acid sequence that is not native to the protein or peptide epitope of interest;
c. cloning B-cells obtained from the animal; and
d. identifying a clone of step (c) that the protein or peptide epitope of interest in its native conformation or binds to the protein or peptide epitope of interest and/or:
i. binds to the first unmodified peptide and a second unmodified peptide comprising a core amino acid sequence that is identical to the first unmodified peptide, a second N-terminal amino acid sequence that is not native to the protein or peptide epitope of interest, and a second C-terminal amino acid sequence that is not native to the protein or peptide epitope of interest; and
ii. does not bind to the modified peptide of step (a).

14. The method of claim 13, wherein:
the animal is a human, a rabbit, a mouse, a rat, a goat, a cow, a pig, a camelid, or a chicken;
the animal is a non-human animal that has a human or humanized immune system;
the modified peptide, the first unmodified peptide, the second unmodified peptide, or a combination thereof, is administered with an adjuvant;
the modified peptide is conjugated to one or more carriers, the first unmodified peptide is conjugated to one or more carriers, or a combination thereof; or
a combination thereof.

15. The method of claim 12, wherein:
(1) generating the B-cell or B-lymphocyte comprises:
a. providing a modified peptide having an amino acid sequence of about 8 to about 20 amino acids identical to an amino acid sequence of the protein or peptide epitope of interest and includes the amino acids of the target sequence, except wherein the wild-type amino acid at the site of interest has been substituted with a non-native amino acid (nnAA);
b. screening the modified peptides against a library;
c. isolating one or more binding agents that bind to the modified peptide;
d. generating a library of clonotypes of the one or more binding agents isolated in step (c);
e. screening the library of clonotypes against:
i. the modified peptide of step (a); and
ii. a first unmodified peptide comprising a core amino acid sequence identical to the modified peptide of step (a), except wherein the non-native amino acid (nnAA) has been substituted with the wild-type amino acid (nAA) and comprising a first N-terminal amino acid sequence that is not native to the protein or peptide epitope of interest and a first C-terminal amino acid sequence that is not native to the protein or peptide epitope of interest; and
f. isolating a binding agent that bind to both the first unmodified peptide and the modified peptide, wherein the binding agent is the antigen-binding protein (e.g., antibody or an antigen-binding fragment or portion thereof) or derived therefrom;
(2) generating the B-cell or B-lymphocyte comprises:
a. providing a modified peptide having an amino acid sequence of about 8 to about 20 amino acids identical to an amino acid sequence of the protein or peptide epitope of interest and includes the amino acids of the target sequence, except wherein the wild-type amino acid at the site of interest has been substituted with a non-native amino acid (nnAA);
b. screening the modified peptides against a library;
c. isolating one or more binding agents that bind to the modified peptide;
d. generating a library of clonotypes of the one or more binding agents isolated in step (c);
e. screening the library of clonotypes against:
i. the modified peptide of step (a); and
ii. a first unmodified peptide comprising a core amino acid sequence identical to the modified peptide of step (a), except wherein the non-native amino acid (nnAA) has been substituted with the wild-type amino acid (nAA) and comprising a first N-terminal amino acid sequence that is not native to the protein or peptide epitope of interest and a first C-terminal amino acid sequence that is not native to the protein or peptide epitope of interest;
f. isolating a binding agent that bind to both the first unmodified peptide and the modified peptide;
g. generating a library of clonotypes of the binding agent isolated in step (f);
h. screening the library of clonotypes of step (g) against:
i. the modified peptide;
ii. the first unmodified peptide; and
iii. a second unmodified peptide comprising a core amino acid sequence that is identical to the first unmodified peptide, a second N-terminal amino acid sequence that is not native to the protein or peptide epitope of interest, and a second C-terminal amino acid sequence that is not native to the protein or peptide epitope of interest; and
i. isolating a binding agent that binds to the first unmodified peptide and the second unmodified peptide, wherein the binding agent does not bind the modified peptide, and the binding agent is the antigen-binding protein or derived therefrom; or
(3) generating the B-cell or B-lymphocyte comprises:
a. providing a modified peptide having an amino acid sequence of about 8 to about 20 amino acids identical to an amino acid sequence of the protein or peptide epitope of interest and includes the amino acids of the target sequence, except wherein the wild-type amino acid at the site of interest has been substituted with a non-native amino acid (nnAA);
b. screening the modified peptides against a library;
c. isolating one or more binding agents that bind to the modified peptide;
d. generating a library of clonotypes of the one or more binding agents isolated in step (c);
e. screening the library of clonotypes against:
iii. the modified peptide of step (a); and
iv. a first unmodified peptide comprising a core amino acid sequence identical to the modified peptide of step (a), except wherein the non-native amino acid (nnAA) has been substituted with the wild-type amino acid (nAA) and comprising a first N-terminal amino acid sequence that is not native to the protein or peptide epitope of interest and a first C-terminal amino acid sequence that is not native to the protein or peptide epitope of interest;
f. isolating a binding agent that bind to both the first unmodified peptide and the modified peptide;
g. generating a library of clonotypes of the binding agent isolated in step (f);
h. screening the library of clonotypes of step (g) against:
i. the modified peptide;
ii. the first unmodified peptide; and
iii. a second unmodified peptide comprising a core amino acid sequence that is identical to the first unmodified peptide, except wherein the wild-type amino acid (nAA) has been substituted with an amino acid corresponding to a variant and comprising a second N -terminal amino acid sequence that is not native to the protein or peptide epitope of interest and a second C-terminal amino acid sequence that is not native to the protein or peptide epitope of interest; and
i. isolating a binding agent that binds to the second unmodified peptide and does not bind the first modified peptide, wherein the binding agent is the antigen-binding protein or derived therefrom.

16. The method of any one of claims 13-15, wherein:
the non-native amino acid (nnAA) is offset from or relative to the site of interest;
the non-native amino acid (nnAA) is a non-synonymous amino acid;
the non-native amino acid (nnAA) is phosphorylated, acetylated, isocyanated, sulfated, or nitrated;
the non-native amino acid (nnAA) acid is O-phosphoserine (SEP), phosphotyrosine, or phosphothreonine;
the N-terminal amino acid sequence is SerGlySer, GlySerGly, GlyGlyGly, or SerSerSer;
the C-terminal amino acid sequence is SerGlySer, GlySerGly, GlyGlyGly, or SerSerSer; or
a combination thereof.
